Europäisches Patentamt

(19) European Patent Office

Office européen des brevets

(11) Publication number: **0 278 940**
A2

## (12) EUROPEAN PATENT APPLICATION

(21) Application number: 88870008.5

(22) Date of filing: 25.01.88

(51) Int. Cl.⁴: **C 12 N 15/00**
A 61 K 39/29, A 61 K 39/21,
A 61 K 39/295, A 61 K 39/015

(30) Priority: 30.01.87 US 9325

(43) Date of publication of application:
17.08.88 Bulletin 88/33

(84) Designated Contracting States:
AT BE CH DE ES FR GB GR IT LI LU NL SE

(71) Applicant: Smith Kline - RIT Société anonyme dite:
rue du Tilleul, 13
B-1320 Genval (Rixensart) (BE)

(72) Inventor: Cabezon, Teresa
Rue de Terheyde, 78
B-1640 Rhode St Genèse (BE)

De Wilde, Michel
Avenue Gevaert, 136
B-1320 Genval (BE)

Harford, Nigel
Jef Lambeaulaan, 9
B-1900 Overijse (BE)

(74) Representative: Tasset, Gérard
SMITHKLINE - RIT rue de l'Institut, 89
B-1330 Rixensart (BE)

The microorganism(s) has (have) been deposited with American Type Culture Collection under number(s) ATCC 20818, 20819, 20820, 67186, 67187, 67193.

(54) **Hepatitis B virus surface antigens and hybrid antigens containing them.**

(57) The invention relates to hepatitis B virus antigens and hybrid antigens containing them and to the cloning of genes which code for said antigens in yeast by use of recombinant DNA techniques. Said antigens are valuable for the preparation of vaccines, e.g. hepatitis B virus vaccines or malaria vaccines.

EP 0 278 940 A2

**Description**

Hepatitis B virus surface antigens and hybrid antigens containing them

FIELD OF THE INVENTION

The invention relates to hepatitis B virus antigens and hybrid antigens containing them and to the cloning of genes which code for said antigens in yeast by use of recombinant DNA techniques.

BACKGROUND OF THE INVENTION

A. Hepatitis B vaccines

Infection with hepatitis B virus (HBV) is a serious, widespread health problem. Infection can be manifested in acute or chronic phases. In the United States, the number of cases of acute hepatitis is estimated to be at least 100,000 per year with a fatality rate of 1 to 2 per cent and the prevalence of chronic carriers of HBV among healthy adults varies between 0.1 and 1% depending on age and social class. In South America, the prevalence of chronic carriers is about 1 to 3%; in the U.S.S.R. and southern Europe, about 3 to 6%; and in Asia and Africa, more than 10%.

In developed countries, there exists a need for a vaccine for people at high risk of exposure, such as patients and personnel in medical units where blood is handled, military personnel, spouses of chronic carriers, travelers to areas of high HBV endemicity, newborns of chronic carriers, homosexuals, prostitutes and drug abusers. In third world countries, there exists a need for an inexpensive vaccine for mass immunization. Mass immunization programs may ultimately affect not only the incidence of acute hepatitis and the pool of chronic carriers but may also reduce the morbidity and mortality from chronic active hepatitis and hepatocellular carcinoma.

Dane particles, which are believed to be hepatitis B virions and which are isolatable from infected patients, have a diameter of about 42nm. Each consists of an envelope comprising the hepatitis B surface antigen (HBsAg), a capsid (HBcAg), an endogenous polymerase and a DNA genome. The genome is circular and double-stranded with a single strand region consisting of about 200 bases. The single strand region can be filled in, in vitro, by the action of the endogenous polymerase. The longer strand contains approximately 3,200 bases.

For a long time, it has been difficult to prepare HBV vaccines because it has proven difficult to propagate the virus in tissue culture and because the only known host is man. However, chimpanzees are also able to be infected in the laboratory with the virus.

Valenzuela et al. (Nature, 298, 347-350, 1982) report synthesis of HBsAg in yeast using an expression vector wherein the HBsAg coding sequence is an 835 base pair (bp) TaqI-HpaI fragment and the promoter is the yeast alcohol dehydrogenase I promotor. Several earlier brief reports noted research preceding this reference. These are Valenzuela et al. (Arch. Biol. Med. Exp. (Chile), 14(1), 21-22, 1981) which reports expression in yeast of a DNA fragment containing a sequence which codes for a protein similar to HBsAg ligated to a yeast alcohol dehydrogenase promotor region; a report in Scrip No. 616, p. 14 (Aug. 12, 1981), which states that a team of U. S. researchers including P. Valenzuela and W. J. Rutter have announced production in yeast of "the protein-coating surrounding hepatitis B virus"; and Zuckerman (Nature, 295, 98-99, 1982) which reports that W. J. Rutter has reported expression of glycosylated HBsAg in yeast cells.

Antigenic components of HBV, such as HBsAg, are reported to have been prepared in bacteria following insertion of a recombinant DNA molecule containing a gene which codes for the antigen. Burrell et al. (Nature, 279, Number 5708, 43-47, 1979) report expression in E. coli HB1O1 of HBV DNA sequences cloned in plasmid pBR322.

Murray et al., European Patent Application Publication Number 13,828, 1980, disclose preparation of a recombinant vector which can code for HBV antigens, including HBsAg, in E. coli strain HB101. The vector is prepared from Dane particle DNA and plasmid pBR322. The authors state that useful hosts may include other bacterial hosts, yeasts and other fungi, animal or plant cells and other hosts, although the only host exemplified in the application is E. coli.

Charnay, et al. (Nature, 286, 893-895, 1980) report construction of a bacteriophage carrying a fusion of the β-galactosidase gene and the HBsAg structural gene. The bacteriophage directs synthesis of a fusion protein comprising antigenic determinants of both HBsAg and β-galactosidase.

Tiollais et al., UK Patent Application Number 2,034,323, disclose preparation of a coliphage containing HBV DNA. Fused phage-HBV DNA is transformed into E. coli strain C600.

In UK Patent Application Number 2,070,621, a plasmid which comprises a part of the HBsAg gene and the promotor and the Z gene of the lactose operon and which can be cloned in E. coli is disclosed.

Rutter et al., European Patent Application Publication Number 20,251, 1980, disclose recombinant vectors including a recombinant vector comprising plasmid pBR322 and BamHI fragments of HBV DNA, which can be used to transform E. coli. Another vector, comprising a BamHI fragment of HBV DNA and a portion of the tryptophan operon, was used to obtain expression in E. coli strain HB1O1.

Edman et al. (Nature, 291, Number 5815, 503-506, 1981) describe construction of plasmids which direct synthesis of HBcAg and a β-lactamase-HBsAg fusion protein, under the control of the tryptophan operon regulatory region, in E. coli.

Pumpen et al. (Gene, 30, 201-210, 1984) disclose that small amounts of HBsAg monomer proteins and fusion proteins are synthesized in E. coli using antibodies raised against the denatured HBsAg monomer for detection.

Other references disclosing insertion of HBV DNA into bacteria include Charnay, et al. (Prog. Med. Virol., 27, 88-92, (1981); MacKay et al. (Proc. Natl. Acad. Sci. U.S. 78, Number 7, 4510-4514, 1981); Fritsch et al. (C.R. Acad. Sci., 287, Number 16, 1453,(1978); U. K. Patent Specification 2,034,323 (Derwent No. 46874C) and Pasek et al. (Nature, 282, No. 6, 575, 1979).

HBV DNA has also been cloned in mammalian cells. These include, human, mouse, and human hepatoma cell lines. For example, Dubois et al. (Proc. Natl. Acad. Sci. U.S., 77, Number 8, 4549-4553, 1980) report transformation of mouse cells with a plasmid containing the HBV genome and expression of HBsAg; Hirschman et al. (Proc. Natl. Acad. Sci. U.S., 77, Number 9, 5507-5511, 1980) report production of HBV-like particles by HeLa cells transformed with HBV DNA.

Procedures for preparing HBV vaccine using HBsAg from human blood are reported by Funakoshi et al. (Prog. Med. Virol., 27, 163-167, 1981) and Maupas et al. (Prog. Med. Virol., 27, 185-201, 1981). The vaccine prepared by Funakoshi et al. contains 40 μg of purified, formalin-treated HBsAg, phosphate, sodium chloride, 20 mg of mannitol; and 0.1% of aluminum hydroxide as adjuvant. In the latter paper, Maupas et al. report that one dose of vaccine was 1 mL of purified, formalin-treated HBsAg containing 2-10 μg/mL of protein (Lowry's method) and 0.1% of aluminum hydroxide. The protocol used in the study reported by Maupas et al. called for three injections at one month intervals with a booster after one year; the authors propose a protocol consisting of two injections of concentrated HBsAg at three month intervals.

Additional references to preparation of HBV vaccines include Maupas et al., Adamowicz et al., and Funakoshi et al. at pages 3, 37 and 57, respectively, of Hepatitis B Vaccine INSERM Symposium No. 18, edit. by Maupas and Guesry, 1981, Elsevier/North-Holland Biomedical Press.

Yeasts have been used as host organisms for expression of certain other non-HBV DNA sequences. For example, Fraser et al., U. K. Patent Application 2,068,969, disclose preparation of chicken ovalbumin in yeast; Scrip No. 640, p. 11 (Nov. 4, 1981) contains a report that a type of interferon is being prepared in yeast. In European Patent 11,562 (Derwent No. 38762C) are reported hybrid yeast plasmids containing the $ura_3^+$ yeast gene in the 2 μ plasmid.

The authentic Hepatitis B Virus surface antigen (HBsAg) can be recovered from plasma of infected individuals as a particle of about 22 nm comprised of two proteins known as P24, and its glycosylated derivative GP28, both of which are encoded by the 226 amino acid coding sequence on the HBV genome known as the S protein coding sequence. The entire 163 amino acid coding sequence which immediately precedes the S protein coding sequence on the HBV genome is referred to herein as the Pre S coding sequence. The 55 amino acids of the Pre S coding sequence which immediately precede the S protein coding sequence are referred to herein as the Pre S2 coding sequence, and the remaining 108 amino acids of the Pre S coding sequence are referred to herein as the Pre S1 coding sequence. The Pre S coding sequence, or any smaller portion thereof, is also referred to herein as the HBsAg precursor protein.

For completeness, it should be noted that the entire pre S coding sequence for some HBV subtypes (e.g., ayw) is 163 codons, while the entire Pre S coding sequence for other HBV subtypes (e.g., adw2) is 174 codons. In either case, the Pre S2 region is the 55 codons immediately preceding the S protein coding sequence.

The Pre S2 coding sequence codes for the polyalbumin binding or receptor site found on the surface of Dane particles and on the surface of some HBsAg particles isolated from sera of HBV-infected patients. Although the Pre S2 region immediately precedes the S protein coding region on the HBV genome, the Pre S2 region is not involved in the assembly of HBsAg particles. See, e.g., Persing et al. (Proc, Natl. Acad. Sci. U.S.A., 82, 3440-3444, 1985).

Valenzuela et al. (Nature, 298, 347-350, 1982) disclose that particles resembling HBsAg were found after disruption of yeast cells transformed with a vector containing the S protein coding sequence, and concluded that HBsAg particles are synthesized in yeast.

Harford et al. (Developments in Biological Standardization, 54, 125-139, 1983) disclose that particles resembling HBsAg were found after disruption of yeast cells transformed with a vector containing 18 amino-acids of the coding sequence of ornithine carbamoyl transferase immediately preceding the N-terminal 42 amino acids of the Pre S2 coding sequence immediately preceding the S protein coding sequence.

Laub et al. (J. Virology, 48(1), 271-280, 1983) disclose construction of a Simian Virus 40 early replacement vector that has a large portion of HBV genome DNA including the Pre S-S protein coding sequence, and expression of such portion by SV40- transformed CV-1 cells (COS cells) transformed by such vector.

Stibbe et al. (J. Virology, 46(2), 626-628, 1983) disclose that minor HBsAg glycoproteins, named GP33 and GP36, are coded for by the Pre S2-S protein coding sequence. Stibbe et al. (Dev. Biol. Stand., 54, 33-43, 1983) disclose that HBsAg particles containing large amounts of GP33 and GP36 did not induce higher antiS protein antibody titers than those HBsAg particles almost devoid of GP33 and GP36.

Heerman et al. (J. Virol., 52 (2), 396-402, 1984) disclose that the entire 389 amino acid coding sequence comprising both the S protein coding sequence and the Pre S coding sequence codes for a polypeptide, P39, which is found in HBV particles and viral surface antigen filaments along with its glycosylated form, GP42, and the other HBV surface antigen associated polypeptides P24, GP27, GP33 and GP36.

Neurath et al. (Science, 224, 392-394, 1984) disclose that polypeptides having the sequence of the 26 amino-terminal amino acids of the Pre-S2 coding sequence act as an immunogen, and suggest that antibodies elicited by the immunogen can be utilized for diagnostic tests.

Michel et al. (Proc. Natl. Acad. Sci. U.S.A., 81, 7708-7712, 1984) disclose synthesis of HBsAg carrying human serum polyalbumin receptors in Chinese hamster ovary (CHO) cells transfected with a plasmid carrying the Pre S2-S protein coding sequence.

Persing et al. (Proc. Natl. Acad. Sci. U.S.A., 82, 3440-3444, 1985) disclose that mouse L cells transformed with a Pre S2-S protein coding sequence produce three HBsAg related polypeptides (24,000, 27,000 and 35,000 daltons) all of which may be organized into complex immunoreactive HBsAg particles of 22 nm diameter which bind to polymerized human serum albumin (HSA); while mouse L cells transformed with the Pre S2-S protein coding sequence bearing a frame-shift mutation near the 3′ end of the Pre S2 region produce only the 24,000 and 27,000 dalton polypeptides organized into 22 nm diameter immunoreactive HBsAg particles which are unable to bind HSA. Persing et al. conclude that the Pre S2-S protein coding sequence encodes the 35,000 dalton species, that the Pre S2 protein accounts for the HSA binding activity of HBsAg but is not required for assembly and secretion of the HBsAg particles; and that the major polypeptide of HBsAg (i.e., the 24,000 dalton species) is not derived primarily by cleavage of larger precursors (i.e., the 27,000 and 35,000 dalton species) encoded by the Pre S2-S protein coding sequence.

Milich et al. (Science, 228, 1195-1199, (1985) disclose that vaccines that contain HBV particles with both Pre S2 and HBsAg, prepared from Chinese Hamster Ovary (CHO) cells transfected with a plasmid containing the Pre S2-S protein coding sequence, can circumvent nonresponsiveness in certain mice to vaccines which just contain only HBsAg; and that the 26 amino acid residues at the $NH_2$-terminus of the 33,000 dalton polypeptide coded for by the Pre S2-S protein coding sequence represent a dominant antibody binding site on the Pre S2 region.

Michel et al., "Vaccines 86", Brown et al., Ed., Cold Spring Harbor Laboratory (1986), 356-363, discuss the synthesis in CHO cells of Hepatitis B surface antigen particles containing the Pre S2 region expression product.

Neurath et al. (Nature, 315, 154-156, 1985) disclose that the Pre S region codes for proteins on the HBV envelope with domains specifically recognized by liver cells; the Pre S2-S protein coding sequence codes for a protein present in HBV particles; synthetic peptides corresponding to the gene encoding Pre-S proteins are immunogenic; and conclude that HBV vaccines should contain Pre S determinants.

Valenzuela et al. (Biotechnology, 3, 317-320, 1985) disclose the expression of the entire pre S2-S protein coding sequence in yeast transformed with such coding sequence; and also disclose that such yeast cells do synthesize a particle containing both HBsAg and Pre S2 which is very similar in electronmicroscopy and sedimentation properties to particles containing only HBsAg, and that the Pre-S2 region does not infer the ability to form particles resembling the 22 nm HBsAg particles. Valenzuela et al. also disclose that it has been hypothesized that HBV gets into the liver by binding polyalbumin to its polyalbumin receptor which in turn binds the polyalbumin receptor to the liver cell and thus, the virus gets internalized, and that the polyalbumin receptor of the HBV is encoded by the Pre S2 region. Valenzuela concludes that a vaccine containing Pre S2 will elicit antibodies which, in addition to inactivating HBV through the normal mechanism, might interfere with the way the virus enters liver cells.

Takeda Chemical Ind. KK, European Patent Application Publication No. 171,908-A, claims the production of non-glycosylated hepatitis-B virus surface antigen P31 protein in yeast.

Chiron Corporation, European Patent Application Publication No. 0,174,444 A2, claims a method for preparing HBsAg particles containing P31 protein in yeast.

The following Table I compares known Pre S2 region amino acid sequences with the HBV pre S2 amino acid sequence of the subject invention :

### Table I Comparison of amino acid sequence of pre S2 region from different serotypes of HBV

| HBV sub-type | Pre S2 coding region | | | | | | | Reference |
|---|---|---|---|---|---|---|---|---|
| | -55 | -50 | -40 | -30 | -20 | -10 | 0 | |
| adw | MQWNSTAFHQALQDPRVRGLYFPAGGSSSGTVNPAPNIASHISSSSARTGDPVTN | | | | | | | The subject Pre S2 coding region |
| adw | | | T | L | | I | | 1 |
| adw | | L | | L | | I | I | 2 |
| adw | | | | | | I | | 3 |
| adw | | | K | | | I | | 4 |
| adr | | T | L | | V TT | P | IFS | AP | 5 |
| adr | | T | L | | V TT | P | I S | AP | 6 |
| ayw | | T | T | | VLTT | PL | IFS I | AL | 7 |
| adyw | H | T | T | | V TTT | P | IFS I | AL | 8 |

### Amino Acid Abbreviation Key

| | | | | | |
|---|---|---|---|---|---|
| Asp | D | Aspartic acid | Ile | I | Isoleucine |
| Thr | T | Threonine | Leu | L | Leucine |
| Ser | S | Serine | Tyr | Y | Tyrosine |
| Glu | E | Glutamic acid | Phe | F | Phenylalanine |
| Pro | P | Proline | His | H | Histidine |
| Gly | G | Glycine | Lys | K | Lysine |
| Ala | A | Alanine | Arg | R | Arginine |
| Cys | C | Cysteine | Trp | W | Tryptophan |
| Val | V | Valine | Gln | Q | Glutamine |
| Met | M | Methionine | Asn | N | Asparagine |

Reference Key
1. Valenzuela et al., In Animal Virus Genetics (Field, Jaenisch and Fox eds) p. 57-70, Academic Press, New York (1980).
2. Chow et al., Fourth Annual Congress for Recombinant DNA Research, San Diego, USA, 1984.
3. Fujisawa et al., Nucl. Acids Res., 11, 4601-4610 (1983).
4. Lo et al., Biochem Biophys. Res. Com., 2, 382-388 (1986).
5. Fujisawa et al., cited above.
6. Ono et al., Nucl. Acid Res., 11, 1747-1757 (1983).
7. Galibert et al., Nature, 281, 646-650 (1979).
8. Pasek et al., Nature, 282, 575-579 (1979).

## B. Malaria Vaccines

Numerous recent studies suggest that protective immunity in a sensitive host to infection by the sporozoite stage of a particular species of Plasmodium can be mediated via antibodies produced by such host to the circumsporozoite protein (CS protein) of the particular sporozoite.

The sporozoite proteins of some types of Plasmodium have been cloned and some of those have been expressed by recombinant DNA techniques.

Nussenzweig et al. (U.S. Patent 4,466,917) disclose a sporozoite polypeptide, identified as the P-44 protein, and its cloning and expression in E. coli.

Sharma et al. (Science, 228, 879-882, 1985) disclose the expression of a portion of the circumsporozoite (CS) protein of Plasmodium knowlesi in yeast by employing an expression vector containing the 5' regulatory region of the yeast alcohol dehydrogenase I gene in place of the 5' upstream region of the P. knowlesi CS gene sequence.

New York University, PCT application Publication Number WO84-02922-A, disclose cloning of a portion of the coding region for the P. knowlesi circumsporozoite (CS) protein repeat unit and expression of beta-lactamase and beta-galactosidase fusions thereof in E. coli.

Kemp et al., PCT Patent Application Publication Number WO84-02917-A, disclose cloning and expression in E. coli of a coding sequence for the CS protein derived from the blood stage of P. falciparum.

Dame et al. (Science, 225, 593, 1984) report cloning and expression of the CS protein of P. falciparum in E. coli. The protein is described as comprising about 412 amino acids with an approximate molecular weight of 44,000. It comprises 41 tandem repeats of a tetrapeptide. Synthetic 7-, 11- and 15- residue peptides derived from the repeat region bound to monoclonal antibodies raised against the CS protein.

Ellis et al. (Nature, 302, 536-538, 1983) report expression of a beta-lactamase - P. knowlesi CS protein fusion in E. coli.

Enea et al. (Proc. Natl. Acad. Sci., USA, 81, 7520-7524, 1984) report an analogous repeat unit structure within the CS protein of P. cynomolgi, and the cloning and expression of the CS protein in E. coli as well as a disclosure of its coding sequence.

Ballou et al. (Science, 228, 996-999, 1985) disclose that synthetic peptides of the repeating region of the CS protein of Plasmodium falciparum conjugated to bovine serum albumin (BSA) or to thyroglobulin produced antibody responses in mice and rabbits, and conclude that a vaccine against the sporozoite stage of the malaria parasite can be developed by using synthe tic peptides of the repeating region of the CS protein conjugated to a carrier protein.

Weber et al. (Molecular and Bacterial Parasitology, 15, 305-316, 1985) disclose the use of a cloned CS protein gene of a Brazilian strain of P. falciparum as a probe to analyze the structure of 17 other P. falciparum strains by nucleic acid hybridization, and conclude that the CS protein gene is highly conserved and that malaria vaccine development with the CS protein is unlikely to be complicated by strain variation.

Arnot et al. (Science, 230, 815-818, 1985) disclose the cloning of the CS protein of P. vivax and disclose its entire coding sequence and conclude that the coding sequence of the CS protein of P. vivax is homologous to that of the CS gene of P. knowlesi but not P. falciparum.

Sharma et al. (Science, 229, 779-782, 1985) disclose the complete nucleotide sequence of the coding region of the CS gene of the Nuri strain of P. knowlesi.

Young et al. (Science, 228, 958-962, 1985) disclose the expression of P. falciparum CS proteins in E. coli for potential use in a human malaria vaccine.

The Walter and Eliza Hall Institute of Medical Research, PCT Patent Application Publication No. WO84/02917, 1984. disclose artificially constructed polynucleotide sequences substantially corresponding to all or a portion of P. falciparum mRNA or genomic DNA; the peptide corresponding to such sequence and a method for production thereof; a composition for stimulating immune responses against P. falciparum antigens in a mammal comprising such peptide.

Mazier et al. (Science, 231, 156-159, 1986) disclose that antibodies raised in mice immunized with several recombinant and synthetic peptides of the circumsporozoite protein of P. falciparum were evaluated for protective activity in a human hepatocyte culture system and were found to exhibit a protective effect against the parasite at three points: sporozoite attachment to the hepatocyte surface, entry and subsequent intracellular development.

## C. Polyvalent vaccines

Valenzuela et al. (Biotechnology, 3, 323-327, 1985) disclose the use of the HBsAg polyalbumin receptor coded for by the Pre S2 coding sequence as a means to prepare polyvalent vaccines. Valenzuela et al. also disclose preparation of a hybrid HBsAg-Herpes simplex 1 virus glycoprotein D (HSV-lgD) particle by expressing a hybrid HSV-lgD-Pre S2-S protein coding sequence in yeast.

Valenzuela, "Hepatitis B subunit vaccines using recombinant DNA Techniques," May 15, 1985 Bio-Expo-5-Meeting, Boston Massachussetts, discloses that with a hybrid such as the HBsAg-HSV-lgD particle described above, there is a problem of immunodominance of the HBsAg over the foreign immunogen presented on its surface.

Chiron Corporation, European Patent Application Publication No. 0,175,261 claims a novel hybrid particle containing at least a major portion of the Hepatitis B surface antigen fused to one or more oligopeptides, where the hybrid polypeptide is capable of forming a particle in a cellular host with presentation of at least one epitope of at least one oligopeptide.

Proteins can undergo post-translational modifications and some of these can profoundly affect the protein's conformation and function. The presence of oligosaccharide chains on human derived preS1-preS2-S and preS2-S protein (Heerman et al. in J. Virol., 52, 396-402, 1984; Stibbe and Gerlich in Virology, 123, 436- 442, 1982; Stibbe and Gerlich in J. Virol., 46, 626-628, 1983) and presumably of myristic acid on the preS1- preS2-S protein (Persing et al., Abstracts of papers presented at the 1986 meeting on Molecular Biology of Hepatitis B viruses, August 28-31, 1986, Cold Spring Harbor Laboratory) might influence particle formation, conformation of the proteins in the particle and the antigenicity and immunogenicity of these particles.

Yeast (Saccharomyces cerevisiae) has the enzymaticcapability for protein glycosylation (Ballou, "The Molecular Biology of the Yeast Saccharomyces, Metabolism and Gene Expression"; Strathern, Jones and Broach Ed., Cold Spring Harbor Laboratory (1982), p. 335-360) and fatty acid acylation (Towler and Glasier in Proc. Natl. Acad. Sci., 83, 2812-2816, 1986) similar to those of higher eukaryotic cells.

Viral surface glycoproteins expressed in yeast have been found to be glycosylated. Jabbar et al. (Proc. Natl. Acad. Sci., 82, 2019-2023, 1985) disclose that expression of the influenza viral hemagglutinin gene in yeast resulted in a glycosylated hemagglutinin protein. Wen and Schlesinger (Proc. Natl. Acad. Sci., 83, 3639-3643, 1986) disclose that expression of Sindbis and Vesicular stomatitis virus glycoproteins in yeast gave glycosylated forms of these viral proteins. Fujisawa et al. (Abstracts of papers presented at the 1986 meeting on Molecular Biology of Hepatitis B viruses, August 28-31, 1986, Cold Spring Harbor Laboratory, page 62) disclose that expression of the preS2-S gene in yeast results in the synthesis of two glycosylated forms of the preS2-S protein. Kniskern et al. (Abstracts of papers presented at the 1986 meeting on Modern Approaches to New Vaccines, Including Prevention of AIDS, September 9-14, 1986, Cold Spring Harbor Labo ratory, page 89) disclose that expression of the preS1-preS2-S gene in yeast results in the synthesis of two preS1-preS2-S proteins of 45 kD and 39 kD. Persing et al. (Abstracts of papers presented at the 1986 meeting on Molecular Biology of Hepatitis B viruses, August 28-31, 1986, Cold Spring Harbor Laboratory, page 19) disclose that labeling of COS7 cells expressing preS1-preS2-S, preS2-S and S proteins with [3]Hmyristic acid results in the presence of label in the preS1-preS2-S protein.

Fusion proteins expressed in yeast and reassembled in a particle are purified by the general scheme:

Aerosil adsorption and desorption (1) → $CaCl_2$ precipitation (2) → phenylagarose or phenylboronateagarose adsorption and desorption (3) → CsCl gradient centrifugation or DEAE-ion exchange chromatography.

Step 1 is described in EP application publication 0 204 680 and step 3 is described in EP application publication 0 199 698 as far as phenylagarose is concerned. The specific combination of step (1) and (3) is able to perform a drastic clean-up of contaminating material (elimination of 90% protein, carbohydrates and 50% lipids). This novel combination allows the good functioning of the next chromatographic steps.

In case that fusion proteins are glycosylated, phenylboronate (PBA) is used as an affinity adsorbent. At pH 9.0 the boronate group makes covalent complexes with potential cis-diol groups on the glycosylated sidechains. PBA gels have been used occasionally for the purification of soluble glycoproteins but not for the purification of particles containing glycoproteins embedded in the lipid bilayer (See, Cook, et al., Analytical Biochemistry, 149, 349-353, 1985; Middle, et al., Biochemical Journal, 209, 771-779, 1983; Cook, et al., Biochimica et Biophysica Acta, 828, 205-212, 1985; Maestasane, et al., Journal of Chromatography, 189, 225-231, 1980). Because of the multiple ligand binding sites on the particle, PBA gels with very low PB-ligand density have to be used, e.g., PBA gels with boronate content < 10μg/ml gel, preferably 5μg boronate/ml gel. The use of phenylboronate gels with very low boronate content allow affinity chromatography of particles containing embedded glycoproteins.


## SUMMARY OF THE INVENTION

This invention relates to a recombinant DNA molecule comprising a functional DNA coding sequence fused, in phase, to a portion of the Pre S2 region of a Hepatitis B virus (HBV) Pre S2-S protein coding sequence. The term "coding sequence" or "coding region" as used herein also encompasses any functional derivative thereof. By the term "functional derivative" is meant a coding sequence with amino acid alterations which, where appropriate, don't interfere with particle formation and/or which retain immunogenicity where such retention is desirable. Such functional derivative can be prepared by conventional site specific mutagenesis such as by the methods disclosed by Botstein et al. (Science, 229, 1193-1201, 1985. Preferably such DNA molecule also comprises a regulatory region which is preferably derived from the yeast arg[3] gene.

This invention also relates to a recombinant vector which comprises a recombinant DNA molecule operatively linked to a regulatory region, wherein the DNA molecule contains a functional DNA coding sequence fused, in phase, to a portion of the Pre S2 region of a HBV pre S2-S protein coding sequence. By the term "regulatory region" as used herein is meant a DNA sequence which contains a promotor region and other sequences necessary for transcription and translation of a coding sequence.

This invention also relates to a eukaryotic host cell transformed with a recombinant vector, wherein said vector comprises a DNA molecule operatively linked to a regulatory region, and wherein said DNA molecule contains a functional DNA coding sequence fused, in phase, to a portion of the pre S2 region of a HBV Pre S2-S protein coding sequence. This invention also relates to a method of preparing such transformed host cell which comprises transforming a eukaryotic host cell with such recombinant vector.

This invention also relates to a method for preparing a hybrid particle containing HBsAg protein which contains and/or presents a peptide encoded by a functional DNA coding sequence, which comprises culturing a eukaryotic host cell transformed with a recombinant vector in appropriate culture, media and isolating the particle form a cell lysate or extract of such host's culture, wherein said vector comprises a DNA molecule operatively linked to a regulatory region, wherein the DNA molecule contains the functional DNA coding sequence fused, in phase, to a portion of the Pre S2 region of a HBV Pre S2-S protein coding sequence.

This invention also relatives to a hybrid immunogenic particle comprising a Hepatitis B virus surface antigen (HBsAg) particle which contains and/or presents a peptide encoded by a functional DNA coding sequence.

This invention also relates to a vaccine comprising an immunoprotective amount of such hybrid particles.

This invention also relates to micelle comprising such hybrid particle and polysorbate and to a vaccine comprising an immunoprotective amount of such micelles.

By the term "functional DNA coding sequence" is meant a DNA coding sequence which, when fused, in phase, to a portion of the Pre S2 region of a HBV Pre S2-S protein coding sequence, is not involved with, and does not interfere with, the assembly of the HBsAg particle.

Preferred functional DNA coding sequences include, but are not limited to, (a) the entire HBV Pre S protein coding sequence, or an immunogenic derivative thereof, such as, but not limited to, the Pre S2, Pre S1 or Pre S1-Pre S2 protein coding sequence; and (b) other immunogenic coding sequences such as the coding sequence of the circumsporozoite protein of Plasmodium, or any immunogenic derivative thereof, the envelope gene coding sequence of HIV or any immunogenic derivative thereof, especially the $C_7$ peptide, peptide 121 or Dreesman peptide coding sequence or an immunogenic derivative thereof.

This invention also relates to a HBV Pre S1 protein coding region which codes for the following amino acid sequence:

$-163$

```
ATG GGG ACG AAT CTT TCT GTT CCC AAC CCT CTG
Met Gly Thr Asn Leu Ser Val Pro Asn Pro Leu


GGA TTC TTT CCC GAT CAT CAG TTG GAC CCT
Gly Phe Phe Pro Asp His Gln Leu Asp Pro


GCA TTC GGA GCC AAC TCA AAC AAT CCA GAT
Ala Phe Gly Ala Asn Ser Asn Asn Pro Asp


TGG GAC TTC AAC CCC ATC AAG GAC CAC TGG
Trp Asp Phe Asn Pro Ile Lys Asp His Trp
```

```
CCA GCA GCC AAC CAG GTA GGA GTG GGA GCA
Pro Ala Ala Asn Gln Val Gly Val Gly Ala


TTC GGG CCA GGG CTC ACC CCT CCA CAC GGC
Phe Gly Pro Gly Leu Thr Pro Pro His Gly


GGT ATT TTG GGG TGG AGC CCT CAG GCT CAG
Gly Ile Leu Gly Trp Ser Pro Gln Ala Gln


GGC ATA TTG ACC ACA GTG TCA ACA ATT CCT
Gly Ile Leu Thr Thr Val Ser Thr Ile Pro


CCT CCT GCC TCC ACC AAT CGG CAG TCA GGA
Pro Pro Ala Ser Thr Asn Arg Gln Ser Gly


AGG CAG CCT ACT CCC ATC TCT CCA CCT CTA
Arg Gln Pro Thr Pro Ile Ser Pro Pro Leu


AGA GAC AGT CAT CCT CAG GCC
Arg Asp Ser His Pro Gln Ala
```

or to a HBV Pre S2 protein coding region or to a HBV Pre S2-S protein coding region wherein the Pre S2 portion thereof codes for the following amino acid sequence:

```
-55                          -50
Met-Gln-Trp-Asn-Ser-Thr-Ala-Phe-His-Gln-Ala-Leu-Gln-Asp-

   -40                                        -30
Pro-Arg-Val-Arg-Gly-Leu-Tyr-Phe-Pro-Ala-Gly-Gly-Ser-Ser-

Ser-

                          -20
Gly-Thr-Val-Asn-Pro-Ala-Pro-Asn-Ile-Ala-Ser-His-Ile-Ser-

       -10
Ser-Ser-Ser-Ala-Arg-Thr-Gly-Asp-Pro-Val-Thr-Asn;
```

a recombinant DNA vector comprising such Pre S1, Pre S2 or Pre S2-S protein coding region operatively linked to a regulatory region; a transformed host cell containing such vector; a process for preparing such transformed cell which comprises transforming a host cell with such vector; the protein coded for by such coding region; a process for preparing such protein; a vaccine comprising an immunoprotective amount of such protein, the particle code for by such Pre S2-S protein coding region; a process for preparing such particle, a vaccine comprising an immunoprotective amount of such particle, and a micelle comprising such particle and polysorbate, and a vaccine comprising an immunoprotective amount of such micelles.

This invention also relates to a recombinant DNA vector comprising an entire Pre S1-Pre S2-S protein coding sequence or PreS1-functional DNA coding sequence-PreS2-S protein coding sequence, or immunogenic derivative thereof, operatively linked to a regulatory region; to a yeast host cell transformed with such vector; to a method of preparing such transformed host which comprises transforming a yeast host cell with such vector; to a method of preparing protein encoded by the Pre S1-Pre S2-S protein coding sequence, or Pre-S1-functional DNA coding sequence - Pre S2-S protein coding sequence, or an immunogenic derivative thereof; to the protein prepared by such method; to a vaccine comprising an immunoprotective amount of such protein; to a particle containing polypeptide encoded by the Pre S1-Pre S2-S protein coding sequence or Pre S1-functional DNA coding sequence - Pre S2-S protein coding sequence or an immunogenic derivative thereof; to a method of preparation thereof, a vaccine comprising an immunoprotective amount of such particles, a micelle comprising such particle and polysorbate, and a vaccine comprising an immunoprotective amount of such Micelles.

This invention also relates to a Pre S1-Pre S2 protein coding sequence or Pre S1-Pre S2-S protein coding sequence of which the Pre S1-Pre S2 portion comprises the following amino acid sequence:

## PRE-S1 REGION

-163

```
ATG GGG ACG AAT CTT TCT GTT CCC AAC CCT CTG
Met Gly Thr Asn Leu Ser Val Pro Asn Pro Leu


GGA TTC TTT CCC GAT CAT CAG TTG GAC CCT
Gly Phe Phe Pro Asp His Gln Leu Asp Pro


GCA TTC GGA GCC AAC TCA AAC AAT CCA GAT
Ala Phe Gly Ala Asn Ser Asn Asn Pro Asp


TGG GAC TTC AAC CCC ATC AAG GAC CAC TGG
Trp Asp Phe Asn Pro Ile Lys Asp His Trp


CCA GCA GCC AAC CAG GTA GGA GTG GGA GCA
Pro Ala Ala Asn Gln Val Gly Val Gly Ala


TTC GGG CCA GGG CTC ACC CCT CCA CAC GGC
Phe Gly Pro Gly Leu Thr Pro Pro His Gly


GGT ATT TTG GGG TGG AGC CCT CAG GCT CAG
Gly Ile Leu Gly Trp Ser Pro Gln Ala Gln


GGC ATA TTG ACC ACA GTG TCA ACA ATT CCT
Gly Ile Leu Thr Thr Val Ser Thr Ile Pro


CCT CCT GCC TCC ACC AAT CGG CAG TCA GGA
Pro Pro Ala Ser Thr Asn Arg Gln Ser Gly
```

```
AGG CAG CCT ACT CCC ATC TCT CCA CCT CTA
Arg Gln Pro Thr Pro Ile Ser Pro Pro Leu


AGA GAC AGT CAT CCT CAG GCC
Arg Asp Ser His Pro Gln Ala
```

PRE-S2 REGION

-55

```
ATG CAG TGG AAT TCC ACT GCC TTC CAC CAA
Met Gln Trp Asn Ser Thr Ala Phe His Gln


GCT CTG CAG GAT CCC AGA GTC AGG GGT CTG
Ala Leu Gln Asp Pro Arg Val Arg Gly Leu


TAT TTT CCT GCT GGT GGC TCC AGT TCA GGA
Tyr Phe Pro Ala Gly Gly Ser Ser Ser Gly


ACA GTA AAC CCT GCT CCG AAT ATT GCC TCT
Thr Val Asn Pro Ala Pro Asn Ile Ala Ser


CAC ATA TCG TCA AGC TCC GCG AGG ACT GGG
His Ile Ser Ser Ser Ser Ala Arg Thr Gly


GAC CCT GTG ACG AAC
Asp Pro Val Thr Asn
```

This invention also relates to a recombinant vector comprising such novel Pre S1-Pre S2 or Pre S1-Pre S2-S protein coding sequence operatively linked to an expression control sequence; a host cell transformed with such vector; a method of preparing such transformed host which comprises transforming a host cell with such vector; to a method of preparing protein encoded by such Pre S1-Pre S2 or Pre S1-Pre S2-S protein coding sequence, or an immunogenic derivative thereof; to the protein prepared by such method; to a vaccine comprising an immunoprotective amount of such protein; to a particle containing polypeptide encoded by such Pre S1-Pre S2-S protein coding sequence or an immunogenic derivative thereof; to a method of preparation thereof, a vaccine comprising an immunoprotective amount of such particles, and a micelle comprising such particle and polysorbate.

The following represents the key to the amino acid abbreviations used in this application:

Asp Aspartic acid
Thr Threonine
Ser Serine
Glu Glutamic acid
Pro Proline
Gly Glycine
Ala Alanine
Cys Cysteine
Val Valine
Met Methionine
Ile Isoleucine

Leu Leucine
Tyr Tyrosine
Phe Phenylalanine
His Histidine
Lys Lysine
Arg Arginine
Trp Tryptophan
Gln Glutamine
Asn Asparagine

## BRIEF DESCRIPTION OF THE FIGURES

Figure 1 is a restriction endonuclease cleavage map of pRIT10601.

Figure 2 is a restriction endonuclease cleavage map of pRIT10616.

Figure 3 is a restriction endonuclease cleavage map of pMC200.

Figure 4 is the nucleotide sequence of a portion of the 3300 bp] HindIII yeast DNA insert in pMC200 which portion contains HincII, BglII and EcoRI sites.

Figure 5 is a flow sheet illustrating preparation of pRIT10671 and pRIT10673.

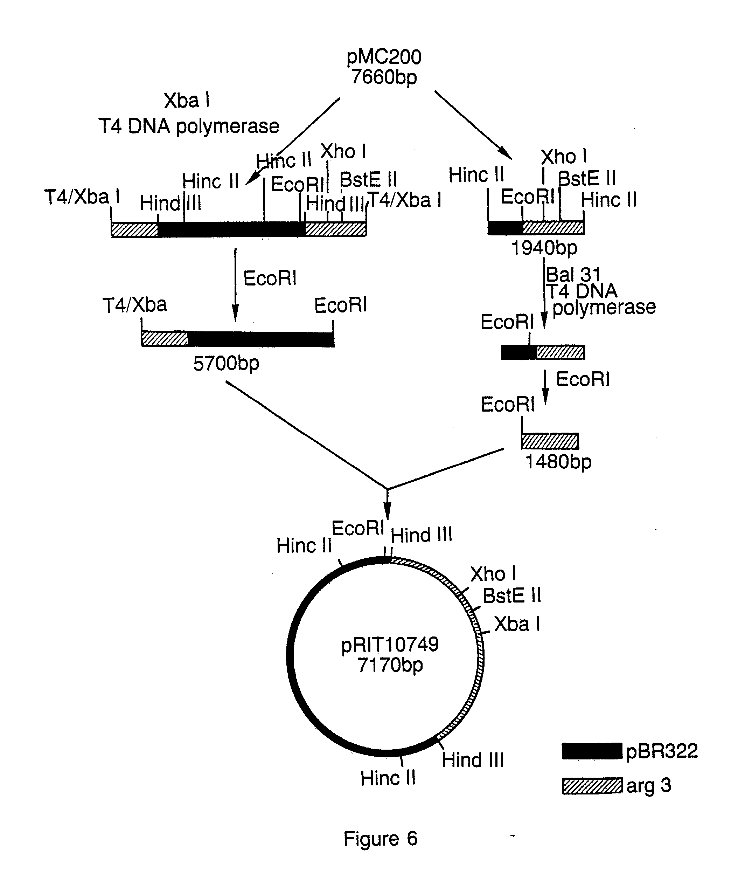

Figure 6 is a flow sheet illustrating preparation 35 of pRIT10749.

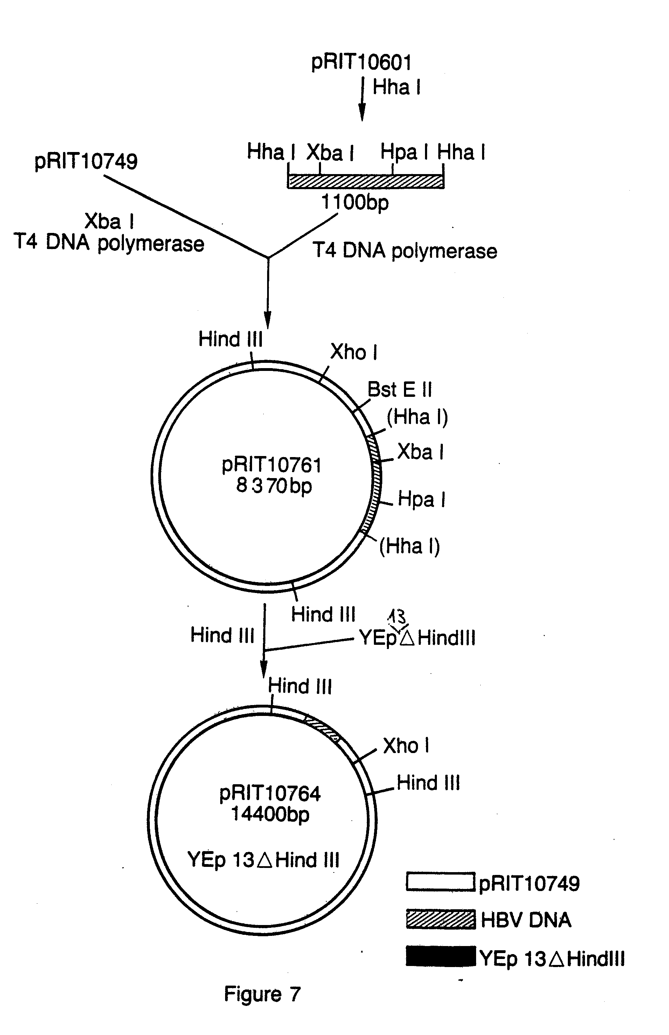

Figure 7 is a flow sheet illustrating preparation of pRIT10761 and pRIT10764.

Figure 8 is a flow sheet illustrating preparation of pRIT10167 (Example 1), pRIT10158 and pRIT10162 (Example 3); pRIT10158, pRIT10677, and pRIT10909 (Example 4); pRIT10911 (Example 5); pRIT10679, pRIT10903, pRIT10914 (Example 6); pRIT12211, pRIT12209 and pRIT12230 (Example 7); and pRIT12288 and pRIT12322 (Example 8).

Figure 9 is a restriction endonuclease cleavage map of pRIT10172.

Figure 10 is a restriction endonuclease cleavage map of pRIT12290.

Figure 11 is a restriction endonuclease cleavage map of pRIT12322 which illustrates which portions thereof were derived from pRIT12230 and pRIT12288.

Figure 12 is a flow sheet illustrating preparation of pRIT12571; pRIT12573; pRIT12572 and pRIT12574.

Figure 13 is a restriction endonuclease map of pRIT12309.

Figure 14 is a flow sheet illustrating preparation of pRIT12314 and pRIT12544.

Figure 15 is a flow sheet illustrating preparation of pRIT12658.

Figure 16 is a restriction endonuclease cleavage map of pRIT12662.

Figure 17 is a flow sheet illustrating preparation of pRIT12660.

Figure 18 is a flow sheet illustrating preparation of pRIT12845 (a,b,c,d,e,f).

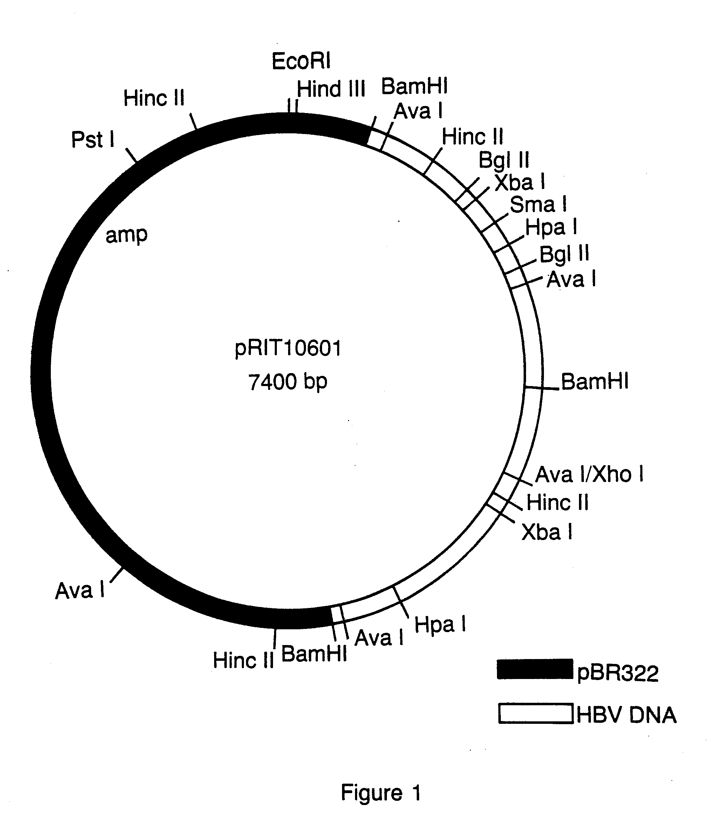

Figure 1A is a flow sheet illustrating the preparation of pRIT12662.

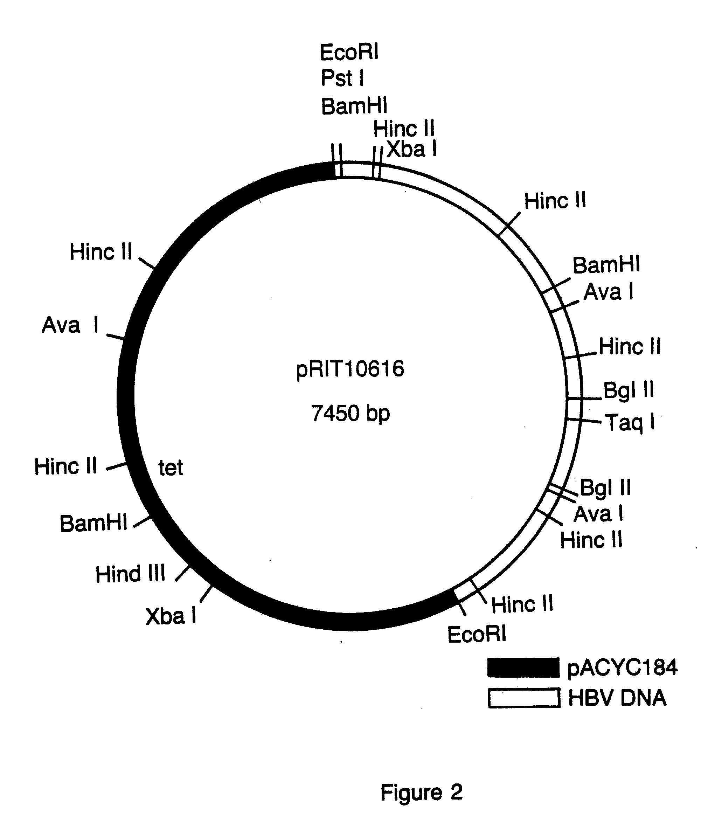

Figure 2A shows a schematic restriction map and sequencing strategy of clone λMPfl.

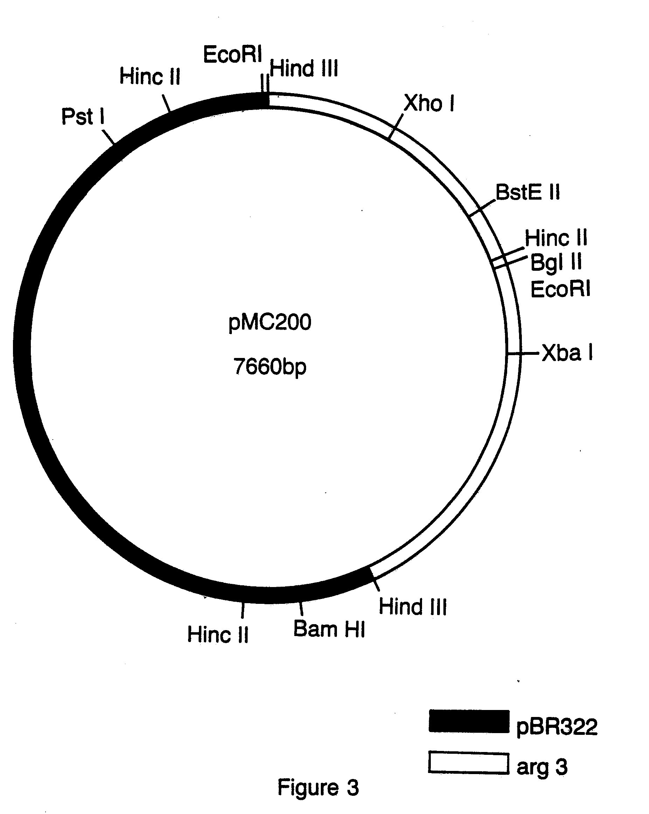

Figure 3A shows the nucleotide sequence of the circumsporozoite protein gene from P. falciparum.

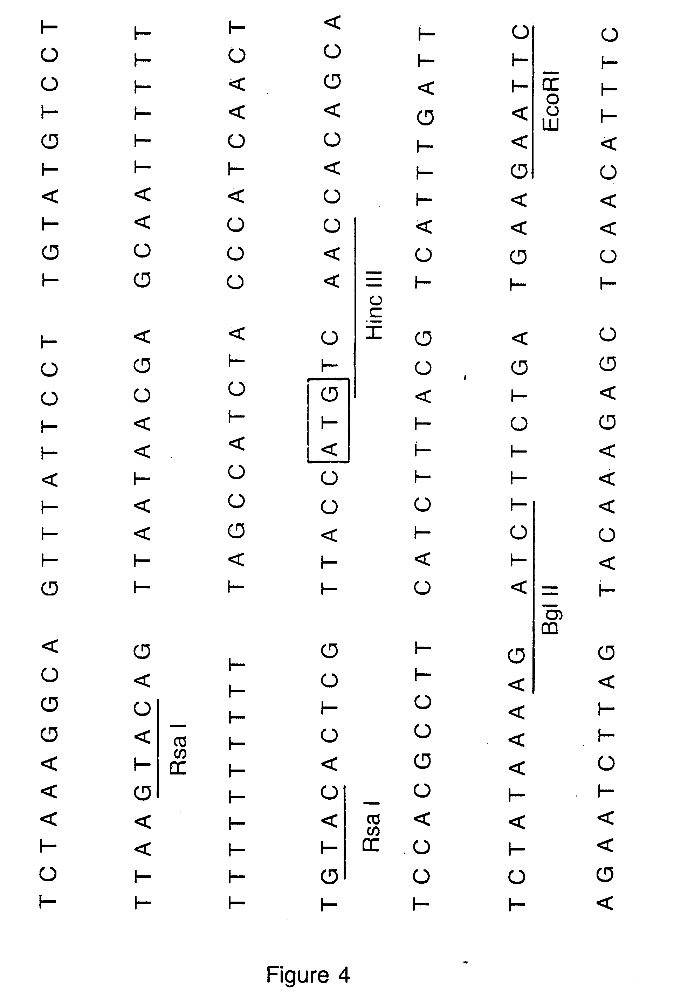

Figure 4A shows the PreS1-PreS2 DNA coding sequence comprised by pRIT12792.

Figure 1B is a schematic restriction map of the HIV B virus envelope region.

Figure 2B illustrates the construction of plasmid pRIT12893.

Figure 3B illustrates the construction of plasmid pRIT12897.

Figure 4B illustrates the construction of plasmid pRIT12899.

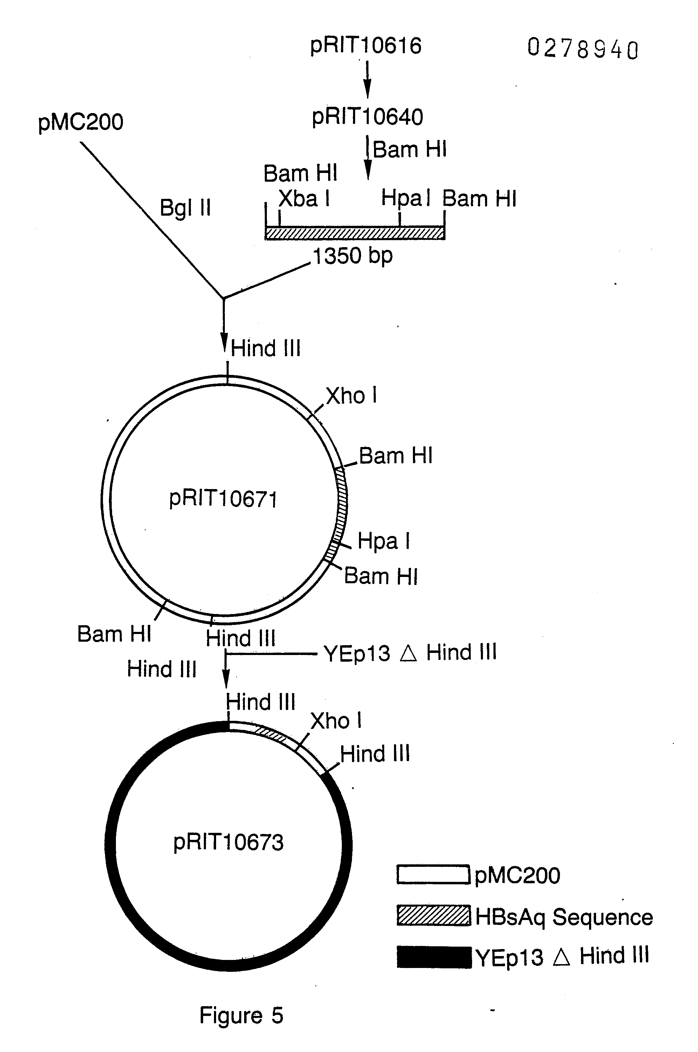

Figure 5B illustrates the construction of plasmid pRITX.

## DETAILED DESCRIPTION OF THE INVENTION

### EXPRESSION OF HBsAg IN YEAST

By "regulatory region" as used herein is meant a DNA sequence which contains sequences necessary or desirable for transcrption and translation of a coding sequence. Such regulatory regions are generally placed 5′ and adjacent to the coding sequence which is to be expressed. Preferably a further region of yeast DNA containing a signal for the termination of transcription is placed immediately 3′ to the coding region to be expressed so as to effect termination of the transcript.

By "HBsAg" as used herein is meant an antigen containing proteins encoded by the S protein coding sequence of the HBV genome, wherein such HBsAg is structurally similar to authentic HBsAg or has substantially the same S protein coding sequence encoded antigenic determinants as authentic HBsAg, that is, is capable of stimulating an immune response which speci fically recognize and react with authentic HBsAg or is specifically recognized by anti-HBsAg antibodies.

The HBsAg coding sequence can be isolated from DNA extracted from Dane particles in infected human serum by filling in the single strand region with a DNA polymerase, preferably the endogenous polymerase, followed by digestion with a restriction endonuclease. The choice of endonuclease will depend, in part, on the particular Dane particles. For example, the HBsAg coding sequence of HBV DNA of certain Dane particles of the adw serotype can be isolated on a single BamHI fragment; the HBsAg coding sequence of HBV DNA of certain Dane particles of the ayw serotype can be isolated on a HhaI fragment. HBV DNA of Dane particles of the same serotype may also exhibit different patterns of restriction sites.

Restriction of DNA to prepare DNA fragments used in the invention, ligation of such fragments to prepare recombinant DNA molecules used in the invention and insertion into microorganisms are carried out by known techniques such as techniques disclosed in the previously and subsequently cited references. Conditions are selected to avoid denaturation of the DNA and enzymes. For example, the pH is buffered to remain at about 7.0 to 11.0 and the temperature is kept below about 60°C. Preferably restriction is carried out at a temperature of about 30 to 40°C and ligation is carried out at about 0 to 10°C. Restriction enzymes and ligases used in carrying out this invention are commercially available and should be used in accordance with instructions included therewith. T4 DNA ligase is the preferred ligase.

The various fragments and final constructions may be joined together in accordance with conventional ways. In many cases, genes have been isolated and res triction mapped, as well as sequenced. To that extent, one can select the sequence of interest, such as the HBsAg sequence, by restriction of the gene, employing further manipulation as necessary such as resection with Bal31, in vitro mutagenesis, primer repair, or the like, to provide a fragment of a desired size, including the desired sequence, and having the appropriate termini. Linkers and adapters can be used for joining sequences, as well as replacing lost sequences, where a restriction site employed was internal to the region of interest. The various fragments which are isolated, may be purified by electrophoresis, electroeluted, ligated to other sequences, cloned, reisolated and further manipulated.

The use of regulatory regions of controlling transcription of the structural gene of interest, such as the HBsAg sequence, may allow for growing the host cells to high density with no or low levels of expression of the structural gene, such as the HBsAg sequence, and then inducing expression by changing the environmental conditions, e.g., nutrient, temperature, etc.

For example, with the GAL4 regulatory region, the yeast cells can be grown in rich media with a glycerol-lactic acid combination to high density, e.g., mid or late log phase, followed by switching the carbon source to galactose. As another example, for PHO5 regulation, one could grow the cells at a high phosphate concentration of about 7 mM $KH_2PO_4$, and then recover and resuspend them in medium lacking phosphate to effect derepression and expression. For temperature sensitivity, one could grow the cells at 25° to 37°C and then change the temperature as appropriate by about 5° to 20°C. The host cells would have the regulatory system associated with the regulatory region employed.

Fusing of the HBsAg sequence to the regulatory region can be accomplished by use of intermediate vectors. Alternatively, the HBsAg sequence can be inserted directly into a vector which contains the regulatory region. A vector is DNA which can carry and maintain the DNA fragment the invention, including, for example, phages and plasmids. Techniques for cloning DNA fragments in phages are disclosed, for example, by Charnay et al. Nature, Volume 286, 893-895 (1980) and Tiollais, United Kingdom Patent Application 2,034,323. Preferably, the HBsAg sequence is positioned relative to the regulatory region such that the HBsAg synthesized by expression of the HBsAg sequence is devoid of extraneous amino acid residues.

In one embodiment of this invention the HBsAg coding sequence together with 42 codons of the HBsAg precursor (preS-S) protein were fused in phase after the 18th amino acid of the coding sequence of the yeast OCT protein which is transcribed from the $arg^3$ promoter. This construct gives a hybrid particle containing HBsAg protein containing an additional 60 N-terminal amino acids to the 226 amino acids of the S-protein proper.

An exemplary regulatory region is derived from the yeast $arg^3$ gene which codes for ornithine carbamoyl-transferase (OCT). The $arg^3$ regulatory region is subject to both specific and general control mechanisms. It has been cloned in E. coli on plasmid $pYeura^3arg^3$ as reported by Crabeel et al., Proc. Natl. Acad. Sci. U.S.A., Volume 78, 6026 (1981). Preferred hosts are S. cerevisiae strains in which the arginine biosynthetic pathway is derepressed, such as strain Icl697d. Use of such strains results in increased expression from the $arg^3$ promoter as compared to strain DC5. Examples of other useful regulatory regions include those derived from yeast genes of the glycolytic pathway such as those coding for enolase, aldolase, phosphoglycerate kinase and glyceraldehyde-3-phosphate dehydrogenase; the yeast alcohol dehydrogenase gene; and, in general, genes involved in catabolism such as the arginase gene.

A preferred vector for cloning the fused DNA fragment into yeast is the plasmid YEp13, which is capable of replication and maintenance in both E. coli and S. cerevisiae and is, therefore, known as a shuttle vector. Several other yeast vectors are known and available. The HBsAg and regulatory regions can be inserted into a yeast vector sequentially or simultaneously. Transformation with plasmid vectors containing autonomous replication sequences (replicons) functional in yeast will normally result in extra-chromosomal maintenance of the DNA molecule of the invention as a plasmid. Such replicons are well known in the art. Transformation with plasmid vectors not containing replicons functional in yeast can result in incorporation of the DNA molecule into chromosomal DNA.

Vaccines for stimulating protection against HBV infection in humans comprising HBsAg produced by yeast in accordance with the invention and a suitable carrier can be prepared by known techniques. Use of an adjuvant, such as aluminum hydroxide or aluminum phosphate, is desirable. The HBsAg so produced can also be combined with other immunogens to prepare combination vaccines. The HBV or combination vaccines can be administered, for example, by the subcutaneous, intravenous or intramuscular route. The DNA fragment of the invention and the HBsAg produced thereby, can also be used as a probe for detection of HBV in biological samples by DNA hydridization techniques and various immunoassays.

13

0 278 940

## EXPRESSION OF A DNA MOLECULE CONTAINING A FUNCTIONAL DNA CODING SEQUENCE FUSED, IN PHASE, TO A PORTION OF THE PRE S2 REGION OF A HBV PRE S2-S PROTEIN CODING SEQUENCE

This invention also relates to a recombinant DNA molecule comprising a functional DNA coding sequence fused, in phase, to a portion of the Pre S2 region of a HBV Pre S2-S protein coding sequence. The term "coding sequence" or "coding region" as used herein also encompasses any functional derivative thereof. By the term "functional derivative" is meant a coding sequence involving amino acid alterations which, where desired, don't intefere with particle formation and/or which retain immunogenicity. Such functional derivatives can be prepared by conventional site specific mutagenesis. Site specific mutagenesis techniques are taught by Botstein et al., Science, 229, 1193-1201 (1985). Such fusion may occur at the N-terminus of the Pre S2 region or at some point within the Pre S2 region. There can be four types of fusions, i.e., at the 5'-terminus of the entire Pre S2 region, at some point within the Pre S2 region so that Pre S2 DNA is flanking both sides of the functional DNA coding sequence, at the 5'-terminal of a truncated portion of the Pre S2 region, or at the 5'-terminus of the S-protein coding region so that the fusion contains no pre S2 DNA. Various Pre S2-S protein coding sequences are known and can be prepared or isolated by known techniques. [See, references cited herein, supra]. In one embodiment of this invention, the ARG3 regulatory region together with 18 amino acids of the yeast OCT protein were fused, in phase, to a truncated Pre S2-S protein coding sequence such that the fused sequence contained 42 codons of the Pre S2-S protein coding sequence together with the complete S protein coding sequence. Preferred functional DNA coding sequences include the circumsporozoite protein coding sequence of Plasmodium, or any immunogenic derivative thereof, the envelope gene coding sequence of HIV or any immunogenic derivative thereof, especially the $C_7$ peptide, peptide 121 or Dreesman peptide coding sequence or any immunogenic derivative thereof, and the Pre S2, Pre S1 or the entire Pre S1-Pre S2-S protein coding sequence or any immunogenic derivative thereof. For example, a preferred functional DNA coding sequence is the first 13 Nterminal codons of the Pre S2 coding sequence which are then ligated to the C-terminal 42 codons of a truncated pre S2 region of a Pre S2-S protein coding sequence. By the term "immunogenic derivative" is meant a sequence which is a derivative or modified version of a naturally occurring sequence which retains or enhances the protective immunogenic activity of the natural sequence. Such immunogenic derivatives can be prepared by conventional techniques. The term "Pre S2, Pre S1 or the entire Pre S1-Pre S2-S protein coding sequence" as used herein includes any immunogenic derivative thereof.

By the term " the circumsporozoite protein of Plasmodium" is meant the immunodominant surface antigen on the sporozoite of Plasmodium or any immunogenic derivative thereof. Useful circumsporozoite proteins (CS) include, but are not limited to, those derived from Plasmodium falciparum, P. vivax, P. ovale, and P. malariae. P. falciparum, P. vivax, P. ovale and P.malariae infect humans.

The CS protein coding sequence of Plasmodium falciparum is disclosed by Dame et al., Science, 225, 593-599 (1984). The CS protein coding sequence of P. vivax is disclosed by Arnot et al., Science, 230, 815-818 (1985). The CS protein coding sequence of P. know lesi is disclosed by Sharma et al., Science, 229, 779-782 (1985). Enea et al., Proc. Natl. Acad. Sci. USA, 81, 7520-7524 (1984), disclose the CS protein coding sequence of P.cynomolgi. The CS protein coding sequences of other species of Plasmodium are known (see references cited above) and/or can be derived by conventional techniques.

As used herein, the terms "CSP" or "CS protein" will include both the natural, full length circumsporozoite protein of Plasmodium as well as any immunogenic derivative thereof. By the term "immunogenic derivative" of the circumsporozoite protein of Plasmodium is meant (a) any derivative of the circumsporozoite protein of Plasmodium which retains protective immunogenic activity or (b) any protein derived from a modified coding sequence of the circumsporozoite protein of Plasmodium which retains protective immunogenic activity. Such immunogenic derivatives can be prepared by conventional techniques. Some immunogenic synthetic derivatives of the CS protein coding sequence of P. falciparum are disclosed in Ballou et al., Science, 228, 996-999 (1985). See also, Mazier et al., Science, 231, 156-159 (1986).

The CS coding sequence for various species of Plasmodium is known and can be prepared or isolated by known techniques. [See, e.g., Colman et al., WO84-02922-A (P. knowlesi CS protein) and Dame et al., Science, 225, 593 (1984) (P. falciparum CS protein)]. A DNA molecule containing the CS coding sequence, or any other functional DNA coding sequence, fused in phase to a portion of the Pre S2 region of a HBV Pre S2-S protein coding sequence can be constructed by conventional techniques, such as by ligating the CS coding sequence, or any other functional DNA coding sequence, in phase to any codon within the 55 amino acid Pre S2 coding region of the Pre S2-S protein coding sequence.

Preferably, enough of the Pre S2 coding sequence remains after ligation of the CS coding sequence, or any other functional DNA coding sequence, to insure optimal presentation of the CS protein, or any other functional DNA coding sequence protein, on the HBsAg particle surface, i.e., enough Pre S2 region should remain so that the Pre S2 region encoded polypeptide acts as a bridge between the functional DNA protein coding sequence product and the HBsAg particle surface. It has been reported that the 26 amino-terminal amino acids of the Pre S2 coding sequence represent a dominant antibody binding site on the Pre S2 region. [See, e.g., Milich et al., Science, 228, 1195-1199 (1985)]. Thus, if it is desired to prepare a hybrid particle which contains and/or presents both Pre S2 epitopes and the CS, or any other functional coding sequence, epitopes, it is preferable to insert the CS coding sequence, or any other functional DNA coding sequence, at a point within the Pre S2 coding region which enables preparation of a hybrid particle which contains and/or presents both Pre S2 epitopes and CS, or any other functional DNA coding sequence, epitopes.

The recombinant vector of this invention comprises the recombinant DNA molecule of the subject invention

14

(i.e., a functional DNA coding sequence fused, in phase, to a portion of the Pre S2 region of a HBV Pre S2-S protein coding sequence) operatively linked to a regulatory region. Such vector may additionally also comprise a replicon functional in yeast. By the term "replicon" is meant that minimum region of DNA which functions to maintain stably, and extrachromosomally, such recombinant vector in a host yeast organism transformed with such vector. Such replicons are well known in the art. By the term "regulatory region" is meant any DNA region which regulates transcription and translation of the coding sequence desired to be expressed. Such regulatory regions are well known in the art. Such a vector can be prepared by conventional techniques such as by inserting the DNA molecule of this invention in phase into a vector already containing a replicon and regulatory region in such a way that the DNA molecule is operatively linked to such regulatory region. Preferably the DNA molecule is ligated to a vector capable of expression in the yeast genus Saccharomyces.

This invention also relates to a yeast host cell transformed with such recombinant vector and to a method of preparing such transformed host which comprises transforming a yeast host cell with the recombinant vector of this invention. Such transformation is carried out by conventional techniques such as those already described herein. Preferred yeast cells include those belonging to the genus Saccharomyces, particularly those belonging to the species Saccharomyces cerevisiae and Saccharomyces carlsbergensis.

This invention also relates to a method of preparing a hybrid particle containing HBsAg protein which contains and/or presents the functional DNA coding sequence product wherein such method comprises culturing the transformed yeast host cells of this invention in appropriate culture media, and isolating the particle from a cell lysate or extract of such host's culture. By the term "presented" or "contains and/or presents" is meant that the functional DNA coding sequence protein is contained within the hybrid particle containing HBsAg protein in such a way as to enable an immune response to the functional DNA coding sequence protein to be produced. When desirable, although not essen tial, the presentation of the functional DNA coding sequence protein does not interfere with the immunogenic activity of the HBsAg epitopes, thus yielding a bivalent vaccine. Most preferably the functional DNA coding sequence protein is presented on the HBsAg particle in such a way that the maximum number of either the functional DNA coding sequence protein's epitopes or both the functional DNA coding sequence protein and HBsAg epitopes are properly exposed. By "appropriate culture media" is meant that media which will enable the transformed host to survive and which will also enable such host to express the hybrid functional DNA coding sequence-Pre S2-S protein coding sequence protein product of the vector it is transformed with in recoverable quantity. It will be appreciated by one of skill in the art that the appropriate culture media to use will depend upon the host cell employed. The isolation of the hybrid particle of this invention from a cell lysate or extract of such host's culture is carried out by conventional protein isolation techniques.

This invention also relates to a vaccine containing the hybrid particles of this invention. Such vaccine will contain an immunoprotective quantity of the hybrid particles of this invention and is prepared by conventional techniques. By "immunoprotective" is meant that enough of the hybrid particles of this invention are administered to elicit sufficient protective antibody response against the agent desired to be protected against without serious side effects. Preferably, the vaccine of this invention comprises the micelle of this invention, i.e., a micelle comprising the particle of this invention and polysorbate. The preferred amount of polysorbate comprised by such micelle is 5-50 µg polysorbate per 100 µg protein. The micelle may be prepared by the method disclosed in European Patent Application Publication No. 0 199 698.

In the vaccine of the invention, an aqueous solution of the hybrid particles of the invention, preferably buffered at physiological pH, can be used directly. Alternatively, the particle, can be admixed or adsorbed with any of the various known adjuvants. Such adjuvants include, among others, aluminum hydroxide.

Vaccine preparation is generally described in New Trends and Developments in Vaccines, edited by Voller et al., University Park Press, Baltimore, Maryland, U.S.A., 1978.

The amount of the hybrid particle of the present invention present in each vaccine dose is selected as an amount which induces an immunoprotective response without significant, adverse side effects in typical vaccines. Such amount will vary depending upon which specific immunogen is employed and whether or not the vaccine is adjuvanted. Generally, it is expected that each dose will comprise 1-1000 µg of protein, preferably 1-200 µg. An optimal amount for a particular vaccine can be ascertained by standard studies involving observation of antibody titres and other responses in subjects. Following an initial vaccination, subjects will preferably receive a boost in about 4 weeks, followed by repeated boosts every six months for as long as a risk of infection exists.

The immune response to the hybrid particle of this invention may be enhanced by the use of adjuvant, such as, but not limited to, aluminum hydroxide (alum).

To exemplify the hybrid particle of this invention, a 64 codon DNA sequence coding for the repetitive epitope of the circumsporozoite protein (CS) of Plasmodium falciparum and an eight codon CS coding sequence [see Dame et al., Science, 225, 593-599 (1984)] were each inserted in phase to a portion of the Pre S2 coding sequence in a yeast expression vector comprising a replicon, the Pre S2-S protein coding sequence and an appropriate regulatory region. Both expression vectors, i.e., one containing the 64 codon Cs sequence and one containing the 8 codon CS sequence, were each introduced into yeast host cells, and extracts of such transformed cells were analyzed for the presence of HBsAg epitopes and CS epitopes on the same molecule.

The results obtained from immunoblot analyses using a pool of five monoclonal antibodies specific for CS and a monoclonal antibody raised against purified yeast-derived HBsAg showed that a 37,000 kilodalton (Kd) hybrid protein was obtained from lysates of yeast transformed with the vector containing the 64 codon CS coding sequence fused in phase with the Pre S2-S protein coding sequence, and a 30,000 Kd hybrid protein was obtained from lysates of yeast transformed with the vector containing the 8 codon CS coding sequence fused in phase with the Pre S2-S protein coding sequence. Assembly of these proteins into HBsAg particle-like structures was shown by CsCl and sucrose gradient analysis, high pressure liquid chromatography (HPLC) and electron microscopy. Presentation of the CS epitopes on the exterior of such particles was shown by the accessibility of the Cs epitope to CS specific antibodies in an ELISA assay. Presentation of HBsAg epitopes on the exterior of such particles was shown by radioimmune assay (AUSTRIA II, Abbott) or ELISA assay (Enzygnost-HBsAg, Behringwerke).

## NOVEL PRE S2 AND PRE S2-S PROTEIN CODING SEQUENCES

The novel HBV Pre S2 and Pre S2-S protein coding sequences of this invention are from an HBV adw subtype which was isolated from plasmid pRIT10616. The term "coding sequence" or "coding region" as used herein also encompasses any functional derivative thereof. By the term "functional derivative" is meant a coding sequence with amino acid alterations which, where appropriate, don't interfere with particle formation and/or which retain immunogenicity where such retention is desirable. Such functional derivatives can be prepared by conventional site specific mutagenesis such as by the method of Botstein et al., Science, 229, 1193-1201 (1985). Such Pre S2 and Pre S2-S protein coding sequences can be recovered by conventional recombinant DNA procedures from plasmid pRIT10616 which was deposited (within E. coli K12 strain C600) in accordance with the Budapest Treaty at the American Type Culture Collection, Rockville, Maryland, on June 2, 1982, under accession number ATCC 38131. The Pre S2 region of such novel protein coding sequences codes for the following amino acid sequence:

```
-55                         -50
Met-Gln-Trp-Asn-Ser-Thr-Ala-Phe-His-Gln-Ala-Leu-Gln-Asp-

       -40                                        -30
Pro-Arg-Val-Arg-Gly-Leu-Tyr-Phe-Pro-Ala-Gly-Gly-Ser-Ser-

                                  -20
Ser-Gly-Thr-Val-Asn-Pro-Ala-Pro-Asn-Ile-Ala-Ser-His-Ile-

        -10
Ser-Ser-Ser-Ser-Ala-Arg-Thr-Gly-Asp-Pro-Val-Thr-Asn.
```

Such region may also be obtained by conventional synthetic oligonucleotide synthesis. A preferred functional derivative of the Pre S2-S coding sequence of this invention is one wherein the alanine (GCT) amino acid at -45 is modified via site specific mutagenesis to a threonine (ACT). Such functional derivative results in two times greater level of expression by Saccharomyces cerevesiae transformed therewith as com pared to the unmodified coding sequence of this invention.

A recombinant vector of this invention comprises the Pre S2 or Pre-S2-S protein coding sequence of the subject invention operatively linked to a regulatory region. Such vector may also additionally contain a replicon depending on preference and/or the host to be employed. By the term "replicon" is meant that minimum region of DNA which functions to maintain stably, and extrachromosomally, such recombinant vector in a host eukaryotic or prokaryotic organism transformed with such vector. Such replicons are well-known in the art. By the term "regulatory region" is meant any DNA sequence which contains a promoter region and other sequences necessary for transcription of a coding sequence. Such regulatory regions are well-known in the art. Such a vector can be prepared by conventional techniques such as by inserting the Pre S2 or Pre S2-S protein coding sequence of this invention in phase into a vector already containing a replicon and regulatory region in such a way that the DNA molecule is operatively linked to such regulatory region. Preferably the DNA molecule is ligated to a vector capable of expression in yeast such as Saccharomyces.

This invention also relates to a host cell transformed with such recombinant vector and to a method of preparing such transformed host. Such transformation is carried out by conventional techniques. Host cells can be prokaryotic or eukaryotic. Preferred host cells are eukaryotic especially yeast cells and include those belonging to the genus Saccharomyces, particularly those belonging to the species Saccharomycescerevesiae and Saccharomyces carlsbergensis.

This invention also relates to a method of preparing a protein encoded by the Pre S2 or Pre S2-S pro tein coding sequence of this invention wherein such method comprises culturing the transformed host cells of this invention in appropriate culture media, and isolating the protein from the host cell culture fluid or from a cell lysate or extract of such host's culture depending upon the host cell's ability to secrete such protein. By "appropriate culture media" is meant that media which will enable the transformed host of this invention to survive and which will also enable such host to express the Pre S2 or Pre S2-S protein coding sequence of this invention in recoverable quantity. It will be appreciated by one of skill in the art that the appropriate culture media to use will depend upon the host cell employed. The isolation of the Pre S2-S protein of this invention from a culture lysate or the culture fluid of such host is carried out by conventional protein isolation techniques. The protein prepared by the method of this invention may be glycosylated depending upon the host cell's ability to do so. If a deglycosylated protein is desired, the protein of this invention should be prepared by utilizing a host cell incapable of effecting such glycosylation or by employing conventional site specific mutagenesis on the protein's coding sequence prior to carrying out the process of this invention, such as by the method of Botstein et al., Science, 229, 1193-1201 (1985).

This invention also relates to a vaccine containing an immunoprotective amount of the Pre S2 or Pre S2-S protein of this invention. Such vaccine may be prepared by conventional techniques.

This invention also relates to a method of preparing a hybrid particle containing HBsAg protein which comprises protein encoded by the PreS2-S protein coding sequence of this invention wherein such method comprises culturing the transformed eukaryotic host cells of this invention in appropriate culture media, and isolating the particle from the host cell culture fluid or from a cell lysate or extract of such host's culture depending upon the transformed host cells ability to secrete such particles. By "appropriate culture media" is meant that media which will enable the transformed host of this invention to survive and which will also enable such host to express the Pre S2-S protein coding sequence of this invention in particle form and in recoverable quantity. It will be appreciated by one of skill in the art that the appropriate culture media to use will depend upon the host cell employed. The isolation of the hybrid particle of this invention from a culture lysate or the culture fluid of such host is carried out by conventional isolation techniques.

This invention also relates to a vaccine containing the hybrid particles of this invention. Such vaccine will contain an immunoprotective quantity of the hybrid particles of this invention and are prepared by conventional techniques. Preferably, the vaccine of this invention comprises the micelle of this invention, i.e., a micelle comprising the particle of this invention and polysorbate. The preferred amount of polysorbate comprised by such micelle is 5-50 μg polysorbate per 100 μg protein. The micelle may be prepared by the method disclosed in European Patent Application Publication No. 0 199 698.

In the vaccine of the invention, an aqueous solution of the Pre S2-S protein or hybrid particle of the invention, preferably buffered at physiological pH, can be used directly. Alternatively, the protein or particle can be admixed or adsorbed with any of the various known adjuvants. Such adjuvants include, among others, aluminum hydroxide.

Vaccine preparation is generally described in New Trends and Developments in Vaccines, edited by Voller et al., University Park Press, Baltimore, Maryland, U.S.A., 1978. Encapsulation within liposomes is described, for example, by Fullerton, U.S. Patent 4,235,877. Conjugation of proteins to macromolecules is disclosed, for example, by Likhite, U.S. Patent 4,372,945 and by Armor et al., U.S. Patent 4,474,757.

The amount of the Pre S2-S protein or hybrid particle of the present invention contained in each vaccine dose is selected as an amount which induces an immunoprotective response without significant, adverse side effects in typical vaccines. Such amount will vary depending upon which specific immunogen is employed and whether or not the vaccine is adjuvanted. Generally, it is expected that each dose will comprise 1-1000 μg of protein, preferably 1-200 μg. An optimal amount for a particular vaccine can be ascertained by standard studies involving observation of antibody titres and other responses in subjects. Following an initial vaccination, subjects will preferably receive a boost in about 4 weeks, followed by repeated boosts every six months for as long as a risk of infection exists.

The immune response to the Pre S2-S protein or hybrid particle of this invention is enhanced by the use of adjuvant such as, but not limited to, alum.

## NOVEL PRE S1 PROTEIN CODING SEQUENCE

The novel HBV Pre S1 protein coding sequence of this invention is from an HBV adw subtype which was isolated from plasmid pRIT10616. The term "coding sequence" or "coding region" as used herein also encompasses any functional derivative thereof. By the term "functional derivative" is meant a coding sequence involving amino acid alterations which, when desired, don't interfere with particle formation and/or which retain immunogenicity. Such functional derivatives can be prepared by conventional site specific mutagenesis such as by the method of Botstein et al., Science, 229, 1193-1201 (1985). Such Pre S1 protein coding sequence can be recovered by conventional recombinant DNA procedures from plasmid pRIT10616 which was deposited (within E. coli K12 strain C600) in accordance with the Budapest Treaty at the American Type Culture Collection, Rockville, Maryland, on June 2, 1982 under accession number ATCC 38131. The Pre S1 region codes for the following amino acid sequence:

```
ATG GGG ACG AAT CTT TCT GTT CCC AAC CCT CTG
Met Gly Thr Asn Leu Ser Val Pro Asn Pro Leu


GGA TTC TTT CCC GAT CAT CAG TTG GAC CCT
Gly Phe Phe Pro Asp His Gln Leu Asp Pro


GCA TTC GGA GCC AAC TCA AAC AAT CCA GAT
Ala Phe Gly Ala Asn Ser Asn Asn Pro Asp


TGG GAC TTC AAC CCC ATC AAG GAC CAC TGG
Trp Asp Phe Asn Pro Ile Lys Asp His Trp


CCA GCA GCC AAC CAG GTA GGA GTG GGA GCA
Pro Ala Ala Asn Gln Val Gly Val Gly Ala


TTC GGG CCA GGG CTC ACC CCT CCA CAC GGC
Phe Gly Pro Gly Leu Thr Pro Pro His Gly


GGT ATT TTG GGG TGG AGC CCT CAG GCT CAG
Gly Ile Leu Gly Trp Ser Pro Gln Ala Gln


GGC ATA TTG ACC ACA GTG TCA ACA ATT CCT
Gly Ile Leu Thr Thr Val Ser Thr Ile Pro


CCT CCT GCC TCC ACC AAT CGG CAG TCA GGA
Pro Pro Ala Ser Thr Asn Arg Gln Ser Gly


AGG CAG CCT ACT CCC ATC TCT CCA CCT CTA
Arg Gln Pro Thr Pro Ile Ser Pro Pro Leu


AGA GAC AGT CAT CCT CAG GCC
Arg Asp Ser His Pro Gln Ala
```

Such region may also be obtained by conventional synthetic oligonucleotide synthesis.

A recombinant vector of this invention comprises the Pre S1 protein coding sequence of the subject invention operatively linked to a regulatory region and may additionally comprise a replicon depending on preference and/or the host to be employed. By the term "replicon" is meant that minimum region of DNA which functions to maintain stably, and extrachromosomally, such recombinant vector in a host eukaryotic or prokaryotic organism transformed with such vector. Such replicons are well-known in the art. By the term "regulatory region" is meant a DNA sequence which contains a promoter region and other sequences necessary for transcription of a coding sequence. Such regulatory regions are well-known in the art. Such a vector can be prepared by conventional techniques such as by inserting the Pre S1 protein coding sequence of this invention in phase into a vector already containing a replicon and regulatory region in such a way that the DNA molecule is operatively linked to such regulatory region. Preferably the DNA molecule is ligated to a vector capable of expression in yeast such as Saccharomyces.

This invention also relates to a host cell transformed with such recombinant vector and to a method of preparing such transformed host. Such transformation is carried out by conventional techniques. Host cells can be prokaryotic or eukaryotic. Preferred host cells are eukaryotic especially yeast cells and include those belonging to the genus Saccharomyces, particularly those belonging to the species Saccharomyces cerevisiae and Saccharomyces carlsbergensis.

This invention also relates to a method of preparing protein encoded by the Pre S1 protein coding sequence of this invention wherein such method comprises culturing the transformed host cells of this invention in appropriate culture media, and isolating the protein from the host cell culture fluid or from a cell lysate or extract of such host's culture depending upon the transformed host cell's ability to secrete such protein. By "appropriate culture media" is meant that media which will enable the transformed host of this invention to survive and which will also enable such host to express the Pre S1 protein coding sequence of this invention in recoverable quantity. It will be appreciated by one of skill in the art that the appropriate culture media to use will depend upon the host cell employed. The isolation of the Pre S1 protein of this invention from a culture lysate or the culture fluid of such host is carried out by conventional isolation techniques. The protein prepared by the method of this invention may be glycosylated depending upon the host cell's ability to do so. If a deglycosylated protein is desired, the protein of this invention should be prepared by utilizing a host cell incapable of effecting such glycosylation or by employing conventional site specific mutagenesis on the protein's coding sequence prior to carrying out the process of this invention. Site specific mutagenesis is carried out by such as by the method of Botstein et al., Science, 229, 1193-1201 (1985).

This invention also relates to a vaccine containing the Pre S1 protein of this invention. Such vaccine will contain an immunoprotective quantity of the Pre S1 protein of this invention and is prepared by conventional techniques.

In the vaccine of the invention, an aqueous solution of the Pre S1 protein of the invention, preferably buffered at physiological pH, can be used directly. Alternatively, the Pre S1 protein, can be admixed or adsorbed with any of the various known adjuvants. Such adjuvants include, among others, aluminum hydroxide.

Vaccine preparation is generally described in New Trends and Developments in Vaccines, edited by Voller et al., University Park Press, Baltimore, Maryland, U.S.A., 1978. Encapsulation within liposomes is described, for example, by Fullerton, U.S. Patent 4,235,877. Conjugation of proteins to macromolecules is disclosed, for example, by Likhite, U.S. Patent 4,372,945 and by Armor et al., U.S. Patent 4,474,757.

The amoung of the Pre S1 protein of the present invention present in each vaccine dose is selected as an amount which induces an immunoprotective response without significant, adverse side effects in typical vaccines. Such amount will vary depending upon which specific immunogen is employed and whether or not the vaccine is adjuvanted. Generally, it is expected that each dose will comprise 1-1000 μg of protein, preferably 1-200 μg. An optimal amount for a particular vaccine can be ascertained by standard studies involving observation of antibody titres and other responses in subjects. Following an initial vaccination, subjects will preferably receive a boost in about 4 weeks, followed by repeated boosts every six months for as long as a risk of infection exists. The immune response to the PreS1 protein of this invention is enhanced by the use of adjuvant such as, but not limited to, alum.

## NOVEL PRES1 - PRES2 - S PROTEIN CODING SEQUENCE

This invention also relates to a HBV PreS1-PreS2-S protein coding sequence of which the PreS1-PreS2 portion comprises the following amino acid sequence:

PRE-S1 REGION
-163

ATG GGG ACG AAT CTT TCT GTT CCC AAC CCT CTG
Met Gly Thr Asn Leu Ser Val Pro Asn Pro Leu

GGA TTC TTT CCC GAT CAT CAG TTG GAC CCT
Gly Phe Phe Pro Asp His Gln Leu Asp Pro

GCA TTC GGA GCC AAC TCA AAC AAT CCA GAT
Ala Phe Gly Ala Asn Ser Asn Asn Pro Asp

TGG GAC TTC AAC CCC ATC AAG GAC CAC TGG
Trp Asp Phe Asn Pro Ile Lys Asp His Trp

CCA GCA GCC AAC CAG GTA GGA GTG GGA GCA
Pro Ala Ala Asn Gln Val Gly Val Gly Ala

TTC GGG CCA GGG CTC ACC CCT CCA CAC GGC
Phe Gly Pro Gly Leu Thr Pro Pro His Gly

GGT ATT TTG GGG TGG AGC CCT CAG GCT CAG
Gly Ile Leu Gly Trp Ser Pro Gln Ala Gln

GGC ATA TTG ACC ACA GTG TCA ACA ATT CCT
Gly Ile Leu Thr Thr Val Ser Thr Ile Pro

CCT CCT GCC TCC ACC AAT CGG CAG TCA GGA
Pro Pro Ala Ser Thr Asn Arg Gln Ser Gly


AGG CAG CCT ACT CCC ATC TCT CCA CCT CTA
Arg Gln Pro Thr Pro Ile Ser Pro Pro Leu


AGA GAC AGT CAT CCT CAG GCC
Arg Asp Ser His Pro Gln Ala

PRE-S2 REGION


-55

ATG CAG TGG AAT TCC ACT GCC TTC CAC CAA
Met Gln Trp Asn Ser Thr Ala Phe His Gln


GCT CTG CAG GAT CCC AGA GTC AGG GGT CTG
Ala Leu Gln Asp Pro Arg Val Arg Gly Leu


TAT TTT CCT GCT GGT GGC TCC AGT TCA GGA
Tyr Phe Pro Ala Gly Gly Ser Ser Ser Gly


ACA GTA AAC CCT GCT CCG AAT ATT GCC TCT
Thr Val Asn Pro Ala Pro Asn Ile Ala Ser


CAC ATA TCG TCA AGC TCC GCG AGG ACT GGG
His Ile Ser Ser Ser Ser Ala Arg Thr Gly


GAC CCT GTG ACG AAC
Asp Pro Val Thr Asn


The novel HBV Pre S1-Pre S2-S protein coding sequence of this invention is from an HBV adw subtype which was isolated from plasmid pRIT10616 which, as stated above, is available from the American Type Culture Collection, Rockville, Maryland, under accession number ATCC 38131. The term "coding sequence" or "coding region" as used herein also encompasses any functional derivative thereof. By the term "functional derivative" is meant a coding sequence with amino acid alterations which, where appropriate, don't interfere with particle formation and/or which retain immunogenicity where such retention is desirable. Such functional derivatives can be prepared by conventional site specific mutagenesis such as by the method of Botstein et al., Science, 229, 1193-1201 (1985).

A recombinant vector of this invention comprises the Pre-S1-Pre S2-S protein coding sequence of the subject invention operatively linked to a regulatory region. Such vector may also additionally contain a replicon depending upon preference and/or the host to be employed. By the term "replicon" is meant that minimum region of DNA which functions to maintain stably, the extrachromosomally, such recombinant vector in a host eukaryotic or prokaryotic organism transformed with such vector. Such replicons are well-known in the art. By the term "regulatory region" is meant any DNA sequence which contains a promoter region and other sequences necessary for transcription of a coding sequence which regulates transcription of a structural

**0 278 940**

gene's coding sequence. Such regulatory regions are well-known in the art. Such a vector can be prepared by conventional techniques such as by inserting the Pre S1- Pre S2-S-protein coding sequence of this invention in phase into a vector already containing a replicon and regulatory region in such a way that the DNA molecule is operatively linked to such regulatory region. Preferably the DNA molecule is ligated to a vector capable of expression in yeast such as Saccharomyces.

This invention also relates to a host cell transformed with such recombinant vector and to a method of preparing such transformed host. Such transformation is carried out by conventional techniques. Host cells can be prokaryotic or eukaryotic. Preferred host cells are eukaryotic expecially yeast cells and include those belonging to the genus Saccharomyces, particularly those belonging to the species Saccharomyces cerevisiae and Saccharomyces carlsbergensis.

This invention also relates to a method of preparing a protein encoded by the Pre S1-Pre S2-S protein coding sequence of this invention wherein such method comprises culturing the transformed host cells of this invention in appropriate culture media, and isolating the protein from the host cell culture fluid or from a cell lysate or extract of such host's culture depending upon the host cell's ability to secrete such protein. By "appropriate culture media" is meant that media which will enable the transformed host of this invention to survive and which will also enable such host to express the Pre-S1 Pre S2-S protein coding sequence product of this invention in recoverable quantity. It will be appreciated by one of skill in the art that the appropriate culture media to use will depend upon the host cell employed. The isolation of the Pre S1-Pre S2-S protein of this invention from a culture lysate or the culture fluid of such host is carried out by conventional protein isolation techniques. The protein prepared by the method of this invention may be glycosylated depending upon the host cell's ability to do so. If a deglycosylated protein is desired, the protein of this invention should be prepared by utilizing a host cell incapable of effecting such glycosylation or by employing conventional site specific mutagenesis on the protein's coding sequence prior to carrying out the process of this invention, such as by the method of Botstein et al., Science, 229, 1193-1201 (1985). The Pre S1-Pre S2-S coding sequence of this invention will be expressed as a glycosylated protein by a yeast or mammalian host cell transformed therewith. The Pre S1-Pre S2-S coding sequence of this invention will be expressed as a N-myristylated protein by a yeast host cell transformed therewith.

This invention also relates to a vaccine containing an immunoprotective amount of the Pre S1- Pre S2-S protein of this invention. Such vaccine may be prepared by conventional techniques.

This invention also relates to a method of preparing a hybrid particle containing HBsAg protein which comprises protein encoded by the Pre S1-PreS2-S protein coding sequence of this invention wherein such method comprises culturing the transformed eukaryotic host cells of this invention in appropriate culture media, and isolating the particle from the host cell culture fluid or from a cell lysate or extract of such host's culture depending upon the transformed host cells ability to secrete such protein. By "appropriate culture media" is meant that media which will enable the transformed host of this invention to survive and which will also enable such host to express the pre S1-Pre S2-S protein coding sequence of this invention in particle form and in recoverable quantity. It will be appreciated by one of skill in the art that the appropriate culture media to use will depend upon the host cell employed. The isolation of the particle of this invention from a culture lysate or the culture fluid of such host is carried out by conventional isolation techniques.

This invention also relates to a vaccine containing the hybrid particles of this invention. Such vaccine will contain an immunoprotective quantity of the hybrid particles of this invention and are prepared by conventional techniques. Preferably, the vaccine of this invention comprises the micelle of this invention, i.e., a micelle comprising the particle of this invention and polysorbate. The preferred amount of polysorbate comprised by such micelle is 5-50 μg polysorbate per 100 μg protein. The micelle may be prepared by the method disclosed in European Patent Application Publication No. 0 199 698.

In the vaccine of the invention, an aqueous solution of the Pre S1-Pre S2-S protein or the hybrid particle of the invention, preferably buffered at physiological pH, can be used directly. Alternatively, the protein or particle can be admixed or adsorbed with any of the various known adjuvants. Such adjuvants include, among others, aluminum hydroxide.

Vaccine preparation is generally described in New Trends and Developments in Vaccines, edited by Voller et al., University Park Press, Baltimore, Maryland, U.S.A., 1978. Encapsulation within liposomes is described, for example, by Fullerton, U.S. Patent 4,235,877. Conjugation of proteins to macromolecules is disclosed, for example, by Likhite, U.S. Patent 4,372,945 and by Armor et al., U.S. Patent 4,474,757.

The amount of the Pre S1-Pre S2-S protein or hybrid particle of the present invention present in each vaccine dose is selected as an amount which induces an immunoprotective response without significant, adverse side effects in typical vaccines. Such amount will vary depending upon which specific immunogen is employed and whether or not the vaccine is adjuvanted. Generally, it is expected that each dose will comprise 1-1000 μg of protein, preferably 1-200 μg. An optimal amount for a particular vaccine can be ascertained by standard studies involving observation of antibody titres and other responses in subjects. Following an initial vaccination, subjects will preferably receive a boost in about 4 weeks, followed by repeated boosts every six months for as long as a risk of infection exists.

The immune response to the Pre S1-Pre S2-S protein or hybrid particle of this invention is enhanced by the use of adjuvant such as, but not limited to, alum.

22

## EXPRESSION OF PRES1-PRES2-S PROTEIN IN YEAST

This invention also relates to a recombinant DNA vector comprising an entire PreS1-PreS2-S protein coding sequence operatively linked to an expression control sequence. The term "coding sequence" or "coding region" as used herein also encompasses any functional derivative thereof. By the term "functional derivative" is meant a coding sequence with amino acid alterations which, where appropriate, don't interfere with particle formation and/or which retain immunogenicity where such retention is desirable. Such functional derivatives can be prepared by conventional site specific mutagenesis such as by the method of Botstein et al., Science, 229, 1193-1201 (1985). Such vector may additionally comprise a replicon depending upon preference and/or the host to be employed. Preferably, the PreS1-PreS2-S protein coding sequence employed is the one of this invention.

By the term "replicon" is meant that minimum region of DNA which functions to maintain stably, and extrachromosomally, such recombinant vector in a host yeast organism transformed with the vector of this invention. Such replicons are well-known in the art. By the term "regulatory region" is meant any DNA sequence which contains a promoter region and other sequences necessary for transcription of a coding sequence which regulates transcription of a structural gene's coding sequence. Such regulatory regions are well-known in the art. Such a vector can be prepared by conventional techniques such as by inserting a PreS1-PreS2-S protein coding sequence in phase into a vector already containing a replicon and regulatory region in such a way that the DNA molecule is operatively linked to such regulatory region. Preferably the DNA molecule is ligated to a vector capable of expression in the yeast genus Saccharomyces. Preferably the Pre S1- Pre S2-S protein coding sequence contained by the vector of this invention is the Pre S1- Pre S2-S protein coding sequence of this invention described hereinabove. Such vectors are also useful for the insertion therein of functional DNA coding sequences to enable expression of the resulting Pre S1-functional DNA coding sequence-Pre S2-S protein coding sequence comprised by such vector. Therefore, this invention also relates to a recombinant DNA molecule comprising a functional DNA coding sequence fused in phase into the Pre S1 region of the Pre S1-Pre S2-S protein coding sequence, in such a way that the functional DNA sequence either forms an insertion in the Pre S1 region or replaces part of the Pre S1 region and possibly a portion of the Pre S2 region, and to a vector comprising such recombinant DNA molecule. See, for example, Example 21A, infra. By the term "functional DNA coding sequence" is meant a DNA coding sequence which, when fused, in phase, into the Pre S1 region of the Pre S1-Pre S2-S protein coding sequence, is not involved with, and does not interfere with, the assembly of the HBsAg particle. Preferred functional DNA coding sequences include but are not limited to, the circumsporozoite coding sequence of Plasmodium or any immunogenic derivative thereof, the envelope gene coding sequence of HIV or any immunogenic derivative thereof, especially the $C_7$ peptide, peptide 121 or Dreesman peptide coding sequence as hereinafter exemplified, or sequences coding for peptides of interest from other viruses expecially those for coat proteins or for surface proteins from other parasites.

This invention also relates to a yeast host cell transformed with such recombinant vector and to a method of preparing such transformed host. Such transformation is carried out by conventional techniques. Preferred yeast cells and include those belonging to the genus Saccharomyces, particularly those belonging to the species Saccharomyces cerevisiae.

This invention also relates to a method of preparing a protein encoded by a Pre S1-Pre S2-S protein coding sequence or Pre S1-functional DNA coding sequence - Pre S2-S protein coding sequence wherein such method comprises culturing the transformed host cells of this invention in appropriate culture media, and isolating the protein from the host cell culture fluid or from a cell lysate or extract of such host's culture depending upon the host cell's ability to secrete such protein. By "appropriate culture media" is meant that media which will enable the transformed host of this invention to survive and which will also enable such host to express a Pre S1-Pre S2-S protein coding sequence or Pre S1-functional DNA coding sequence - Pre S2-S protein coding sequence product in recoverable quantity. It will be appreciated by one of skill in the art that the appropriate culture media to use will depend upon the host cell employed. The isolation of the Pre S1-Pre S2-S protein or Pre S1-functional DNA coding sequence - Pre S2-S protein from a cell lysate or the culture fluid of such host culture is carried out by conventional protein isolation techniques. The protein prepared by the method of this invention may be glycosylated depending upon the host cell's ability to do so. If a deglycosylated protein is desired, the protein of this invention should be prepared by utilizing a host cell incapable of effecting such glycosylation or by employing conventional site specific mutagenesis on the protein's coding sequence prior to carrying out the process of this invention, such as by the method of Botstein et al., Science, 229, 1193-1201 (1985).

This invention also relates to a vaccine containing an immunoprotective amount of Pre-S1-Pre S2-S protein or Pre S1- functional DNA coding sequence - Pre S2-S protein produced by the method of this invention. Such vaccine may be prepared by conventional techniques.

This invention also relates to a method of preparing a hybrid particle which comprises protein encoded by a PreS1-PreS2-S protein coding sequence or Pre S1-functional DNA coding sequence - Pre S2-S protein coding sequence, wherein such method comprises culturing the transformed yeast host cells of this invention in appropriate culture media, and isolating the particle from the host cell culture fluid or from a cell lysate or extract of such host's culture depending upon the transformed host cells ability to secrete such particles. By "appropriate culture media" is meant that media which will enable the transformed host to survive and which will also enable such host to express the Pre S1-Pre S2-S protein coding sequence or Pre S1-functional DNA coding sequence - Pre S2-S protein coding sequence in particle form and in recoverable quantity. It will be be appreciated by one of skill in the art that the appropriate culture media to use will depend upon the host cell employed. The isolation of the particles of this invention from a culture lysate or the culture fluid of such host is carried out by conventional isolation techniques.

This invention also relates to a vaccine containing the particles of this invention. Such vaccine will contain an immunoprotective quantity of the particles of this invention and are prepared by conventional techniques. Preferably, the vaccine of this invention comprises the micelle of this invention, i.e., a micelle comprising the particle of this invention and polysorbate. The preferred amount of polysorbate comprised by such micelle is 5-50 μg polysorbate per 100 μg protein. The micelle may be prepared by the method disclosed in European Patent Application Publication No. 0 199 698.

In the vaccine of the invention, an aqueous solution of the Pre S1-Pre S2-S protein or Pre S1 functional DNA coding sequence - Pre S2-S protein or the particle of the invention, preferably buffered at physiological pH, can be used directly. Alternatively, the protein particle can be admixed or adsorbed with any of the various known adjuvants. Such adjuvants include, among others, aluminum hydroxide.

Vaccine preparation is generally described in New Trends and Developments in Vaccines, edited by Voller et al., University Park Press, Baltimore, Maryland, U.S.A., 1978. Encapsulation within liposomes is described, for example, by Fullerton, U.S. Patent 4,235,877. Conjugation of proteins to macromolecules is disclosed, for example, by Likhite, U.S. Patent 4,372,945 and by Armor et al., U.S. Patent 4,474,757.

The amount of the Pre S1-Pre S2-S protein or Pre S1-functional DNA coding sequence - Pre S2-S protein or particle of the present invention present in each vaccine dose is selected as an amount which induces an immunoprotective response without significant, adverse side effects in typical vaccines. Such amount will vary depending upon which specific immunogen is employed and whether or not the vaccine is adjuvanted. Generally, it is expected that each dose will comprise 1-1000 μg of protein, preferably 1-200 μg. An optimal amount for a particular vaccine can be ascertained by standard studies involving observation of antibody titres and other responses in subjects. Following an initial vaccination, subjects will preferably receive a boost in about 4 weeks, followed by repeated boosts every six months for as long as a risk of infection exists.

The immune response to the Pre S1-Pre S2-S protein or Pre S1-functional DNA coding sequence - Pre S2-S protein or particle of this invention is enhanced by the use of adjuvant such as, but not limited to, alum.

EXAMPLES

In the following examples of the invention, which are illustrative and not limiting
- All percentages are by weight and all temperatures are in degrees Celsius (Centigrade).
- The enzymes used in DNA manipulations were obtained from Bethesda Research Laboratories, New England Biolabs, and/or Boehringer, and were employed according to the suppliers' directions.
- Yeast growth media : selective medium : YNB (yeast nitrogen base without amino acids (Difco Labs) 0.675 % (w/v) containing 2 % (w/v) glucose).
- YEPD medium : 1 % (w/v) yeast extract, 2 % w/v peptone and 2 % (w/v) glucose.
- DNA recombinant methods are described in "Molecular Cloning", T. Maniatis et al., Cold Spring Harbor Lab. (1982).
- PMSF : phenyl methyl sulphonyl fluoride
- The following abbreviations may be employed:

PBS : Phosphate Buffered Saline (per liter): (pH 7.4)
8.0 gram NaCl
0.2 gram KCl
1.15 gram $Na_2HPO_4$
0.2 gram $KH_2PO_4$
0.1 gram $CaCl_2$
0.1 gram $MgCl_2$ • $6H_2O$

BSA: Bovine Serum Albumin ($A_{4378}$, Sigma)
X-gal : 5-bromo-4-chloroindoyl-β-D-galactopyranoside, commercially available from Sigma Chemical Co., St. Louis, Missouri.

L-BROTH (per liter):
10 gram Tryptone
5 gram NaCl
5 gram Yeast extract
1 ml 0.1M MgSO4
Add 5 ml of aseptic solution of thiamine (1 mg/ml) after autoclaving. For solid media, add 15 g agar per liter.

Example 1. Construction of plasmid pRIT10167.

The starting material was plasmid p6y containing a 2.1 kb HindIII fragment of yeast DNA encoding the TDH3 gene cloned on pBR322. pBR322 is described by Bolivar et al. (Gene, 2, 95-113, 1977). The p6y plasmid was constructed and characterized by Musti et al. (Gene, 25, 133, 1983) and received from Dr. M. Rosenberg of the National Institute of Health. The HindIII fragment was recloned onto a pBR322 derivative in which the EcoRI site had been destroyed to give plasmid pRIT10164 (a non essential manouver). The following manipulations were carried out so as to create a BamHI site located at the G residue of the ATG codon of the TDH3 coding sequence and to obtain a HindIII-BamHI DNA fragment with TDH3 promoter activity in which the TDH3 sequences are intact and unchanged by the manipulation. DNA of pRIT10164, prepared as described above, was purified by two cycles of CsCl - ethidium bromide density gradient centrifugation essentially as described by Kahn et al. (Methods in Enzymology, 68, 268, 1979). 150 µg of pRIT10164 DNA were digested to completion with 75 units of XbaI endonuclease, extracted with phenol and ether, precipitated with ethanol and the DNA resuspended in 0.01 M tromethamine-HCl buffer at a concentration of 1 µg per µl. Samples of 20 µg of this XbaI treated DNA were digested with Bal31 nuclease to resect the 61 base pairs of DNA between the ATG codon and the XbaI site. Digestons with Bal31 were carried out at 30° for 1 to 3 minutes in buffer containing 600 mM NaCl, 12 mM CaCl2, 12 mM MgCl2, 1 mM EDTA, 20 mM tromethamine-HCl, pH3.1 using one unit of Bal31 nuclease per 20 µg DNA in a final reaction volume of 200 µl.

The enzyme reactions were stopped by addition of ethylenebis (oxyethylenemtrilo) tetraacetic acid (EGTA) to give 20 mM final concentration and the samples were extracted with equal volumes of phenol, ether and precipitated with ethanol. Each DNA sample was resuspended in 20 µl 10 mM tromethamine-HCl buffer pH 7.5. The extent of Bal31 digestion was measured by digesting about 2.5 µg of each DNA sample with HpaI endonuclease and comparing the size of the HpaI-XbaI fragments to the 335 bp HpaI-XbaI fragment from pRIT10164. A 2 minute digestion with Bal31 was found to remove about 41 to 88 nucleotides from the XbaI-HpaI fragment of pRIT10164.

This result indicates that a similar number of base pairs had been removed from the other XbaI extremity towards the ATG codon. 5 µg of the DNA recovered from the 2 minute Bal31 digest was digested with BamHI endonuclease, extracted with phenol and recovered by ethanol precipitation. This DNA was treated with 5 units of T4 polymerase in the presence of deoxynucleotide triphosphates to fill in and render flush ended the BamHI and any Bal31 single stranded extremities, extracted with phenol and recovered by ethanol precipitation. 2.3 µg of this DNA was treated with 5 units of T4 DNA ligase and half of the ligation mixture used to transform competent cells of E. coli K12 strain MM294 prepared according to the method of Cohen et al. (Proc. Natl. Acad. Sci. 69, 2110, 1972). One ml of the transformed E. coli population was diluted into 350 ml L-broth with 200 µg/ml ampicillin and total plasmid DNA was purified from the resulting culture.

80 µg of this plasmid DNA containing a population of plasmid molecules with Bal31 induced deletions of about 40 to 80 base pairs was digested sequentially wigh 75 units of HindIII endonuclease and 96 units of BamHI endonuclease to release DNA fragments from those plasmids in which a BamHI site had been recreated after the treatments described above, that is, where the Bal31 digestion had terminated at a G residue. This DNA digest was run on a preparative 1% agarose gel and the desired HindIII-BamHI fragments corresponding to sizes from 1100 to 1000 bp excised from the gel in two slices, one corresponding to DNA of about 1070 bp and the other of about 1030 bp. The DNA was recovered from the two agarose gel slices by cycles of freezing and thawing followed by high speed centrifugation to pellet the agarose. The supernatant liquid was filtered through a Millipore GV Millex filter and the DNA recovered by two cycles of ethanol precipitation and resuspended in 20 µl of 0.01 M tromethamine-HCl buffer pH 7.5.

Analysis by agarose gel electrophoresis and comparison to a HindIII plus XbaI digest of pRIT10164 DNA and to the fragments from a HindIII plus EcoRI digest of phage λ DNA showed that two distinct populations of HindIII-BamHI fragments were obtained, one with an estimated size of about 1070 bp and one with an estimated size of about 1030 bp compared to the 1120 bp parental HindIII-XbaI fragment from pRIT10164. About 100 ng of the 1030 bp HindIII-BamHI fragment population was ligated with 200 ng of plasmid pUC9 which had been digested with HindIII and BamHI endonucleases and treated with alkaline phosphatase (See, Shine, U.S. Patent No. 4,264,731) and the ligation mixture was used to transform competent cells of E. coli strain JM103 with selection being made for ampicillin resistance. Transformation of E. coli strain JM103 was effected according to the method of Cohen et al., cited above.

Both the pUC9 vector plasmid and the recipient E. coli strain JM103 have been described by Vieira and Messing in Gene, 19, 259, 1982 and were obtained from J. Messing (Univ. of Minnesota). The pUC9 vector is commercially available from Amersham (Buckinghamshire, United Kingdom) and Pharmacia (Uppsala, Sweden). E. coli strain JM103 is available from J. Messing (Univ. of Minnesota). Another E. coli strain with the characteristics of E. coli strain JM103, i.e., JM101, can be obtained from the American Type Culture Collection, Rockville, Maryland, under accession number ATCC 33876, and can also be employed as host. About 400 ampicillin resistant colonies per ml were obtained and of 98 colonies tested on medium containing X-gal. 95 were white indicating the successful insertion of a foreign DNA fragment between the HindIII and BamHI sites on the vector.

Plasmids from individual transformant colonies were prepared by a small scale procedure (Birnboim and Doly, Nucl. Acid Res., 7, 1513, 1979) after amplification of the plasmids by addition of spectinomycin (150 µg/ml) to the growing cultures.

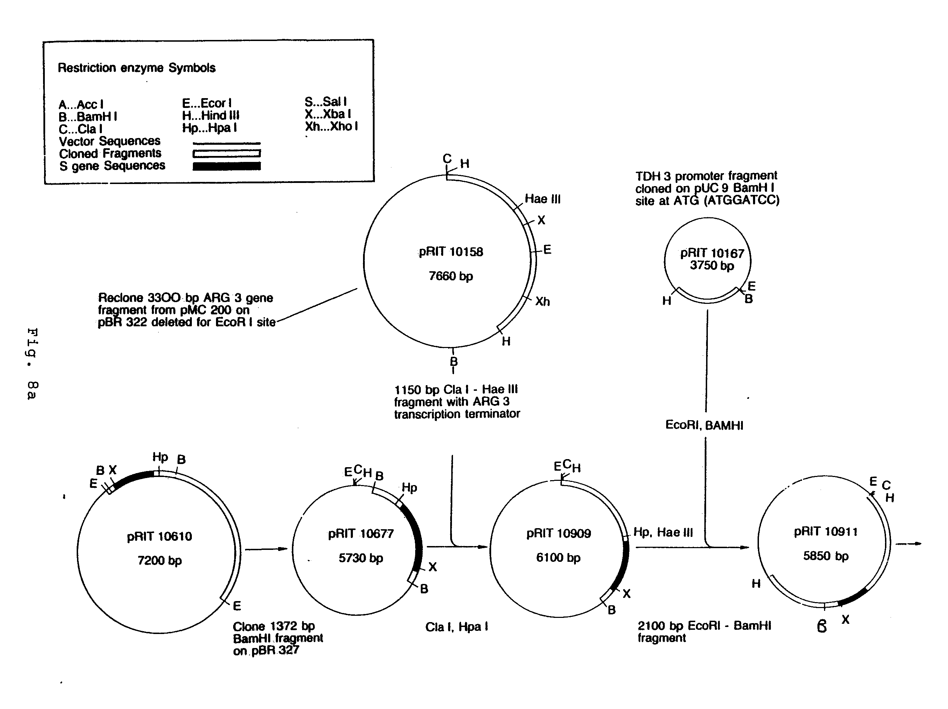

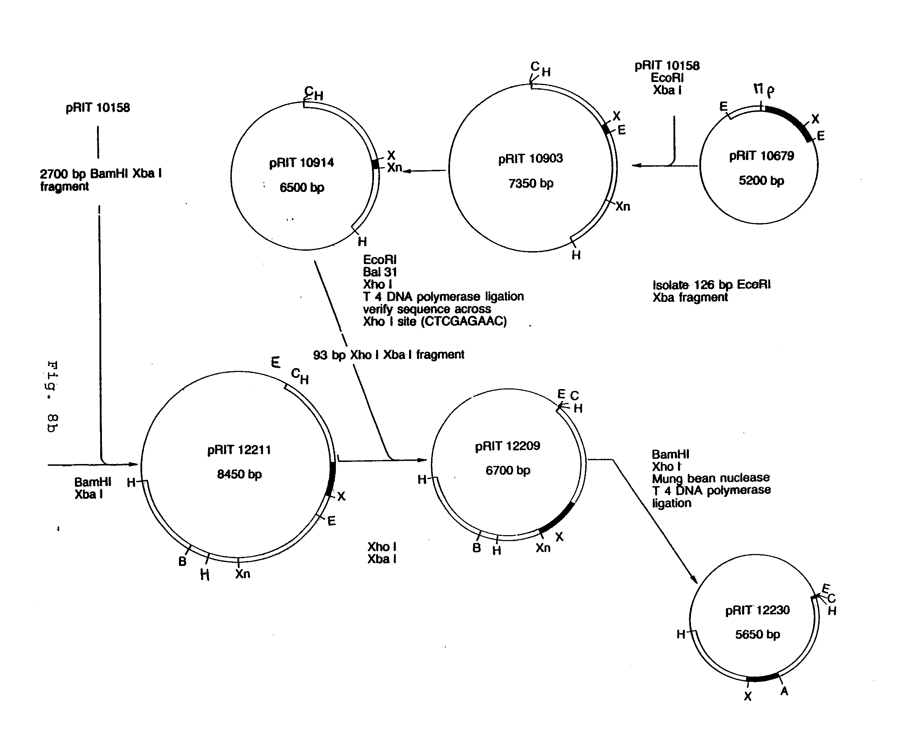

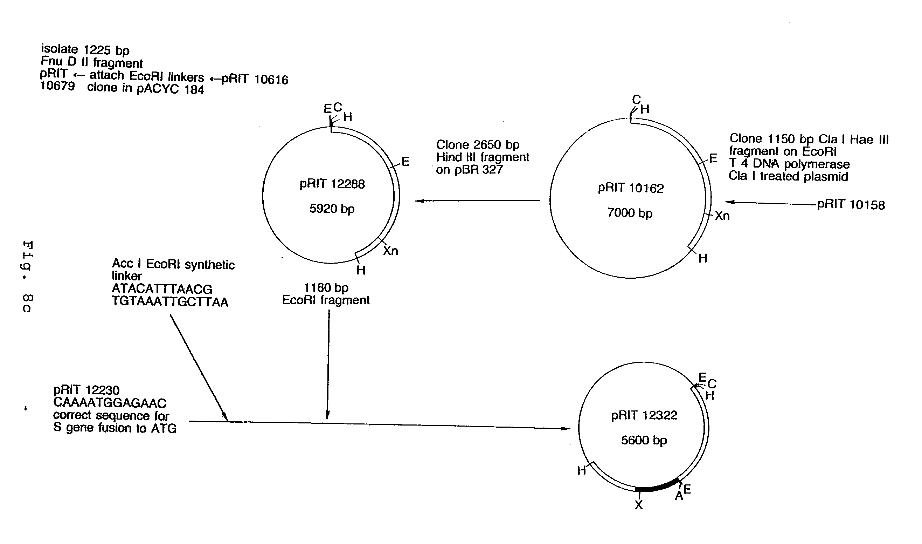

The recombinant plasmids were analyzed on 7.5% acrylamide gels after double digestion with AvaII and BamHI endonucleases and compared to the 450 bp AvaIIXbaI fragment comprising the promoter-N-terminus coding region of pRIT10164 and to the fragments of a HpaII digest of pBR322 DNA. An AvaII-BamHI fragment corresponding to deletions of from 20 to 90 base pairs was present in 35 of 36 plasmids examined when compared to pRIT10164. Three plasmids representing deletions of about 80 base pairs (pRIT10166) 50 base pairs (pRIT10167-Figure 8) and 45 base pairs (pRIT10165) were chosen for further study. Plasmid DNA was prepared by CsCl-ethidium bromide density centrifugation and 25 µg of each digested with EcoRI. The EcoRI extremities were labelled with $\gamma$-$^{32}$P-ATP by exchange kination and the nucleotide sequence of the HindIII-EcoRI fragments from each plasmid determined by the chemical modification methods of Maxam and Gilbert (Methods in Enzymology, 65, 499, 1980). Labelling of and sequencing from the EcoRI site in the pUC9 vector portion of each recombinant plasmid is a convenient means of determining the sequence around the adjacent BamHI site marking the end of the deletion into the TDH3 DNA frag- ment. This sequencing analysis showed that in plasmid pRIT10166, the BamHI site is recreated at a G residue located in the 5' non translated region 25 base pairs upstream of the ATG codon; in pRIT10165 the BamHI site is located at the second base of the third codon; and in pRIT10167 the BamHI site is located at the G residue of the ATG codon.

The HindIII-BamHI fragment of TDH3 DNA thus constructed in pRIT10167 contains all the sequences necessary for TDH3 promoter activity in an unaltered form.

Example 2. Construction of vector pRIT10172.

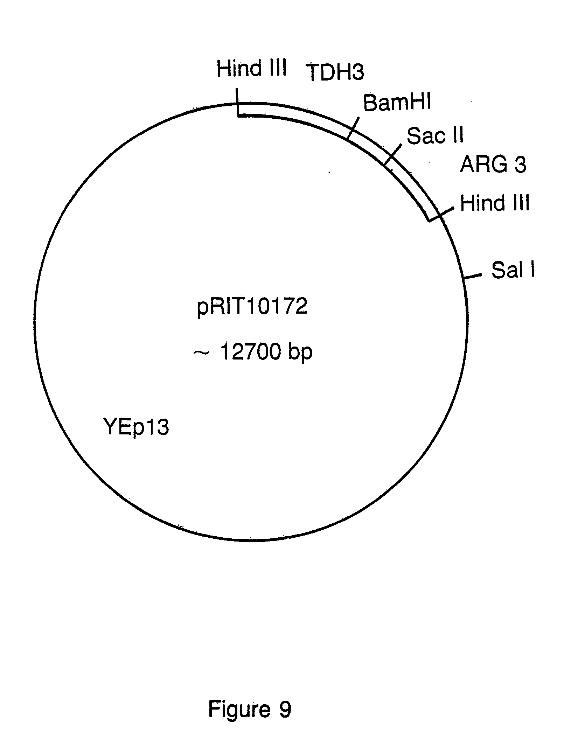

The 1050 bp HindIII-BamHI TDH3 DNA insert from pRIT10167, prepared as described in Example 1, was recloned onto the YEp13 shuttle vector described by Broach et al. (Gene, 8, 121, 1979) between the HindIII site in the 2 micron DNA portion of the vector and the BamHI site to give the recombinant plasmid pRIT12159. DNA of pRIT12159 was then digested with XbaI and BamHI endonucleases and the resulting 1650 bp fragment was ligated in place of a similar XbaI-BamHI fragment containing the arg$^3$ promoter on plasmid pRIT10774. Plasmid pRIT10774 is described by Cabezon et al. (Proc. Natl. Acad. Sci. U.S., 81, 6594-6598, 1986), and comprises the YEp13 replicon from which the natural vector's BamHI and XhoI sites have been deleted by in vitro manipulation together with a 1470 bp HindIII-BamHI insert comprising the arg$^3$ promoter region and a 1150 bp BamHI-HindIII fragment comprising the arg$^3$ transcription termination region. Replacement of the arg$^3$ promoter insert on pRIT10774 by the TDH3 promoter insert gives plasmid pRIT10172 which therefore contains a single BamHI site located at the ATG codon of the TDH3 promoter insert and preceeding a functional signal for transcription termination on the 1150 bp BamHI-HindIII arg$^3$ DNA fragment. Foreign DNA can therefore be cloned at this site. Furthermore the two DNA inserts are bounded by HindIII sites enabling removal of the expression unit module (cassette) as a 2200 bp HindIII fragment for insertion on other vectors. Figure 9 is a restriction endonuclease cleavage map of pRIT10172.

Example 3. Construction of pRIT12290.

A 1050 bp HindIII-EcoRI fragment from pRIT10167 (Figure 8), prepared as described in Example 1, and containing the HindIII-BamHI TDH3 promoter fragment together with the BamHI-SmaI-EcoRI part of the pUC9 polylinker was recloned between the HindIII and EcoRI sites of a pBR322 derivative plasmid previously deleted fro the BamHI site by filling in with T4 DNA polymerase and religation. The resulting recombinant plasmid was identified as pRIT12176.

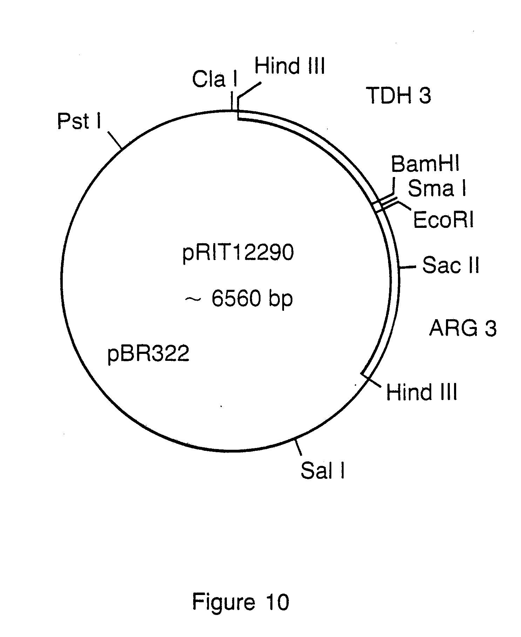

Plasmid DNA of pRIT 10158 (Figure 8), prepared as described in Example 4 (b), below, was digested with EcoRI endonuclease and treated with T4 DNA polymerase to fill in the EcoRI single strand extensions. This preparation was further digested with ClaI endonuclease. A further sample of pRIT10158 DNA was digested with ClaI and HaeIII endonucleases and a 1150 bp ClaIHaeIII fragment carrying the transcription termination region of the arg$^3$ gene was recovered by preparative agarose gel electrophoresis and electroelution. This fragment was ligated with the EcoRI, T4 DNA poly merase, ClaI treated pRIT10158 DNA, and the ligation mixture was used to transform competent cells of E. coli strain MM294 prepared as described by Cohen et al., above, to ampicillin resistance. From the transformed colonies, a plasmid, identified as pRIT10162, was isolated in which the ClaI-HaeIII arg$^3$ transcription termination fragment was inserted on pRIT10158 between the ClaI and filled in EcoRI sites and in which the EcoRI site has been recreated. Figure 8 is a flow sheet which illustrates the preparation of pRIT10162. Plasmid DNA of pRIT10162 was digested with EcoRI and PstI endonucleases, mixed with plasmid DNA of pRIT12176, prepared as described above, (also digested with EcoRI and PstI endonucleases) and the mixture was ligated and used to transform cells of E. coliK12 strain MM294 to ampicillin resistance by the methods of Cohen et al., cited above. A plasmid, pRIT12208, was recovered from a colony from this transformation in which the large 4630 bp EcoRI-PstI fragment from pRIT12176 has been ligated to the small 1930 bp EcoRIPstI fragment from pRIT10162, and in which the arg$^3$ transcription termination region is now juxtaposed to the TDH3 promoter. The arg$^3$ and TDH3 DNA regions can be excised from pRIT12208 as a single 2200 bp fragment by digestion with HindIII endonuclease. To obtain the other orientation of this fragment with respect to vector sequences the 2200 bp HindIII fragment from pRIT12208 was recloned into the HindIII site of a pBR322 derivative plasmid in which the natural EcoRI and BamHI sites had separately been deleted by filling in with T4 DNA polymerase and religation. The resulting plasmid, with the 2200 bp HindIII fragment inserted in the opposite orientation with respect to vector sequences compared to pRIT12208, is identified as pRIT12290 and deposited as ATCC 67186 on August 18, 1986 at the ATCC in accordance with Budapest Treaty's regulations. Figure 10 is a restriction endonuclease cleavage map of pRIT12290. In both the pRIT12208 and pRIT12290 vectors, the TDH3 promoter and arg$^3$ transcription termination fragments are separated by unique BamHI, SmaI and EcoRI restriction sites useful for the cloning of DNA fragments carrying coding sequences, and the promoter and transcription termination regions can be excised together on a 2200 bp HindIII fragment as a module or cassette for transfer to other vectors.

The BamHI site is especially useful as it is located at the ATG codon of the TDH3 gene and can be employed for fusion of other genes to the TDH3 promoter with a view to expressing them in yeast as exemplified below. Such fusions can be recovered from pRIT12208 or pRIT12290 derivatives in the form of cassettes or modules for insertion on yeast shuttle vectors by digestion with HindIII endonuclease or by the use of other restriction endonucleases cutting at flanking restriction sites.

Example 4. Construction of plasmids pRIT10677, pRIT10909 and pRIT10158.

a) pRIT10677

A 1372 bp BamH1 fragment of cloned HBV DNA was excised from pRIT10616 (Harford et al., Dev. Biol. Standard, 54, 125-130, 1983) and mixed and ligated with BamH1 endonuclease digested pBR327 DNA. This BamH1 fragment contains part of the HBsAg precursor region, the HBsAg coding sequence and 3' non coding sequences. From the ligation mixture a plasmid, pRIT10677, was selected which was the HBV BamH1 fragment inserted at the BamH1 site of the vector in the orientation such that the Xba1 site in the HBV DNA is proximal to the Sal1 site in the pBR327 vector. Figure 8 is a flow sheet which illustrates the preparation of pRIT10677.

b) pRIT10909

The 3300 bp HindIII fragment from plasmid pMC200 (available from the American Type Culture Collection, Rockville, Maryland, U.S.A., without restriction under accession number ATCC 39131), described by Crabeel M. et al (EMBO J., 2, 205-212, 1983), was recloned onto a pBR322 derivative plasmid on which the natural EcoR1 site had been deleted by filling in with T4 DNA polymerase and religation. A resulting recombinant plasmid, pRIT10158 (Figure 8), was selected in which the HindIII arg³ gene insert has the 3' region of the arg³ gene proximal to the Cla1 site on the modified pBR322 vector. From pRIT10158, a 1150 bp Cla1HaeIII fragment was recovered which carries the transcription termination region of the arg³ gene. This 1150 bp fragment was ligated with Cla1 and Hpa1 endonucleases digested DNA of plasmid pRIT10677, (prepared as described in Part a), above, to fuse the arg³ termination region at the Hpa1 site downstream of the HBsAg coding region. The resulting plasmid is pRIT10909. Figure 8 is a flow sheet which illustrates the preparation of pRIT10909. Figure 3 is a restriction endonuclease cleavage map of pMC200.

Example 5. Construction of plasmids pRIT10911 and pRIT10912.

The BamHI site on pRIT10167 (Example 1) and the natural BamHI site located in the precursor region of the HBsAg gene from a HBV virus of adw serotype cloned on pRIT10616 (Harford et al., Dev. Biol. Standard, 54, 125-130, 1983) are in the same translational reading frame enabling the construction of a fusion of the TDH3 promoter fragment which supplies the ATG codon to 42 codons of the HBsAg precursor sequence followed by 226 codons of the HBsAg coding sequence proper. The source of the HBV DNA fragment for this fusion was a plasmid pRIT10909 (see, Example 4, part b, above) containing a 935 bp BamHIHpal insert encoding part of the HBsAg precursor region, the full HBsAg coding sequence and 128 bp of 3' non translated DNA to which had been fused, downstream of the Hpa I site, an 1150 bp HaeIII-ClaIII DNA fragment from pRIT10158 containing the arg³ transcription termination region. pRIT10158 is described in Example 4, part B, above. The 2085 bp EcoRI-BamHI fragment was excised from pRIT10909 and inserted between the EcoRI and BamHI sites of pRIT10167, prepared as described in Example A, so as to create plasmid pRIT10911. The 3135 bp HindIII fragment from pRIT10911 containing the yeast DNA transcriptional signals and the HBsAg coding sequences was then inserted on the YEp13 shuttle vector to give plas- mid pRIT10912. Figure 8 is a flow sheet illustrating the preparation of pRIT10911.

Example 6. Construction of plasmids pRIT10903 and pRIT10914

The aim of the constructions described below was to remove excess nucleotides from the 5' end of a DNA fragment encoding at least the N-terminal portion of the mature HBsAg coding sequence in such a fashion as to create a 6 base pair restriction site at the first base of the second codon. The resulting fragment can then be fused to promoter fragments with a 6 base pair restriction site at the initiation codon using Mung Bean or S1 nucleases to trim away the single strand extensions and create blunt ends for ligation as shown by Rosenberg et al., Methods in Enzymology, 101C, 123 (1983).

A 1225 bp FnuDII fragment of HBV DNA from pRIT10616 (See, Harford et al. (1983) Devel. Biol. Standard, 54, 125-130) and encoding the HBsAg gene was excised from this plasmid and ligated with synthetic octomer EcoRI linkers, and the fragment cloned into the EcoRI site of vector pACYC184 to give pRIT10679 (Figure 8). Vector pACYC184 was received from S. Cohen and is described by Chang A.C.Y. and Cohen S. (1978), J. Bacteriol., 134, 1141- 1156. pACYC184 is also available from the American Type Culture Collection, Rockville, Maryland, U.S.A., under accession number ATCC 37033. From this EcoRI (FnuDII) fragment, a 125 bp EcoRI-Xbal fragment was obtained which contains the Cterminal region of the HBsAg precursor, the HBsAg ATG codon and 90 bp of HBsAg coding sequence. This 125 bp EcoRI-Xbal fragment was introduced between the EcoRI and Xbal sites of pRIT10158 (see, Example 4 (b), above). The resulting plasmid, pRIT10903, therefore contains a sin- gle EcoRI site located at the junction between arg³ DNA and HBV DNA and has a single XhoI site located in the arg³ promoter region. Figure 8 is a flow sheet which illustrates preparation of pRIT10903.

150 μg of pRIT10903 plasmid DNA, prepared by CsCl-ethidium bromide density gradient centrifugation, was digested with 80 units of EcoRI endonuclease, extracted with phenol, ethanol precipitated and resuspended at 1 μg per μl in 10 mM tromethamine-HCl buffer (pH 7.5). The DNA was divided into three 50 μg samples and incubated for 50, 75 and 90 seconds with Bal31 nuclease at 30°. Each reaction mixture contained the same reaction buffer cited in Example 1, 50 μg of EcoRI digested pRIT10903 DNA, and 2.5 units of Bal31 nuclease in a final volume of 500 μl. The reactions were stopped by addition of EGTA to 20 mM final concentration, chilling on ice, extraction with an equal volume of phenol and precipitation with etha nol. The Bal31 treated DNA samples were each taken up in 50 μl 10 mM tromethamine-HCl buffer and 2.5 μg digested with BstEll endonuclease and compared on a 7.5% acrylamide gel to an EcoRI plus BstEll endonuclease digest of pRIT10903 which releases a 285 bp fragment. Treatment with Bal31 nuclease for 75 seconds at 30° was found to reduce the size of this EcoRIBstEll fragment by 10 to 60 base pairs giving a series of discrete bands estimated to represent removal of 10,30,45 and 60 base pairs of DNA. Resection of 29 base pairs from the original FnuDll site would remove all the HBsAg precursor DNA and the HBsAg ATG codon.

5 μg of pRIT10903 DNA which had been treated with Bal31 for 75 seconds was digested with 8 units of Xhol nuclease, extracted with phenol and ethanol precipitated. This DNA was then incubated with 5 units of T4 DNA polymerase in the presence of deoxynucleotide triphosphates to fill in and render flush ended the Xhol and Bal31 extremities, treated with phenol and ethanol precipitated. The DNA was then incubated with 5 units of T4 DNA ligase for 16 hours at 16° and the mixture used to transform competent cells of E. coli K12 strain MM294 to ampicillin resistance according to the method of Cohen et al., cited above. About 2000 ampicillin resistant colonies per ml were recovered after plating. Plasmids were prepared from 47 individual colonies by a small scale procedure (See, Birnboim et al, cited above), and 23 of 47 were found to retain a Xhol site indicative of successful filling in of the original Xhol site and ligation to a 3' end terminating in a G residue. These 23 plasmids were further digested with Xhol and Xbal endonucleases to determine the size of the small restriction fragment in comparison with the original EcoRI-Xbal fragment from pRIT10903.

Several plasmids were identified with deletions estimated to range from 25 to 40 base pairs. One plasmid, pRIT10914, which was estimated to contain a XholXbal fragment of 95 to 100 base pairs was selected for further study. Figure 8 is a flow sheet which illustrates preparation of pRIT10914. DNA of this plasmid was purified by CsClethidium bromide density gradient centrifugation and 25 μg of such DNA was digested with 140 units of Xbal endonuclease.

The Xbal terminii were exchange labelled with γ-$^{32}$P-ATP, and the labelled DNA was digested with 15 units of HindIII endonuclease. The small 765 bp HindIII-Xbal fragment was isolated by acrylamide gel electrophoresis and electroelution followed by ethanol precipitation. The nucleotide sequence at and around the Xhol site was determined by sequencing this fragment from the labelled Xbal terminus using the chemical modification methods of Maxam and Gilbert, cited above. The sequence data showed that the Xhol site was located at the first base of the second codon of the HBsAg coding sequence.

## XhoI

CTCGAGAAC

HBsAg nucleotides

This fragment is therefore suitable for fusion to the BamHI extension of the TDH3 promoter fragment on pRIT10167 (Example 1) after removal of single stranded ends.

Example 7. Construction of plasmids pRIT12211, pRIT12209, pRIT12230, and pRIT12265

Plasmid pRIT10911, prepared as described in Example 5, was used as a scaffold for further manipulations. To introduce desirable restriction sites, the plasmid was digested with BamHI and Xbal endonucleases, and the excised fragment replaced by a 2275 BamHI-Xbal fragment from plasmid pRIT10158 (Example 4). This manipulation results in plasmid pRIT12211 in which the HindIII-BamHI TDH3 promoter fragment is next to a BamHI-Xbal fragment containing a Xhol site followed by an Xbal-HindIII fragment comprising the C-terminal region of the HBsAg coding sequence and 128 bp of 3' non coding DNA fused to the 1150 bp arg$^3$ transcription termination region. Figure 8 is a flow sheet which illustrates preparation of pRIT12211. Plasmid pRIT12211 was digested with Xhol and Xbal endonucleases thereby removing a 1600 bp fragment which was replaced by the 94 bp XholXbal fragment of modified HBsAg DNA from pRIT10914, prepared as described in Example 5. This 94 bp fragment was purified by acrylamide gel electrophoresis from a Xhol plus Xbal digest of pRIT10914 DNA followed by electroelution of the appropriate gel slice and recovery of the DNA by ethanol precipitation.

The plasmid, pRIT12209, resulting from the introduction of the 94 bp fragment (See, Figure 8), was purified by CsCl-ethidium bromide density centrifugation, and 50 μg of pRIT12209 DNA was digested with 90 units of BamHI endonuclease and 60 units of Xhol endonuclease to remove the 990 bp of extraneous DNA between the TDH3 promoter and the HBsAg coding region, and then was phenol extracted and ethanol precipitated. 16 μg of this DNA was incubated for 30 minutes at 30° with 20 units of Mung Bean nuclease (PL Biochemicals) in a volume of 125 μl. The buffer used for the incubation was 30 mM Sodium acetate (pH 4.6), 250 mM NaCl, 1 mM ZnCl$_2$ and 5% glycerol. The reaction was stopped by the addition of sodium dodecyl sulphate to give 0.2% final concentration and extraction with an equal volume of phenol followed by ethanol precipitation. An aliquot of 0.5 μg of this Mung Bean nuclease treated sample was incubated with 2 units of T4 DNA ligase for 16 hours at 16° and then digested with 2 units of BamHI endonuclease to destroy any vector molecules which had escaped the above treatments. This mixture was used to transform competent cells of E. coli K12 strain MM294 with selection for ampicillin resistance according to the method of Cohen et al., cited above. About 2000 ampicillin resistant colonies per ml of transformation mixture were recovered and plasmids were prepared by a small scale procedure (according to the method of Birnboim et al., cited above) from amplified cultures of 24 of these transformants.

16 of 24 plasmids gave two bands of DNA with sizes to 2700 bp (pUC9 vector) and 3000 bp after digestion with HindIII endonuclease. The 3000 bp band is the size expected for the fusion of the HBsAg coding sequence and $arg^3$ termination region to the TDH3 promoter fragment.

Four candidate plasmids were taken for further study. DNA of each plasmid was digested with XbaI endonuclease and labelled by exchange kination with γ-$^{32}$P-ATP followed by digestion with HindIII endonuclease and isolation of the labelled 1190 bp HindIII-XbaI fragment by electroelution and ethanol precipitation following acrylamide gel electrophoresis. The sequence of each fragment was determined by the chemical modification methods of Maxam and Gilbert, cited above. Two plasmids were found to have the correct sequence ATGGAGAAC for perfect fusion of the TDH3 promoter region to the HBsAg gene. One of these plasmids, pRIT12230, was used in further manipulations. Figure 8 is a flow sheet which illustrates preparation of pRIT12230. DNA of this plasmid (pRIT12230) was digested with HindIII endonuclease and the 3000 bp fragment containing the HBsAg coding sequence fused to the TDH3 promoter was ligated onto shuttle vector YEp13. The resulting plasmid, pRIT12265, was transformed into yeast strain DC5 (MATa, leu2-3, leu2-112, his3, can1-11) (obtained from J. Broach (State University of N.Y. Stony Brook) and available without restriction from the American Type Culture Collection under accession number ATCC 20630) using the transformation procedure of Ito et al., J. Bact, 153, 163 (1983).

Example 8. Construction of plasmids pRIT12288, pRIT12322

The construction on plasmid pRIT12230 (Example 7) contains 128 bp non coding HBV DNA located 3' downstream of the stop codon of the HBsAg structural gene.

To remove the 128 bp of DNA, about 25 μg of pRIT12230, prepared as described in Example 7 above, is digested with AccI and EcoRI endonucleases. pRIT12230 contains a single AccI site located in the S-gene coding sequence 7 nucleotides before the TAA stop codon. The digested DNA was electrophoresed on a 1% agarose gel and the larger 4200 bp vector fragment was recovered from the gel by electroelution and ethanol precipitation.

Artificial DNA linker molecules composed of a 12-mer strand and a 14-mer strand with the following sequences are synthesized for insertion between the AccI and EcoRI sites of pRIT12330:

```
5' ATACATTTAACG    3'

12 mer

3'    TGTAAATTGCTTAA    5'

14 mer
```

This will restore the correct C-terminal HBsAg codons, the TAA stop codon and provide an EcoRI site not present elsewhere in the TDH3 promoter of HBsAg coding sequences for addition of transcription termination fragments.

100 pmoles of the synthetic 12 mer and 100 pmoles of the synthetic 14 mer single strands are mixed together in a final volume of 10-20 μl, heated at 70° for 15 minutes, and allowed to return slowly to room temperature over a period of 3 hr permitting the annealing of the two strands along their complementary sequences. To this annealed mixture is added about 0.15 pmole (400 ng) of the 4200 bp AccI-EcoRI fragment of pRIT12230, 10x concentrated ligation buffer and 2 units of T4 DNA ligase.

This mixture is incubated for 4 hours at 16° before the addition of a further 2 units of T4 DNA ligase and then incubated overnight on ice. The ligated mixture is used to transform competent cells of strain MM294 to ampicillin resistance according to the method of Cohen et al. (Proc. Natl. Acad. Sci., U.S.A., 69, 2110, 1972). Plasmids are prepared from 12 or more individual colonies by the rapid procedure of Birnboim and Doly (Nucl. Acids Res., 7, 1513, 1979) and examined by restriction endonuclease analysis. Plasmids with insertion of the AccI to EcoRI linker will show a single band of DNA of 4200 bp on digestion with either AccI or EcoRI enzyme. The correctness of the linker insertion on such a plasmid can be verified by DNA sequencing from either the EcoRI site or AccI site by the chemical modification method of Maxam and Gilbert (Methods in Enzymology, 65, 499, 1980).

To provide a transcription termination fragment, a plasmid with the AccI-EcoRI linker insert, obtained as described above, is digested with EcoRI endonuclease and treated with alkaline phosphatase (Shine J., U.S. Patent 4,264,731). The 2650 bp HindIII fragment of pRIT10162, obtained as described in Example 3, was recloned in the HindIII site of the pBR327 vector plasmid to form plasmid pRIT12288. pBR327 is described in Soberon et al. (Gene, 9, 287-305, 1980), and can be prepared as described in Example 10, part b, infra, from plasmid pRIT12309 which can be recovered from the American Type Culture Collection under accession number ATCC 67187. Figure 8 is a flow sheet which discloses construction of pRIT12288.

The orientation of the HindIII fragment on pRIT12288 is such as to juxtapose the arg³ transcription termination region with the EcoRI and ClaI restriction sites on the pBR327 moiety. From pRIT12288, a 1180 bp EcoRI fragment is obtained which carries the arg³ transcription termination region. This fragment is ligated to the EcoRI digested, alkaline phosphatase treated pRIT12230 derivative described above, and plasmids issuing from the ligation mixture are examined for insertion and the orientation of the 1180 bp EcoRI fragment. The orientation of the insert can be obtained by digestion with HindIII endonuclease which will liberate fragments of 2880 and 2720 bp if the insert orientation is as desired.

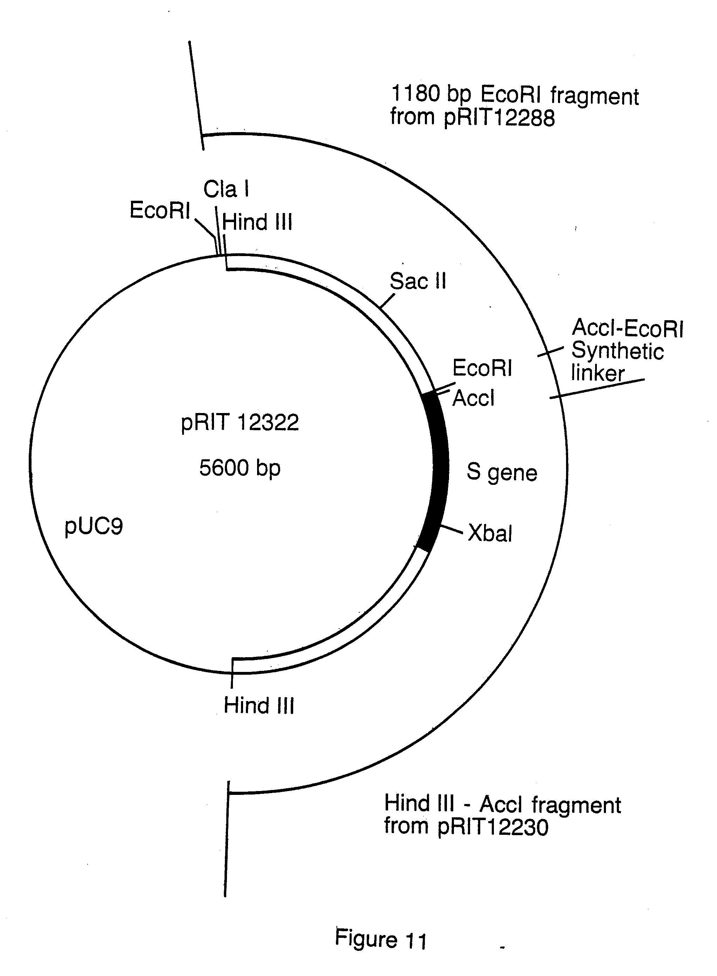

A plasmid, pRIT12322, was constructed in which the AccI-EcoRI linker replaces the 128 bp of unwanted HBV DNA and on which the 1180 bp EcoRI fragment from pRIT12288 was present in the desired orientation, downstream of the HBsAg coding sequence. A 2900 bp HindIII fragment can be excised from pRIT12322 which carries the expression module for HBsAg synthesis including the TDH3 promoter, the HBsAg coding sequence and the arg³ transcription termination region. Figure 8 is a flow sheet which discloses construction of pRIT12322. See, also, Figure 11, which is a restriction endonuclease map of pRIT12232 and shows which portions thereof come from pRIT12288 and pRIT12230.

Example 9. Construction of plasmid pRIT12329

As described in Example 8 above, pRIT12322 contains a 2900 bp HindIII fragment with all the necessary signals for expression of HBsAg from the TDH3 promoter. This module was ligated onto a shuttle vector for introduction into yeast cells.

DNA of shuttle vector YEp13 was digested with HindIII endonuclease and alkaline phosphatase and used as recipient for the introduction of the HindIII fragment from pRIT12322, prepared as described in Example 8. Plasmid pRIT12329 was isolated which consists of the YEp13 replicon together with the 2900 bp HindIII module fragment described in Example 8.

Example 10. Construction of plasmid vectors for the expression of the hybrid antigen HBsAg-CS in yeast

A. Construction of Plasmids pRIT12573 and pRIT12574

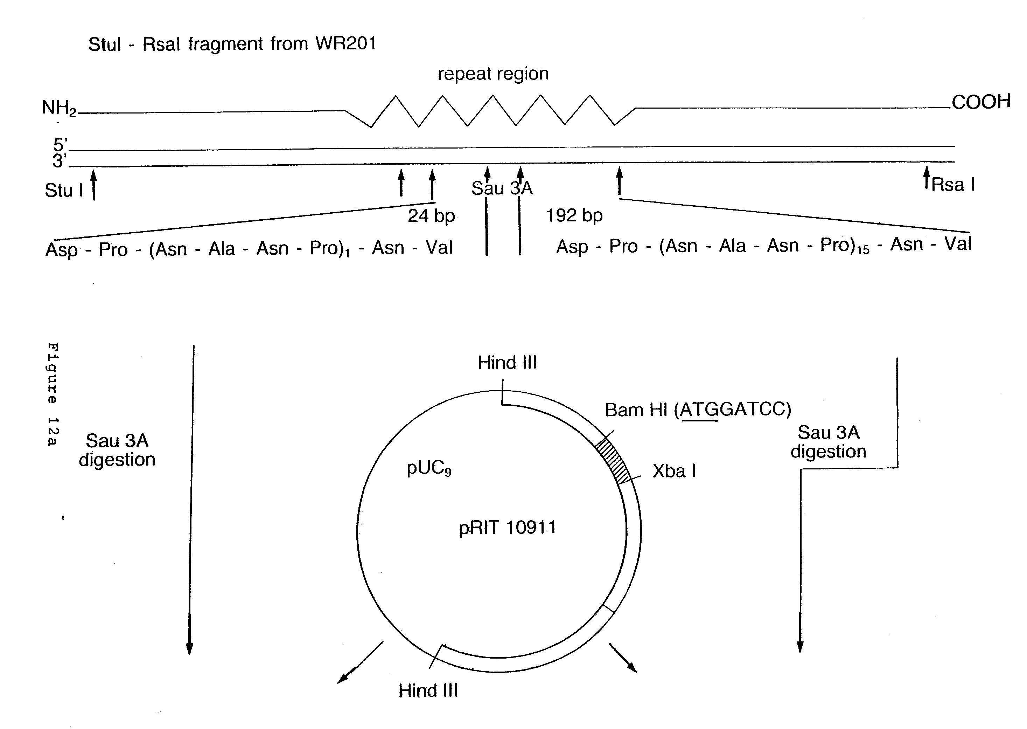

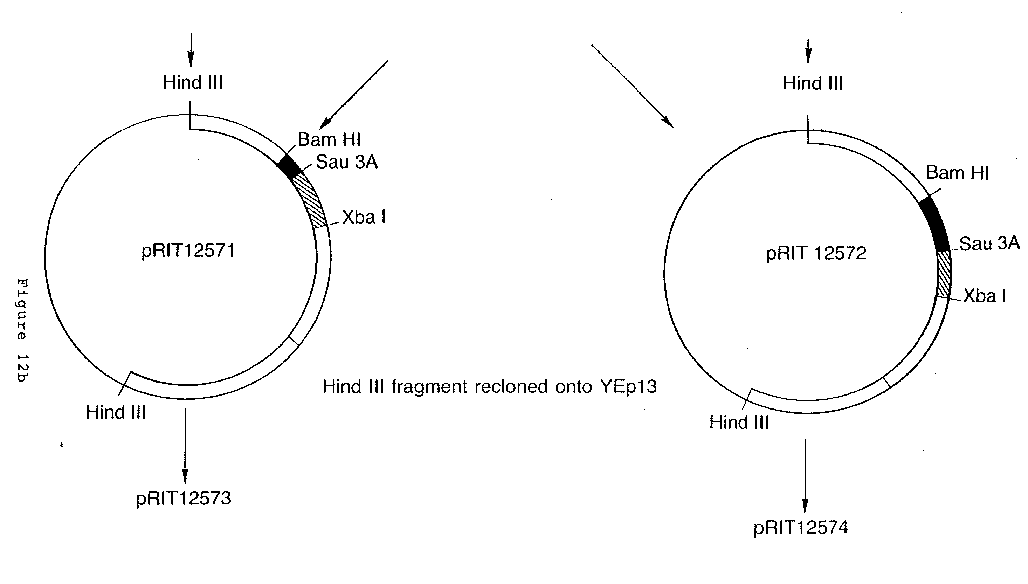

Plasmid WR201 was obtained from the Walter Reed Army Institute of Research and results from insertion of a 2.3 kilobase (kb) EcoRI fragment from λmPfl (Dame et al., Science, 225, 593-599, 1984) encoding the complete circumsporozoite protein gene of Plasmodium falciparum into vector pUC8. Figure 2A shows a schematic restriction map and sequencing strategy of clone λmPfl. The positions of restriction enzyme cleavage sites shown in the figure were determined from the sequence and confirmed by digestion: A, AvaII; Ac, AccI; B, BstnI; D, DraI; Dd, DdeI; F, FokI; N, NdeI; R, RsaI; S, StuI; T, TthIII; Tq, TaqI; X, XhoII. Arrows indicate the origin, direction and extent of the sequences determined. The CS protein coding region is shown as a heavy line. Figure 3A shows the nucleotide sequence of the CS protein gene from P. falciparum. The nucleotide sequence of the CS protein gene in λmPfl is shown. Vector pUC8 is described by Vieira et al. (Gene, 19, 259, 1982). pUC8 is commercially available from Amersham and Pharmacia. A 1215 base pair (bp) StuI-RsaI fragment of plasmid WR201 containing the CS protein coding sequence minus the first 52 bp (Figure 12) was purified by electroelution from a 7.5% polyacrylamide electrophoresis gel. This fragment was digested with Sau3A to generate smaller fragments which included, among others, a 192 bp Sau3A fragment coding for 16 tetrapeptide repeats of the CS protein and three identical 24 bp Sau3A fragments coding for 2 tetrapeptide repeat of the CS protein.

Plasmid pRIT10911 (prepared as described in Example 5) is a pUC9 derivative carrying a 3136 bp HindIII cassette containing a 1050 bp HindIII-BamHI yeast glyceraldehyde-3P-dehydrogenase (TDH3) promoter fragment (which supplies the ATG) fused at the BamHI site to a 935 bp BamHI-HpaI fragment of HBV DNA encoding the C-terminal 42 amino acids (aa) of the pre-S2 region, 226 aa of the S gene (serotype adw) and 128 bp of 3' non-coding DNA. The S gene is flanked by a 1150 bp yeast DNA fragment carrying the arg³ transcription terminator. pUC9 is described by Vieira et al. (Gene, 19, 259-268, 1982). The BamHI site of pRIT10911, located at the ATG initiation codon, is in phase with the Sau3A sites of the repetitive epitope of CS of Plasmodium falciparum. DNA of plasmid pRIT10911 was digested with BamHI endonuclease, treated with alkaline phosphatase, extracted with phenol and recovered by ethanol precipitation. 0.2 µg of this DNA was mixed with 0.2 µg of the Sau3A digest of the StuI-RsaI CS gene fragment prepared as described above, treated with T4 DNA ligase, and the ligation mixture was used to transform competent cells of E. coli K12 strain MM294 prepared according to the method of Cohen et al. (Proc. Natl. Acad. Sci., U.S.A., 69, 2110, 1972). 32 plasmids from individual transformant colonies were prepared by a small scale procedure (Birnboim et al., Nucleic Acids Research, 7, 1513, 1979) after amplification of the plasmid by addition of spectinomycine (300 µg per ml) to the growing cultures. The recombinant plasmids were analysed on 7.5% acrylamide electrophoresis gel, after double digestion with BamHI and XbaI endonucleases, and were compared to the 219 bp BamHI-XbaI fragment of pRIT10911 (comprising the first 42 amino acid coding sequence of the Pre S2 region precursor and the beginning of HBsAg region) and to the fragments of a HaeIII digest of φx174 DNA.

Among the 32 plasmids, one recombinant showed a BamHIXba fragment of 411 bp which corresponds to the insertion of the 192 bp fragment Sau3A in the correct orientation and was designated pRIT12572. Another recombinant showed a BamHI-XbaI fragment of 243 bp corresponding to the insertion of the 24 bp Sau3A fragment in the correct orientation and was designated pRIT12571. Figure 12 is a flow sheet illustrating preparation of pRIT12571 and pRIT12572.

The HindIII fragments of pRIT12572 and pRIT12571, containing the TDH3 promoter, the CS-HBsAG hybrid coding sequence and the arg3 termination region were recloned onto YEp13, to give, respectively, plasmids pRIT12574 and pRIT12573 (Figure 12). YEp13 is described by Broach et al. (Gene, 8, 121-133, 1979). Figure 12 is a flow sheet illustrating preparation of pRIT12573 and pRIT12574.

Plasmids pRIT10912, pRIT12574 and pRIT12573 were introduced into S. cerevisiae yeast strain DC-5 (a, leu2-3, leu2-112, his3, can1-11), and into S. cerevisiae yeast strain 10S44C (pep4-3 leu2-3, leu2-112) obtained from M. Crabeel located at the Ceria Institute, Anderlecht, Belgium, (see, also Cabezon, T. et al., Proc. Natl. Acad. Sci. U.S.A., 81, 6594-6598, 1984) by transformation of lithium acetate treated cells (Ito et al., J. Bacteriol., 153, 163-168, 1983) and selection for leucine independence. Yeast strain DC5 was deposited in the American Type Culture Collection, Rockville, Maryland, U.S.A., in accordance with the regulations of the Budapest Treaty on June 2, 1982, under accession number ATCC 20630. Yeast strain 10S44C was deposited in the American Type Culture Collection, Rockville, Maryland, U.S.A., in accordance with the regulations of the Budapest Treaty on August 18, 1986, under accession number ATCC 20819. The expected hybrid protein for pRIT12573 is predicted to contain 277 amino acids, and for pRIT12574 is predicted to contain 333 amino acids.

It is also possible to add to or enlarge the CS epitope region on the vector of the invention. For example, on plasmids pRIT12572 and pRIT12571, additional Sau3A fragments of CS protein DNA, e.g., 192 bp or 24 bp Sau3A fragments, can be ligated in to the BamHI site as follows:

Plasmid DNA of pRIT12572 or pRIT12571 is digested with BamHI endonuclease and treated with alkaline phosphatase. The 1215 bp StuI-RsaI fragment of plasmid WR201, obtained as described above, is digested with Sau3A endonuclease to liberate the 192 bp and 24 bp fragments bearing the tetrapeptide repeats. These fragments are mixed with the BamHI and alkaline phosphatase treated pRIt12572 or pRIT12571 vectors, treated with T4 DNA ligase and the mixtures transformed into E. coli K12 cells according to conventional procedures with selection for ampicillin resistance. Plasmids from transformant colonies are screened for the size of the BamHI-XbaI fragments by endonuclease digestion and compared to similarly digested DNA of pRIt12572 or pRIT12571, especially the 411 bp and 243 bp BamHI-XbaI fragments from pRIT12572 and pRIt12571 which carry the fusion between the CS repeats and part of the pre S2-S coding sequence. Increase in the size of these fragments by 192 bp or 24 bp is indicative of the insertion of a tetrapeptide repeat fragment at the BamHI site of pRIT12572 and pRIT12571. The presence of a BamHI site on such plasmids is indicative of insertion of the 192 bp or 24 bp Sau3A fragments in the correct orientation for fusion at the ATG codon. This process described above may be repeated if desirable to introduce further 192 bp or 24 bp Sau3A fragments encoding the CS tetrapeptide repeats and elongated the CS tetrapeptide region on the hybrid particle. As stated before, the HindIII fragments from such derivatives of pRIT12572 or pRIT12571 carrying the expression module or cassettes may be excised and inserted on YEp13 or other suitable yeast cloning vector for introduction into yeast cells by conventional techniques.

(B) Construction of pRIT12309, pRIT12314 and an expression shuttle vector pRIT12377 containing the complete 2 micron DNA sequence from yeast

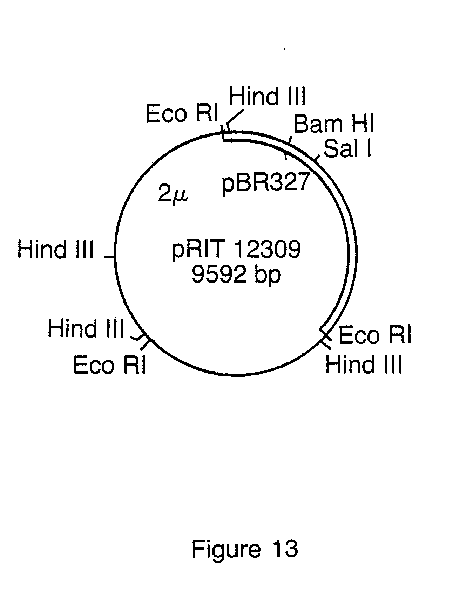

The plasmid pRIT12309 consists of the complete 6318 bp 2 micron DNA sequence from yeast cloned into the EcoRI site of the plasmid vector pBR327. The 6318 bp 2 micron DNA sequence was obtained from plasmid pCV19 from J. Broach, Princeton University, NJ. Plasmid pRIT12309 was deposited on August 18, 1986 in the American Type Culture Collection, Rockville, Maryland, U.S.A., in E. coli K12 strain MM924 in accordance with the regulations of the Budapest Treaty under accession number ATCC 67187. Figure 13 is a restriction endonuclease map of pRIT12309. Insertion on pBR327 of the 2 micron DNA sequence through one of the two EcoRI sites present on this molecule interrupts the 2 micron A coding region and renders the resulting hybrid molecule pRIT12309 ineffective for A gene function, also referred to as FLP function. For a description of 2 micron structure and function see Broach J., "The Molecular Biology of the yeast Saccharomyces: Life cycle and Inheritance", pp. 445-470, Strathern J.N., Jones E.W. and Broach J.R. (Eds), Cold Spring Harbor Laboratory, New York (1981).

pBR327 is described by Soberon et al. (Gene, 9, 287-305, 1980) and was obtained from F. Bolivar (Department of Molecular Biology, National University of Mexico, Mexico 20DF). It will be appreciated by one skilled in the art that pBR327 can be recovered from plasmid pRIT12309 above by preparing pRIT12309 plasmid DNA recovered from the E. coli strain deposited in the American Type Culture Collection under the accession ATCC 67187 by any of a number of conventional methods, digesting this DNA with EcoRI endonuclease, ligating the digest and transforming competent cells of E. coli K12 to ampicillin or tetracycline resistance with the ligation mixture. A substantial number of the transformant colonies will contain plasmids in which only the pBR327 moiety of pRIT12309 is present devoid of either of the two 2 micron DNA fragments.

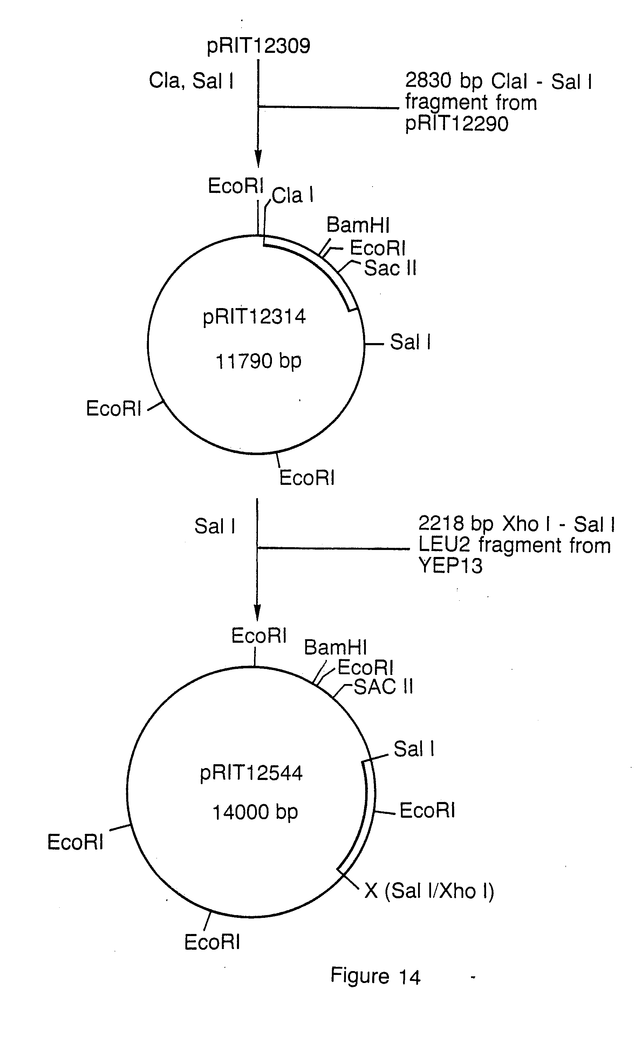

The 2850 bp ClaI-SalI fragment containing the TDH3-arg3 expression cassette and flanking pBR322 sequences from pRIT12290 (described in Example 3, above) was recloned onto the pRIT12309 vector between the ClaI and SalI sites. The resulting plasmid, pRIT12314, was digested with SalI endonuclease and ligated with the 2218 bpSalI-XhoI fragment from YEp13 containing the yeast LEU2 gene. Figure 14 is a flow sheet illustrating preparation of pRIT12314. YEp13 is available from the American Type Culture Collection under accession number ATCC 37115. The ligation mixture was used to transform competent cells of strain JA221 prepared according to the method of Cohen et al (above) to both leucine independence and ampicillin resistance.

Strain JA221 is publicly available, and was obtained from M. Rosenberg, Smith Kline & French Laboratories. Inc., Philadelphia and may be obtained from the American Type Culture Collection under accession number ATCC 33875.

A plasmid, pRIT12544, was obtained from a colony from this transformation in which the 2218 bp SalI-XhoI LEU2 gene fragment was inserted at the SalI site of pRIT12314. The orientation of the insertion is such so as to recreate the SalI site on the side closest to the TDH3-arg³ expression cassette. Plasmid pRIT12544 is an E. coli - yeast shuttle vector with single BamHI and SmaI sites located between the TDH3 promoter and arg³ transcription termination regions which are suitable for insertion of DNA coding sequences to effect their expression from the TDH3 promoter. Figure 14 is a flow sheet illustrating preparation of pRIT12544.

## (C) Construction of Plasmid pRIT12658

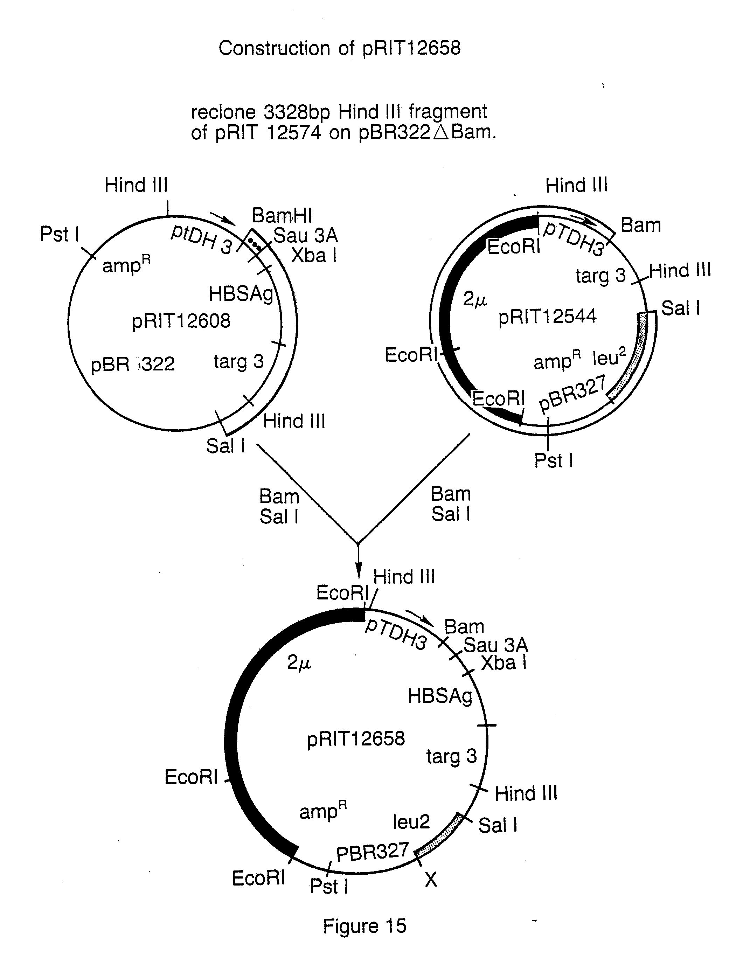

The 3328 bp HindIII fragment of pRIT12574, a YEp13 derivative prepared as described in Example 10, Part A, was recloned onto a pBR322 Δ BamHI plasmid (where the BamHI site has been destroyed by filling in with T4 DNA polymerase) to give pRIT12608 where the BamHI site located at the ATG initiation codon of the CS - HBsAg coding sequence is unique (Figure 15). The 2550 bp BamHI-SalI fragment from pRIT12608 containing the CSHBsAg coding sequence, the arg³ transcription termination region and flanking pBR322 sequence was recloned between the BamHI and SalI sites of pRIT12544 (Figure 15), described above, to give plasmid pRIT12658 (Figure 15). This exchange placed the CS-HBsAg coding sequences under the control of the TDH3 promoter on a complete 2 micron yeast shuttle vector. Figure 15 is a flow sheet illustrating preparation of pRIT12658.

## Example 11. Synthesis of hybrid protein in yeast

Yeast (Saccharomyces cerevisiae) strains DC5 or 10S44C were transformed according to the method of Ito et al. (J. Bacteriol., 153, 163-168, 1983) with each of the plasmids pRIT10912 (Example 5), pRIT12573 (Example 10); pRIT12574 (Example 10) or pRIT12658 (Example 10), or plasmids pRIT12329, (Example 9) and pRIT10172 (Example 2), and were separately grown in liquid medium lacking leucine (YNB or YNB + 80 µg/ml histidine) and harvested in mid-log phase. Cells from 1 or 2 ml culture were collected by centrifugation and were resuspended in 50 µl of 0.125 M tris-HCl (pH6.8) containing 20% glycerol, 4% SDS, 6 M urea and 10% Z-mercaptoethanol. After incubation for 5 minutes (min.) at 100°, the sample was divided in halves and both were electrophoresed through a 12.5% separating gel, 5% stacking gel according to the method of Laemmli (Nature, 227, 680, 1971).

HBsAg and CS protein sequences were identified by protein immunoblotting technique (See, Burnette, Anal. Biochem., 112, 195-203, 1981; Gershoni and Palade, Anal. Biochem., 131, 1-15, 1983; Towbin and Gordon, J. Immunol. Methods, 72, 313-340, 1984). After electro- blotting of the proteins onto a nitrocellulose filter (Schleicher and Schüll, 0.45 µ) (Towbin et al., Proc. Natl. Acad. Sci., U.S.A., 76, 4350-4354, 1979) the filter was preincubated for one hour at 37°C with 3% gelatin in PBS. Subsequently the filter was washed 5 times for 5 min. each time with PBS containing 0.1% Tween 20 (polyoxyethylene sorbitan monolaurate), and one-half of the sheet was treated for one hour at room temperature with a mixture of five monoclonal antibodies against CS protein (Dame et al., Science, 225, 593-599, 1984) in PBS, 1% gelatin. The other half of the sheet was treated with a monoclonal antibody against HBsAg, HepYTAS12, in PBS, 1% gelatin. HepYTAS12 is an antibody raised against purified yeast derived HBsAg which is directed against a reduction and denaturing resistent epitope. The filter sheets were washed 5 times for 5 min. each time with PBS, 0.1% Tween 20, and a second biotinylated sheep anti-mouse antibody (Amersham) in PBS, 1% gelatin was added for one hour at room temperature. The sheets were washed again with PBS, 0.1% Tween 20, 5 times for 5 min. each time, and were incubated for 30 min. at room temperature with streptavidin-biotinylated horseradish peroxidase (HRP) complex (Amersham). The sheets were washed again 3 times for 5 min. each time with PBS, 0.1% Tween 20, and were finally incubated with 30 µl $H_2O_2$ and HRP Color Development Reagent containing 4-chlorol-naphtol (Bio-Rad), 30 mg in 10 ml methanol, in 50 ml PBS. The molecular weight of the antigens was estimated from prestained protein markers, ovalbumin, alphachymotrypsinogen, beta-lactoglobin, lysozyme, cytochrome C (BRL).

Both yeast strains DC5 and 10S44C, transformed with pRIT12573, showed production of an antigen reacting with both anti-CS and anti-HBsAg antibodies, and of an estimated molecular weight of 30,000 kilodaltons (kd). Both yeast strains DC5 and 10S44C, transformed with either pRIT12574 or pRIT12658 showed production of an antigen capable of reacting with both anti-CS and anti-HBsAg antibodies, and of an estimated molecular weight of 37,000 kd. Some lower molecular weight antigen was also detected, indicative of some degradation of the hybrid protein. Control samples of cells containing plasmids without any CS or HBsAg sequences (pRIT10172) did not show any reaction. Control samples of Cells containiong plasmids with only HBsAg sequences (pRIT12329 or pRIT10912) showed reaction only with the anti-HBsAg antibodies.

These results show that a hybrid protein of the expected molecular weight containing both CS and HBsAg sequences is synthesized in yeast transformed with a plasmid containing the CS coding sequence fused in phase to the PreS2-HBsAg coding sequence.

Furthermore, while the band intensity in the anti-HBsAg reaction was about the same for pRIT12573 and pRIT12574, the intensity of the anti-CS reaction was much higher for pRIT12574 than for pRIT12573. This indicates that for the same quantity of HBsAg sequences there are more sequences of the repetitive epitope of the CS protein present in pRIT12574 than in pRIT12573.

Example 12. Synthesis of hybrid particles presenting the CSP epitope

Yeast (Saccharomyces cerevisiae) strains DC5 or 10S44C containing either pRIT10172 (Example 2), pRIT12329 (Example 9), pRIT12573 (Example 10) or pRIT12574 (Example 10), were grown in selective medium (200 ml YNB or YNB + 80 µg/ml histidine) to end of log phase. Cells were collected by centrifugation and resuspended in 10 ml of 50 mM sodium phosphate buffer (pH 7.4) containing 0.5% Tween 20 (polyoxyethylene sor bitan monolaurate), 1 mM PMSF (phenylmethylsulfonyl fluoride) and 2.5% isopropanol. Cells were disrupted by passage twice through a French Press at 20,000 psi (1.38 x 10⁸ Pa). The suspension was centrifuged for 30 min. at 30 000 xg. The total protein concentration of the supernatant liquid (crude cell extract), was measured by the method of Lowry et al. (J. Biol. Chem., 193, 265-275, 1951) with bovine serum albumin as the standard. The presence of HBsAg was assayed using a radioimmune assay kit (AUSRIA II) commercially available from Abbott Laboratories, North Chicago, Illinois or using an ELISA kit (Enzygnost-HBsAg micro) commercially available from Behringwerke, Marburg, W. Germany. The availability of CS sequences in the HBsAg particles to an immunoreaction was tested in the above-mentioned ELISA assay. Appropriate dilutions of samples to be tested (in PBS containing 0.2% BSA) were allowed to bind (overnight at room temperature) to the solid phase anti-HBsAg (Enzygnost kit). The bound HBsAg particles were incubated for one hour at 37°C with an 1/10,000 dilution (in PBS containing 0.1% BSA) of a mixture of five monoclonal antibodies directed against the CS protein (Dame et al., Science, 225, 593-599, 1984) as the first antibody, and then incubated with an 1/500 dilution (in PBS containing 0.1% BSA) of biotinylated sheep anti-mouse Ig (Amersham) as the second antibody for one hour at 37°C. Detection was by incubation with an 1/1000 dilution (in PBS containing 0.1% PBA) of streptavidinbiotinylated horseradish peroxidase complex (Amersham) and chromogen from the Enzygnost kit for 30 min. at 37°C. Between incubations the trays were washed four times with washing solution from the Enzygnost kit. Color development was measured at 510 nm in a Titertek Multiskan (Dynatech).

Measurement of protein concentration and AUSRIA activity (Table II) in crude cell extracts showed that pRIT12573 directed HBsAg expression at the same level as pRIT12329. However, pRIT12574 showed about a ten-fold lower AUSRIA activity. Immunoblot analysis of these extracts showed about an equal amount of HBsAg related protein synthesized in those three cases.

## Table II - AUSRIA activity in crude cell extracts

| Plasmid | Gene | Protein (mg/ml) | HBsAg(RIA) (ng/ml) | HBsAg ng/mg protein |
|---------|------|-----------------|---------------------|---------------------|
| pRIT10172 | – | 5.6 | 0 | 0 |
| pRIT12329 | S | 3.3 | 5700 | 1730 |
| pRIT12573 | CS( 24 bp) PreS2-S | 3.6 | 4000 | 1110 |
| pRIT12574 | CS(192 bp) PreS2-S | 2.8 | 450 | 160 |

The ELISA assay showed that a structure expressing both HBsAg and CS epitopes is present in pRIT12573 and pRIT12574 extracts (Table III) and that there are more CS epitopes per HBsAg epitopes that react in pRIT12574 extracts than in pRIT12573 extracts.

To examine the nature of these RIA and ELISA positive structures, one ml of the crude cell extracts was mixed with 1.5 M CsCl in 50 mM sodium phosphate (pH 7.4) and centrifuged for 40 hours at 40,000xg in a Beckman SW50.1 rotor. Collected fractions were assayed for the presence of HBsAg and CS antibody binding activity with the RIA and ELISA methods described above. HBsAg and CS antigenic activity was found to coequili brate in both pRIT12573 and pRIT12574 gradients. Their buoyant density was found to be slightly higher (rho = 1.20 g/cm³) than the one for pRIT12329 (rho = 1.18 g/cm³).

Immunoblot analysis of the gradient fractions confirmed the RIA/ELISA results. HBsAg and/or CS sequences were found only in those fractions that did react in the RIA/ELISA assay.

## Table -II. HBsAg and CS epitopes present in antigen
## bound to immobilized anti-HBsAg antibody

| Crude cell extract from: | Dilution | ELISA (HBsAg) $OD_{510}$ nm | ELISA (CS) $OD_{510}$ nm |
|---|---|---|---|
| 10S44C (pRIT10172) | 100x | 0.000 | 0.065 |
| 10S44C (pRIT12329) | 100x | 0.580 | 0.054 |
|  | 200x | 0.341 | 0.041 |
|  | 400x | 0.188 | 0.056 |
| 10S44C (pRIT12573) | 100x | 1.298 | 1.596 |
|  | 200x | 1.080 | 1.299 |
|  | 400x | 0.702 | 0.898 |
|  | 800x | 0.336 | 0.560 |
|  | 1600x | 0.155 | 0.323 |
|  | 3200x | 0.075 | 0.189 |
| 10S44C (pRIT12574) | 100x | 0.662 | 1.678 |
|  | 200x | 0.370 | 1.506 |
|  | 400x | 0.183 | 1.130 |
|  | 800x | 0.079 | 0.798 |
|  | 1600x | 0.044 | 0.449 |
|  | 3200x | 0.061 | 0.205 |

These results indicate that the hybrid protein produced by expression of pRIT12573 and pRIT12574 does assemble into particles like the 22 nm HBsAg particles upon synthesis in yeast. The particles produced by expression of pRIT12573 and pRIT12574 do expose the CS sequences on their exterior as shown by their capacity to react with anti-CS antibodies. In the case of pRIT12574, those CS sequences partially obstruct the accessibility of the a determinant of HBsAg (the one mainly responsible for AUSRIA activity) in the RIA test.

Example 13.

A. Purification of hybrid particles

The crude cell extract of yeast strain 10S44C containing pRIT12574, prepared as described in Example 10, was clarified by polyethylene glycol (PEG) 400 precipitation and concentrated by ultrafiltration in an AMICON DC equipped with a cartridge having a cut-off of 100.000 daltons.

Further purification of the hybrid particles was done by methods known in the art, for example, CsCl centrifugation, hydroxyapatite chromatography and gel filtration.

The final purified preparation from cell extract of 10S44C containing pRIT12574 showed one main peak in the exclusion volume of a HPLC TSK-3000 column (LKB) which contained both HBsAg and CS antigenicity. SDS-polyacrylamide gel electrophoresis (Laemmli, Nature, 227, 680, 1971) followed by silver staining (Wray et al., Anal. Biochem., 118, 197-203, 1981) showed one major band of estimated molecular weight (MW) 37,000 kd. Electron microscopy after negative staining with uranyl acetate showed particles of about the same size and shape as yeast derived HBsAg.

B. Purification of hybrid particles

Crude yeast extracts are prepared by Dyno Mill disruption of the washed yeast cells in the presence of an equal weight of 50 mM sodium phosphate extraction buffer (pH 8.1) supplemented with 4 mM Titriplex III, 1 % Tween 20, 4 mM PMSF and 10 % isopropanol.

Cell debris are removed by centrifugation at 11,000xg for 90 min. Five hundred ml centrifuged crude extract are adjusted to pH 7.2 with 1N acetic acid and stirred overnight at 4°C in the presence of 10 gr colloidal Silica (Aerosil 380, Degussa). Subsequently, the suspension is centrifuged at 3,500 g for 1 hour and the pellet is washed three times with 150 ml of 0.15 M NaCl supplemented with 2 mM PMSF and 5 mM EDTA during 15 min. Finally the Aerosil pellet is eluted with 250 ml 10 mM pyrophosphate buffer (pH 9.5) containing 2 % Tween 20 at 37°C for 3 hours. To the desorbate is added dropwise a 1 M $CaCl_2$ solution to a final concentration of 30 mM $CaCl_2$ and the pH is adjusted to 7. The solution is left overnight at 4°C and centrifuged at 6,500g during 15 minutes.The supernatant is dialysed against 2 x 51 10 mM Tris/HCl (pH 7.1) at 4°C and subsequently diluted four times with dialysis buffer.

After addition of 30 mM $CaCl_2$ and 1 M NaCl the diluted antigen solution, adjusted to pH 7.1, is passed over a 150 ml column of Phenyl Sepharose FF, equilibrated in the same $CaCl_2$/NaCl containing Tris buffer, at a flow rate of 30 cm/hr. Subsequently the column is washed with equilibration buffer until $OD_{280\ nm}$ decreases to zero, and eluted at the same flow rate with 6 M urea in 10 mM Tris/HCl pH9.

Alternatively, the dialysed and four times diluted antigen solution is supplemented with 20 % $(NH_4)_2SO_4$ (pH 7) and passed over a 150 ml column of Phenyl Sepharose FF, equilibrated in 10 mM Tris/HCl (pH 7.1) 20 % $(NH_4)_2SO_4$, at a flow rate of 30 cm/hr. After washing the column with equilibration buffer, the column is eluted at the same flow rate with 10 mM Tris/HCl pH 7.1.

The antigen positive fractions are pooled and finally centrifuged in one or two successive CsCl den sity gradients at 4°C and 245,000 g for 65 hrs. The purified hybrid Cs-HBsAg particles have a buoyant density of 1.23 $gr/cm^3$.

Example 14. Immunogenicity of hybrid particles

Hybrid particles, purified from S. cerevisiae strain 10S44C containing pRIT12574, prepared by the method of Example 13, were inoculated into laboratory animals, as such or with aluminum hydroxide as adjuvant (Young et al., Science 228, 958-962, 1985).

C57B1/6 mice (6-8 weeks), guinea pigs, and rabbits were immunized subcutaneously and intraperitoneally on days 0, 21 and 49. Animals were bled on days 7, 28, 56 and 84. Blood was allowed to clot at 4°C overnight and sera were separated and stored at -70°C until used.

Rabbits, 2 groups of 2 animals each, received 10 μg of the hybrid CS-HBsAg protein in 1.0 ml doses, with or without aluminum hydroxide, at each immunization. As a control, 2 rabbits were immunized with 10 μg Heptavax B® (Merck Sharpe & Dohme) following the same inoculation scheme. Mice and guinea pigs, in groups of five animals, received 1 μg and 5 μg hybrid protein respectively, in 0.5 ml doses at each immunization. Nonimmunized animals served as controls.

For determination of antibody responses, sera from mice were pooled for each group while guinea pig and rabbit sera were assayed individually. Anti-CS antibody titers were measured by ELISA assay using the recombinant CS protein R32tet32 as antigen (See, Young et al., Science, 228, 958-962, 1985). Anti-HBsAg titers were determined by using the AUSAB kit (Abbott Laboratories) and the WHO reference.

Neither guinea pigs nor mice developed appreciable antibody responses to HBsAg despite repeated boosting. In contrast, both animal species developed anti-CS antibodies and these responses were augmented by boosting. An absorbance of 1.0 in the ELISA assay was obtained with serum dilutions of 1600 fold (mice) and 9000 fold (guinea pigs). Immunogenicity of the hybrid particles was not enhanced by adsorption to aluminum hydroxide.

In contrast rabbits developed antibodies to both CS and HBsAg. Reciprocal titers at an absorbance of 1.0 in the anti-CS ELISA assay were between 800 and 3200. Anti-HBsAg titers obtained with the hybrid particles adsorbed to aluminum hydroxide were between 4000 and 20,000 mUI/ml while the rabbits immunized with Heptavax gave titers of $10^5$ to $10^6$ mUI/ml. Adsorption of the hybrid particles to aluminum hydroxide enhanced the immune response.

Sera from mice, guinea pigs and rabbits all reacted strongly in the circumsporozoite precipitin reaction as well as inhibited sporozoite invasion of hepatoma cells in vitro (Young et al., Science, 228, 958-962, 1985). (See Table IV). Both reactions are indicative of a protective immune response.

Table IV. Circumsporozoite precipitin (CSP) reactivity, and percent inhibition of sporozoite invasion (% ISI) of HepG2-A 16 hepatoma cells

| Animal(s) | CSP Reactivity | | | % ISI |
|---|---|---|---|---|
| 1 week post 2nd boost | (4+ | 2+ | 0) | |
| Pooled mouse sera | 14 | 9 | 2 | 91% |
| Rabbit #36 | 22 | 3 | 0 | 96% |
| Guinea pig #1107 | 24 | 1 | 0 | 100% |

Degree of CSP reactivity is shown in parentheses:

0  - No CSP reactivity detectable

2+ - granular precipitate on surface of sporozoite

4+ - threadlike filament from end of sporozoite

Example 15. Vaccine Preparation

An illustrative vaccine of the subject invention is prepared as follows: To a buffered, aqueous solution of 3% aluminum hydroxide (10 mM sodium phosphate, 150 mM NaCl, pH 6.8; sterilized by filtration), the particle of the invention in similar buffer is added with stirring to a final concentration of 100 µg/ml of polypeptide and 0.5 mg/ml of aluminum ($Al^{3+}$). The pH is maintained at 6.8. The mixture is left overnight at about 0°C. Thimersol is added to a final concentration of 0.005%. The pH is checked and adjusted, if necessary, to 6.8.

While the above fully describes the invention and all preferred embodiments thereof, it is to be appreciated that the invention is not limited to the embodiments particularly described but rather includes all modifications thereof coming within the scope of the following claims.

EXAMPLES OF Pre S2-S PROTEIN AND PreS1-Pre S2-S PROTEIN PRODUCTION BY YEAST

It has been discovered that the insertion of the Pre S2-S protein coding region of this invention in a yeast expression vector into a yeast results in the synthesis of particles resembling the authentic 22 nm HBsAg particles exposing glycosylated Pre S2 protein on their surface. It has not been previously correctly reported that yeast, transformed with any portion of the HBV genome, express a glycosylated product.

The following examples relate to the expression in yeast of the glycosylated form of a protein containing the Pre S2 region in addition to the S protein of Hepatitis B surface antigen. The protein can be extracted and purified from yeast cells in the form of particles for use as a human vaccine. The Pre S1-Pre S2-S protein expressed in yeast cells is also glycosylated and may also be extracted and purified therefrom in the form of particles for use in a vaccine against Hepatitis B virus.

With respect to the finding of glycosylation, the following should be noted:

1. Examples 25, 26 and 27 concern concanavalin A binding, tunicamycin effect and sensitivity to Endo H, respectively. Each prove that the pre S2-S protein species of 33Kd is glycosylated. Thus, glycosylation has been proven by three independent methods.

2. The specificity of these reagents are, in general terms:

a) concanavalin A binds preferentially to mannose residues.

b) tunicamycin inhibits all types of glycosylation that are N-glycosidically bound to asparagine.

c) endoglycosidase H cleaves asparagine-linked high mannose oligosacchardies leaving one N-acetylglucosamine residue attached to the asparagine.

3. From the combined results of Examples 25, 26, 27 and the above, it is deduced that there is a N-linked oligosaccharide chain present on the 33Kd form of the pre S2-S protein (tunicamycin inhibition, sensitivity to Endo H) which is of the high-mannose type (sensitivity to Endo H). The shift in molecular weight observed (about 3000) upon tunicamycin treatment and Endo H digestion, indicates that the oligosaccharide structure is composed of about 10 sugar residues. This is about the size of a core glycosylation in yeast. Thus, probably only one of the three potential glycosylation sites in the Pre S2-S protein is glycosylated.

4. The purification schemes describeD in Examples 28 and 29 both contain an affinity chromatography step based on sugar residues of a glycoprotein.

5. Phenylboranate has a specificity for 1,2-cis-diol groups (2 adjacent OH groups sterically positioned in the same direction).

6. Lentil Sepharose is glucose/mannose specific.

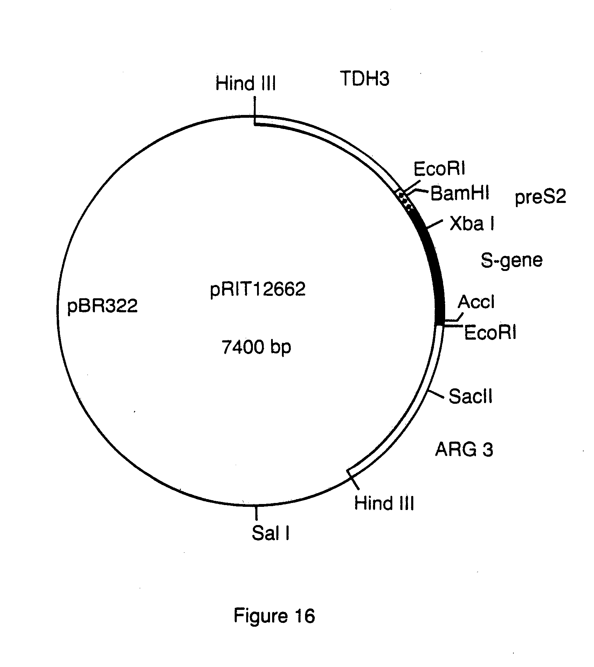

Example 16. Construction of pRIT12662 - an E. coli vector containing the PreS2-S expression cassette (Figure 16).

Plasmid pRIT12290, obtained as described in Example 3, above, was modified by the insertion of a BamHI-EcoRI synthetic adaptor fragment encoding the N-terminal amino acids of the pre S2 region between the TDH3 promoter and ARG3 terminator fragments. This was done as follows:

Plasmid pRIT12290 was digested with BamHI and EcoRI enzymes and dephosphorylated with alkaline phosphatase. 200 pmoles of the phosphorylated synthetic adaptor.

```
                         Gln Trp

BamHI        5'  GATC CAG TGG        3'          EcoRI

             3'       GTC ACCTTAA    5'
```

were ligated with 0.1 pmoles of the BamHI-EcoRI dephosphorylated plasmid pRIT12290 using 1 unit (U) of T4 ligase and used to transform the E. coli strain MM294 to ampicillin resistance. One transformant harboring the desired plasmid with the synthetic adaptor inserted between the BamHI and EcoRI sites was identified and retained. The plasmid is identified as pRIT12621. Starting with plasmid pRIT12621, the next steps were the elimination of the four extra base pairs (the core of the BamHI site) in order to put the ATG codon in phase with the second codon of the Pre S2 region, and the insertion of an EcoRI fragment containing the rest of the pre S2 coding region and the complete S coding region into the EcoRI site of pRIT12621 to complete the entire pre S2-S coding region.

At this stage the DNA manipulations including subsequent linearization, deletion and recircularization of the plasmid pRIT12621, were realized without amplification by transformation of intermediate plasmids: the procedure was as follows:

30 pmoles (120 μg) of pRIT12621 DNA were linearized with 120 U of BamHI. After elimination of the restriction enzyme by phenol extraction, the plasmid was ethanol precipitated and resuspended in the appropriate buffer to digest the BamHI single strand extensions with 280 units (U) of mung bean nuclease. The nuclease was eliminated by phenol extraction followed by ethanol precipitation. The so treated plasmid was resuspended in the ligation buffer and recircularized with 10 units of T4 ligase.

The preparation containing the ligated plasmid was phenolized, ethanol precipitated and resuspended into appropriate buffer and digested with EcoRI enzyme. After elimination of EcoRI endonuclease by phenolization and ethanol precipitation, 0.05 pmoles (0.2 μg) of DNA were resuspended in the ligation buffer in order to be ligated with 0.4 pmoles (0.2 μg) of a 838 base pair EcoRI fragment isolated from pRIT12581 that contains the entire C-terminal coding region of the pre S2-S protein. A plasmid essentially similar to pRIT12581 can be constructed as follows. The vector pUC9 (available commercially from Amersham and Pharmacia) is digested with EcoRI endonuclease and treated with alkaline phosphatase (Shine, U.S. patent 4,264,731). Plasmid pRIT10616 (ATCC 39131), is digested with EcoRI and AccI restriction endonucleases and the 826 EcoRI-AccI fragment containing part of the pre S2-S coding region is recovered by preparative acrylamide gel electrophoresis and electroelution. About 0.4 pmoles (0.2 μg) of this fragment is mixed with about 10 pmoles of the following 12 bp synthetic adaptor previously annealed as

```
                              Ile

                    Tyr     Stop

       5'         ATACATTTAACG           3'

AccI                                          EcoRI

       3'         TGTAAATTGCTTAA          5'
```

described in Example 8, above, and the mixture is treated with T4 DNA ligase and digested with EcoRI restriction endonuclease. To the so treated mixture is added about 0.2 μg of the EcoRI digested alkaline phosphatase treated pUC9 vector, prepared as described above, and the mixture is treated with T4 DNA ligase and used to transform competent cells of E. coli K12 to ampicillin resistance by conventional procedures. Colonies from the transformant are examined for the presence of a plasmid consisting of the 2650 bp pUC9 vector together with a 838 bp EcoRI fragment comprised of the 826 bp EcoRI-AccI pre S2-S fragment together with the 12 bp AccI-EcoRI synthetic linker. The correctness of the construction so identified can be verified by DNA sequencing using the chemical modification method of Maxam and Gilbert (Methods in Enzymology, 65, 499, 1980), preferably from a unique flanking restriction site in the pUC9 polylinker.

The ligation mixture containing plasmid DNA of pRIT12621, treated as described above, and the 838 bp EcoRI fragment of pRIT12581 was used to transform the E. coli strain MM294 according to the method of Cohen et al., cited above. One transformant having a plasmid with the EcoRI fragment in correct orientation was retained and the plasmid is identified as pRIT12662 (See, Figure 1A and Figure 16). Figure 1A is a flow sheet illustrating the preparation of pRIT12662. Figure 16 is a restriction endonuclease map of pRIT12662. The pre S2 region from pRIT12662 was sequenced by the chemical modification procedure of Maxam and Gilbert (Methods in Enzymology, 65, 499, 1980) to check that the coding reading frame at the N-terminus was as follows:

```
         Met Gln Trp Asn Ser

AAAC AAAC AAA ATG CAG TGG AAT TCC

    pTDH3                pre S2-S

              coding region
```

It will be appreciated by one skilled in the art that the vector pRIT12662 may also be used for the introduction of further functional DNA sequences into the preS2 region by appropriate in vitro manipulation of vector DNA. The preS2 coding sequence contains restriction sites for EcoRI, PstI and BamHI endonucleases. Any of these sites may be used for introduction of functional DNA sequences encoding peptides of interest to create in phase fusions with the preS2 region. Such fusions will be of the type preS2 - introduced sequence - preS2-S. These restriction sites may also be manipulated in vitro to create other vectors and provide other sites or reading frames for insertion of functional DNA sequences, such as by the method of Botstein et al. (Science, 229, 1193-1201, 1985).

Example 17. Construction of pRIT12377 and of pRIT12660 a yeast plasmid expressing the preS2-S protein

DNA of plasmid pRIT12309, obtained as described in Example 10(B), was digested with SalI endonuclease and ligated with a 2218 bp XhoI-SalI fragment of DNA containing the yeast LEU2 gene obtained by digestion of YEp13 and purification of the fragment by acrylamide gel electrophoresis. The ligated mixture was used to transform cells of E. coli K12 strain JA221, prepared according to the procedure of Cohen et al., cited above, with selection being made for both ampicillin resistance and leucine independence. Strain JA221 was obtained from M. Rosenberg, Smith Kline and French Laboratories, Philadelphia, Pennsylvania, U.S.A., and is available from the American Type Culture Collection under accession number 33875. A plasmid, pRIT12377, was identified from one transformant colony which contains the 2218 bp LEU2 XhoI-SalI fragment inserted at the SalI site of pRIT12309.

The orientation of the fragment on the vector is such as to recreate the SalI site on the AvaI side of the pBR327 part of the vector. Plasmid pRIT12377 is an E. coli - yeast shuttle vector having the necessary functions for selection, replication and maintenance in both organisms.

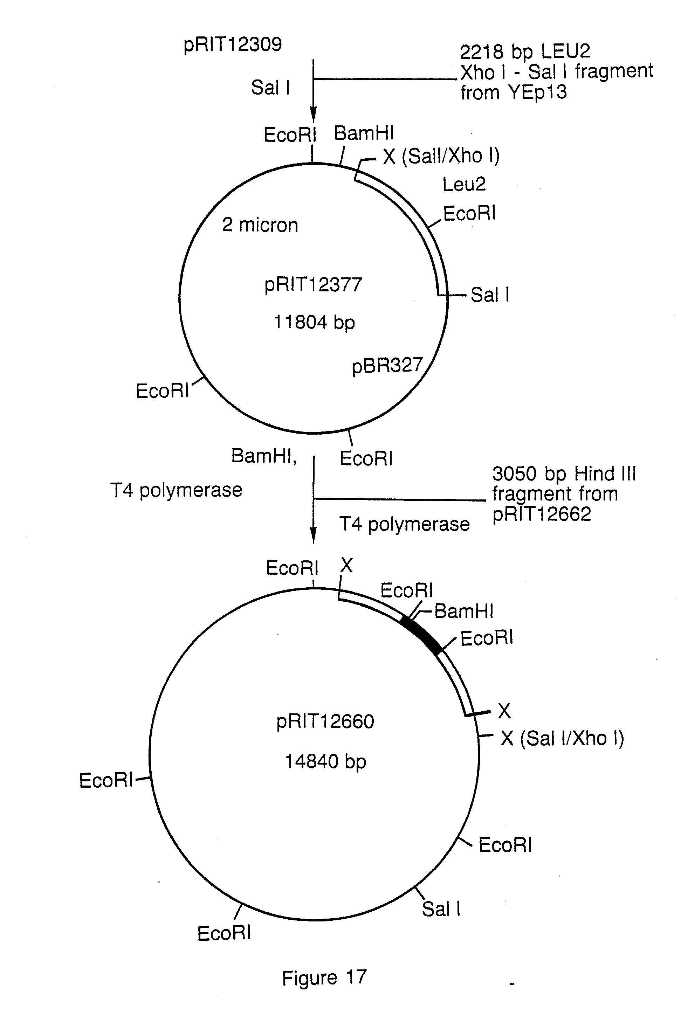

The 3050 base pair HindIII fragment from pRIT12662 (Example 16), containing the pre S2-S expression cassette was purified by acrylamide gel electrophoresis, treated with T4 polymerase and ligated to pRIT12377 previously opened by BamHI enzyme and T4 polymerase repaired. This preparation was used to transform strain E. coli MM294 to ampicillin resistance according to the method of Cohen et al., cited above. An E. coli transformant harboring the plasmid pRIT12660 was retained. Figure 17 is a flow sheet illustrating preparation of pRIT12660.

This plasmid, pRIT12660, was used to transform two strains of S. cerevisiae, i.e., strain DC5 cir° and strain 10S44C cir°, according to the method described by Hinnen et al. (Proc. Natl. Acad. Sci., U.S.A., 79, 2157-2161, 1978).

Both strain DC5 cir° and 10S44C cir° were deposited in the American Type Culture Collection, Rockville, Maryland, U.S.A., in accordance with the Budapest Treaty on August 18, 1986 under accession numbers ATCC 20820 and ATCC 20818, respectively.

Example 18. The preS2 region in plasmid pRIT12662.

The preS2 region in plasmid pRIT12662 was sequenced using the chemical modification method of Maxam and Gilbert, Proc. Natl. Acad. Sci., 74, 560-564, 1977) and it was found that there were four base pair changes leading to three amino acid changes when compared to another virus of adw2 serotype (Valenzuela et al., 1980 - Table I page 11). An alanine residue instead of a threonine residue at position -45, a phenylalanine instead of a leucine at position -34, and a serine residue instead of an isoleucine residue at position -11. The last change affects a residue that had been found invariable in all known serotypes until now (Lo et al., Biochem. Biophys. Res. Com., 2, 382-388, 1986).

Example 19. Synthesis of particles carrying a receptor for polymerized human serum albumin (pHSA)

Yeast (S. cerevisiae) strains DC5 cir° or 10S44C cir° containing either pRIT12660 or pRIT12363 (Example 16) were grown in selective medium [200 ml YNB + 80 μg/ml histidine (DC5 cir°) or YNB (10S44C cir°)] into log phase. (pRIT12363 is identical to pRIT12660 except for the hepatitis derived gene; pRIT12363 contains the S gene only). Cells were collected by centrifugation and resuspended in 50 mM sodium phosphate pH 8.0 containing 0.5% Tween 20 (polyethylene sorbitan monolaurate), 1 mM PMSF (phenylmethylsulfonyl fluoride) and 2.5% isopropanol. Cells were disrupted by passaging twice through a French Press at 20,000 psi (1.38 x $10^8$ Pa). The suspension was centrifuged for 30 min. at 30,000 X g. Total protein concentration in the supernatant liquid (crude cell extract) was measured by the method of Lowry et al., (J. Biol. Chem., 193, 265-275, 1951), with bovine serum albumin as standard. The presence of HBsAg related material was assayed using a radioimmune assay kit (AUSRIA II) commercially available from Abbott Laboratories. The results (Table V) indicate that pRIT12660 directs the synthesis of material antigenically related to HBsAg particles. The expression level, based on AUSRIA activity, is about the same in yeast strains DC5 cir° and 10S44C cir°.

In a radioimmune assay to detect a receptor for polymerized human serum albumin (pHSA) (Pontisso et al., J. Virol Methods, 6, 151-159, 1983), crude cell extracts from pRIT12660 containing cells gave a positive reaction while crude cell extracts from pRIT12363 containing cells did not. No reaction above background was observed with polymerized bovine serum albumin.

CsCl equilibrium centrifugation of crude cell extracts from pRIT12660 containing cells showed the AUSRIA positive material to band around rho = 1.20 g/cm$^3$ as do serum derived or yeast derived HBsAg particles. Material recovered from this portion of the gradient gives a positive result in the pHSA test.

The aforementioned results show that in pRIT12660 containing yeast cells a HBsAg related protein is expressed which is assembled into a structure like that of 22 nm particles from serum and exposing a receptor for pHSA on its surface.

## Table VA.   AUSRIA activity in crude cell extracts

| Strain | Plasmid | Protein mg/ml | HBsAg related antigenicity ng/ml | HBsAg related antigenicity ng/mg protein |
|--------|---------|------|------|------|
| DC5 cir° | pRIT12660 | 13.4 | 7 | 522 |
| DC5 cir° | pRIT12660 | 12.6 | 7 | 555 |
| 10S44C cir° | pRIT12660 | 10.8 | 6 | 556 |
| 10S44C cir° | pRIT12660 | 9.5 | 6 | 631 |

Example 20. Four S-related protein species are found to be expressed in yeast transformed with pRIT12660.

To analyze the HBsAg related protein monomers expressed in pRIT12660 containing cells, cells were grown in selective medium, either in Erlenmeyer flasks or in fermenters, into log phase and collected by centrifugation.

To the packed cells (0.1 gram), first 20 μl 40 mM PMSF in isopropanol, and then 200 μl sample buffer for SDS-polyacrylamide gel electrophoresis (0.125 M tris-HCl, pH 6.8 containing 20% glycerol, 4% SDS, 6 M urea and 10% 2-mercapto-ethanol), were added. Samples were vortexed and aliquots of 2 to 20 μl were further diluted in sample buffer to a final volume of 40 μl, incubated for 5 minutes at 100° and electrophoresed through a 12.5% separating, 5% stacking gel according to the method of Laemmli (Nature, 227, 680, 1971).

After electroblotting (Towbin et al., Proc. Natl. Acad. Sci., U.S.A., 76, 350-4354, 1979) of the proteins onto a nitrocellulose filter (0.45 μm, BioRad), the filter was preincubated for one hour at 37°C with 3% gelatin in PBS. The filter was incubated with a monoclonal antibody directed against a denaturation and reduction resistant epitope of human derived HBsAg (monoclonal 6 obtained from H. Thomas, Royal Free Hospital, London, England) and antibody binding was detected by successive incubation with biotinylated sheep anti-mouse Ig (RPN 1021, Amersham, dilution 1/250 in PBS containing 1% gelatin), steptavidin-biotinylated horseradish peroxidase complex (RPN 1051, Amersham, dilution 1/400 in PBS containing 1% gelatin) and finally 30 μl H$_2$O$_2$ and HRP Color Development reagent containing 4-chloro-1-naphtol (BioRad), 30 mg in 10 ml methanol, in 50 ml PBS. Between incubations the filter was washed with PBS containing 0.1% Tween 20.

The immunoblot showed 4 protein bands related to the S protein (p23) of estimated molecular weights of about 33Kd, 30Kd, 28Kd and 25Kd which are detected by the monoclonal antibody. The majority of S related material is found in the 33K protein band and was found to migrate slightly faster than the gp33 protein described by Stibbe and Gerlich (Virology, 123, 436-442, 1982). The smallest protein band detected migrates slower than the S protein (p23) synthesized in yeast.

Example 21. The four S-related protein species (of 33Kd, 30Kd, 28Kd and 25Kd) are assembled in particles.

Crude cell extracts of pRIT12660 (Example 16) containing yeast cells were prepared as described in Exampled 19 or by grinding yeast cells in a Dynomill in the presence of an equal weight of extraction buffer (200 mM tris, 3 M NaCl, 10 mM EDTA, 10 mM ethylene glycol bis (beta-aminoethyl ether) N,N,N',N'-tetraacetic acid (EGTA), 4 mM PMSF, 10% isopropanol) and subsequent centrifugation for 45 minutes at 30,000 X g.

The pRIT12660 directed particles were partially purified from the crude cell extract by ultrafiltration, CsCl equilibrium centrifugation and hydroxyapatite chromatography which are methods known in the art. The four protein bands of S-related material detected by Western blot analysis copurify during these purification steps. An immuno-blot analysis (as described in Example 20) of the AUSRIA positive CsCl fractions after equilibrium centrifugation showed that the four S-related bands were present in the same relative proportions in each fraction.

The material is thus distributed in homogenous fashion throughout the peak fractions of the gradient.

Example 22. The 33Kd and 30Kd proteins carry a receptor for polymerised human serum albumin.

Human serum albumin (Sigma, A 8763) was polymerised by glutaraldehyde treatment (Merck, A-12179) and purified according to Pontisso et al (J. Virol. methods, 6 151-159, 1973). Reactivity of the four S-related proteins towards pHSA was tested in a Western blot procedure. Partially purified preparations of pRIT12660 directed particles (Example 21) were separated by SDS polyacrylamide gel electrophoresis and transferred to a nitrocellulose membrane as described in Example 20. The nitrocellulose filter was successively incubated with pHSA (15 μg/ml in PBS containing 1% gelatine), rabbit anti-serum to human albumin (Behringwerke, ORCB 04/05, 1/125 dilution in PBS containing 1% gelatin), biotinylated donkey anti-rabbit Ig (Amersham RPN 1004, 1/250 dilution in PBS containing 1% gelatin), streptavidin-biotinylated horseradish peroxidase (Amersham RPN 1051 1/400 dilution in PBS containing 1% gelatin) and finally with $H_2O_2$ and 4-chloro-1-naphtol as described in Example 20.

The two largest S-related protein bands (the 33Kd and 30Kd) reacted with pHSA, the two smallest ones (28Kd and 25Kd) gave no reaction.

Example 23. The 33Kd and 30Kd proteins contain a sequence specific for the preS2 region of the preS2-S protein

A peptide corresponding in 23 of 26 positions to the 26 N-terminal amino acids of the preS2-S protein was synthesized ($NH_2$-Met-Glu-Trp-Asn-Ser-Thr-Ala-Phe-His-Gln-Ala-Leu-Gln-Asp-Pro-Arg-Val-Arg-Gly-Leu-Tyr-Leu-Pro-Ala-Gly-Cys-COOH) and coupled via the C-terminal to keyhole Limpet hemocyanin (Peninsula Laboratories). Rabbits were immunized subcutaneously with 100 μg of the conjugate (which corresponds to 20 μg peptide) in Freund's complete adjuvant, boosted at days 8 and 15 with another 100 μg conjugate in Freund's complete adjuvant and at days 28, 69 and 149 with 100 μg conjugate in PBS. Development of antibody titer was followed by taking blood samples at regular intervals and testing serum dilutions by incubation on solid-phase fixed synthetic peptide. Bound antibodies were detected by iodinated protein A (New England Nuclear). For immunoblot applications, sera with antipeptide titers greater or equal to 1/5120 were selected. The serum was used as such after a 100 fold dilution or after partial purification of IgG. IgG was partially purified by precipitation with $Na_2SO_4$ (120 mg per ml of serum), resuspension in PBS and reprecipitation with 14% $Na_2SO_4$. The resuspended IgG solution was desalted by passage on a PD10 column (Pharmacia).

Detection of reactivity of these antibodies towards the S-related proteins in immunoblots was by biotinylated donkey anti-rabbit Ig, streptavidin-biotinylated horseradish peroxidase and $H_2O_2$ and 4-chloro-1-naphtol as described in Example 22.

Proteins from partially purified preparations of pRIT12660 directed particles (Example 21) were separated by SDS polyacrylamide gel electrophoresis and transferred to nitrocellulose as described in Example 20. Also run on the gel was S protein (p23) from particles purified from yeast harboring pRIT12363. The immunoblot showed that the two largest S-related protein bands (the 33Kd and 30Kd) react with the anti-peptide antibodies while the two smallest ones (28Kd and 25Kd) give no reaction. The S protein (p23) gives no reaction with the anti-peptide antibodies.

Example 24 Human anti-Hepatitis B antibodies from naturally infected persons recognize epitope(s) on the 33Kd and 30Kd preS2-S proteins not present on the S protein.

Reactivity of pooled gamma globulin from anti-hepatitis B positive human subjects (Belgian Red Cross, 100-150 IU/ml Lot 85A08) towards the four S-related proteins was tested by the immunoblot procedure described in Example 20. A partially purified preparation (Example 21) of pRIT12660 directed particles was dissociated by SDS and the proteins separated by SDS polyacrylamide gel electrophoresis. After transfer of the proteins to nitrocellulose, the membrane was incubated with a ten-fold dilution of anti-Hepatitis B gamm globulin in PBS containing 1% gelatine. Antibody binding was detected by successive incubation with biotinylated sheep anti-human Ig (Amersham RPN1003, 1/250 dilution in PBS containing 1% gelatin), steptavidin-biotinylated horseradish peroxidase and finally with $H_2O_2$ and 4-chloro-1-naphtol as described in Example 20.

The two largest S-related protein bands (the 33Kd and 30Kd band) react with the human antibodies, the two smallest ones (the 28Kd and the 25Kd band) give no reaction. The S protein (p23) derived from particles from yeast cells containing pRIT12363 does not react with these human antibodies. These results show that gamma globulin obtained from infected persons specifically recognizes epitopes present on denatured and reduced preS2-S proteins which are absent on denatured and reduced S protein. It is known that the epitopes present on S protein derived from human serum and yeast are predominantly conformational.

Example 10A. The 33Kd protein species carries a mannosyl containing oligosaccharide structure.

PreS2-S protein from partially purified preparations of particles from pRIT12660 containing 10S44C cir° cells was electrophoretically separated and transferred to nitrocellulose as described in Example 20. Also run on the gel were S protein from particles purified from yeast containing pRIT12363 and HBsAg particles from infected human serum obtained from Dr. W.H. Gerlich, Department of Medical Microbiology, University of Göttingen, Federal Republic of Germany.

One half of the nitrocellulose filter was incubated in 50 µg/ml concanavalin A (Calbiochem A grade) in 10 mM tris pH 7,5, 150 mM NaCl 1 mM $CaCl_2$ 1 mM $MnCl_2$ 0.05% Tween 20 and subsequently with 30 µg/ml horseradish peroxidase (Sigma type VI P 8375) in 10 mM tris pH 7.5 150 mM NaCl 0.05% Tween 20 and finally $H_2O_2$ and 4-chloro-1-naphtol as described in Example 20.

Between incubations the filter was washed with 10 mM tris pH 7.5 150 mM NaCl 0.05% Tween 20 (Hawkes, Anal Biochem, 123, 143-146, 1982; Clegg, Anal. Biochem.,127, 389-394, 1982). The other half of the nitrocellulose filter was incubated with monoclonal antibody 6 and treated as described in Example 20.

The 33K protein band reacts with the concanavalin A-peroxidase reagents while none of the 30Kd, 28Kd, 25Kd protein bands or the S protein derived from pRIT12363 containing cell reacts. In the presence of 0.1M methyl alpha-D-mannopyranoside the binding of concanavalin A to the 33K protein is abolished.

Example 26. An oligosaccharide structure is N-glycosidically bound to asparagine

pRIT12660 and pRIT12377 (Example 17) containing cells were grown in selective medium (YNB + 80 ug/ml histidine (DC5 cir°) or YNB (10S44C Cir°)] to $OD_{620nm} = 0.2$. Tunicamycin (Calbiochem) was added to a final concentration of 10 µg/ml and the cultures incubated for 30 min at 30°C. Then 20 µCi $^{35}$S-methionine (1000 Ci/mmol) (New England Nuclear) per ml was added and the cultures were incubated another 15 minutes. 2 ml of labeled cell culture, either treated or untreated with tunicamycin, were centrifuged in a microfuge and the packed cells were resuspended in 40 µl 1% SDS and disrupted by agitation with a Vortex mixer for 2 min. in the presence of 0.3 gram glass beads (diameter -0.45- 0.50 mm).

Immune precipitation was essentially according to Julius et al.,(Cell, 36, 309-318, 1984). The lysate was heated for 3 minutes (min.) at 100°, diluted with 0.5 ml PBS containing 1% Triton X100, 0.5% sodium deoxycholate 0.1% SDS and heated again for 1 min. at 100°.

25 µl of a 10% suspension of prewashed Immuno-precipitin (Formalin-fixed Staph A cells, Bethesda Research Laboratories) was added to the boiled extract and incubated at 0° for 30 min. The mixture was clarified by centrifugation for 5 min. in a microfuge. To the supernatant liquid, 2.5 µl monoclonal antibody 6 specific for S protein (see Example 20) was added, and the mixture was incubated for 1 hour at 0°. Another portion (25 µl) of Immuno-precipitin was then added and incubated for 30 min. at 0°. The immune complexes were collected by centrifugation (5 min. in a micro fuge) and washed with PBS containing 2M urea, PBS containing 1% 2-mercaptoethanol and finally PBS. The final washed pellets were resuspended in sample buffer for electrophoresis as in Example 20.

Samples containing $10^5$ cpm were electrophoresed through a 12.5% SDS polyacrylamide gel (see Example 20). After electrophoresis, gels were fixed in 40% methanol 10% acetic acid, treated with Amplify (Amersham) for fluorography, dried on filter paper under vacuum and subjected to fluorography using Kodak X-Omat S film at -70°.

This showed that pRIT12660 containing cells synthesize a 33Kd protein in the absence of tunicamycin and a 30Kd protein in the presence of tunicamycin. Both bands are absent in the corresponding samples of pRIT12377 containing cells.

This results shows that the 33Kd preS2-S protein expressed in yeast strains DC5 cir° carries an oligosaccharide portion N-glycosydically bound to asparagine. There is most probably only one oligosaccharide chain attached to the 33Kd form of the preS2-S protein which is not much larger than a core glycosylation.

Example 27. The oligosaccharide structure is of the high-mannose type.

Crude cell extracts from pRIT12660 containing cells or partially purified preparations of preS2-S containing particles from these extracts (Example 21) were treated with Endo-N-acetylglucosaminidase (Endo H.; EC.3.2.1.96) from Streptomycesplicatus obtained from New England Nuclear.

Partially purified particles (50 µl, 450 µg/ml protein) were boiled for 3 minutes at 100° in the presence of 0.1% sodium dodecyl sulphate, diluted ten-fold with 100 mM sodium-citrate pH5 and to 240 µl of this mixture 2.4 µl Endo H (74 µg/ml) was added. Samples with and without added Endo H were incubated for 4 hrs at 37°. Analysis of the Endo H treated and untreated samples by SDS-polyacrylamide gel electrophoresis and immunoblotting with detection by the monoclonal antibody 6 directed against the S protein as described in Example 20, showed the disappearance of the 33Kd band and the appearance of a 31Kd band upon Endo H treatment. The 31Kd band still reacts with the concanavalin A-peroxidase reagents as described above. The position of the other bands (30Kd, 28Kd and 25Kd) did not shift upon Endo H treatment. Longer incubation times or higher Endo H concentrations gave identical results. The result shows that the oligosaccharide structure linked to the 33Kd protein is of the high-mannose type and has about the size of a core glycosylation.

Example. 28 Purification of preS2-S protein containing particles.

Crude yeast extract containing preS2-S particles is prepared by grinding the washed yeast cells in a Dynomill in the presence of an equal weight of extraction buffer, either 50 mM sodium phosphate pH 8.1, 4 mM EDTA 1.0% Tween 20, 4 mM PMSF and 10% isopropanol or 200 mM tris-HCl pH 9.0, 3.0 M NaCl, 10 mM EDTA, 10 mM EGTA (ethylene glycol bis (beta)aminoethyl ether N,N,N',N'-tetra acetic acid), 1% Tween 20, 4 mM PMSF and 10% isopropanol.

The homogenate is adjusted to pH 8.0 or 9.0 respectively and subsequently centrifuged for 45 minutes at 30,000 X g.

To 500 ml of the crude yeast extract, adjusted to pH 7.2, is added 10 gr colloidal Silica (Aerosil 380 Degussa). The colloidal suspension is stirred over night at 4° and subsequently centrifuged at low speed. The Aerosil pellet is washed three times with 150 ml 0.15 M NaCl for 1/4 hour and then eluted batchwise with 250 ml 10 mM pyrophosphate buffer (pH 9.3) containing 2% Tween-20 at 37° for 3 hours. The desorbate, a yellowish, lightly opalescent solution, is passed over a 50 ml column of phenylboronate Agarose (Amicon) equilibrated in 10 mM pyrophosphate buffer pH 9.0. The flow rate is 15 ml/hour.

The column is further washed with 2 column volumes of equilibration buffer, and is then eluted in the reversed direction with 100 mM sorbitol in 50 mM Tris (pH 7.2) at a flow rate of 15 ml/hour. Eluted peak fractions are pooled and after adjustment to pH 8.1, passed over a DEAE-Fractogel TSK column equilibrated in 50 mM Tris pH 8.1. The particle is eluted in the same buffer containing 75 mM NaCl. After this step residual nucleic acids were removed (<1 µg/20 µg protein). The balance sheet is shown in Table VI.

## Table V. Two step purification of preS2-S containing particles

| Fraction | ml | mg S-related protein (RIA) | mg protein | mg lipids | mg poly-saccharides |
|---|---|---|---|---|---|
| Crude extract | 500 | 11.3 | 14,600 | 5,286 | 7.850 |
| Desorbate aerosil | 270 | 5.96 | 385 | 554 | 249 |
| Flow through PBA-column | 315 | 2.83* | 365 | 360 | 267 |
| Eluate PBA-column | 70 | 2.8 | 2.9 | 5 | 2.5 |

*not sufficiently glycosylated to be retained on the column.

Material purified from extracts of cells containing pRIT12660 as described in this Example 28, followed by a CsCl equilibrium centrifugation, was prepared for electron microscope examination after staining with uranyl acetate. Examination showed the presence of discrete particle structures about 19 microns in diameter, and that such hybrid particles contained between 5 to 20 µg Tween 20 per 100 µg protein.

Example 29. Affinity chromatography on lentil sepharose.

In this example, the phenylboronate agarose chromatography step is replaced by affinity chromatography on lentil sepharose (Pharmacia).

50 ml of Aerosil-desorbate, obtained as described in Example 28, was passed over a 5 ml column of lentil Agarose equilibrated in equilibration buffer such as Tris buffer 10 mM (pH 7.2), 0.14 M NaCl and 0.3% Tween. The column was washed with 2 column volumes of equilibration buffer such as Tris Buffer 10 mM (pH 7.2) and the eluted with sugar additive with specificity for lentil such as 0.5 M alpha-methylmannopyranoside in equilibration buffer. The balance sheet is shown in Table VII.

## Table VII.   Application on lentil Sepharose affinity
## chromatography in the purification on
## preS2-S containing particles.

| Fraction | Volume (ml) | μg S-related protein (RIA) | Total Protein |
|---|---|---|---|
| Crude | 100 | 2170 | 3 gr |
| Desorbate aerosil | 50 | 1700 | 138 mg |
| Flow through Lentil-sepharose | 50 | 428 | - |
| Eluate Lentil-sepharose | 12.5 | 925 | 1.26 mg |

Examination revealed that the hybrid particles purified as described herein contained between 5 to 50 μg Tween 20 per 100 μg protein.

Example 30. Immunogenicity of the preS2-S particle.

The immunogenicity of particles prepared from extracts of cells containing pRIT12660 (Example 16) was studied in 2 strains of mice: Balb/c (H2-d) and NMRI (H2-Q). The particles were absorbed on Al(OH)3 before injection and the mice (5 mice/group) were injected intraperitoneally (I.P.) with 0.3 μg of RIA reacting material. One month after the immunization, the mice were bled and the sera from the mice of the same group were pooled. The titer of anti-S antibodies was determined in the pools by using the AUSAB kit (Abbott Labs) and the WHO reference. The titers were converted in mIU/ml by using the Hollinger formula (Hollinger et al., in Szmuness et al., eds. Viral Hepatitis, Philadelphia : Franklin Institute Press, 451-466, 1982). The anti-preS2 antibodies were determined using a solid phase assay (RIA) in which the synthetic preS2 peptide given in Example 23, is absorbed on plastic and bound antibodies were detected with a goat anti-mouse IgG labelled with [125]I. The results summarized in Table VIII show that the particles from pRIT12660 containing cells induce both anti-S and anti-preS2 peptide antibodies in both strains of mice with higher titers of anti-preS2 peptide antibodies induced in NMRI than in Balb/c mice. The anti-S titer obtained in Balb/c or NMRI mice is 2 to 3 times higher than the titer obtained with a higher dose of particles from pRIT12363 containing cells which do not contain the preS2 sequence. PreS determinants enhance the response to the S determinants. This enhancing effect is not shown if the preS2 synthetic peptide is injected together with particles which lack the preS2. Moreover, the preS2 synthetic peptide alone is unable to elicit anti-preS2 antibodies.

This indicates that the association of preS2 determinants and S determinants on the same particle is required to obtain an anti-preS2 response. Furthermore this association results in an improved anti-S response.

The results of the immunogenicity studies referred to herein related to the PreS2-S particle are summarized in Table VIII.

## Table VIII.   Immunogenicity of the preS2-S particle

| Preparation injected | Dose of S related antigen (μg)(1) | Titre of Anti-S antibodies (mIU/ml)(2) | | Anti-preS2 antibodies (3) | |
|---|---|---|---|---|---|
| | | Balb/c | NMR1 | Balb/c | NMR1 |
| Yeast-derived S particle directed by pRIT12363 | 1.7 | 1404 | 719 | 0 | 2 |
| Yeast-derived S particle directed by pRIT12363 + synthetic preS2 peptide: 0.08 μg | 1.55 | 748 | 1163 | 0 | 4 |
| Synthetic preS2 peptide: 0.08 μg | – | 2 | 1 | 0 | 4 |
| Yeast-derived (preS2-S) particle directed by pRIT12660 | 0.3 | 3344 | 2418 | 8 | 120 |

(1) determined by using the AUSRIA Kit (Abbott Labs) and the Proposed International Reference Preparation supplied by Dr. Schild, National Institute of Biological Standardization, London, as standard.

(2) determined by using the AUSAB kit (Abbott Labs) and the World Health Organization standard.

(3) determined by using a solid phase radio immuno assay and synthetic preS2 peptide adsorbed to plastic.

The titer is the dilution which gives a significant binding of the antibody (2.5 times the value obtained with a negative control serum).

## EXAMPLES OF THE EXPRESSION OF THE ENTIRE PreS1-PreS2-S PROTEIN CODING SEQUENCE IN YEAST

Example 31. Construction of plasmid pRIT12845 that expresses the preS1-preS2-protein in yeast

The first step was the fusion of the TDH3 promoter region with the pre S1 N-terminal region from the HBV genome. The starting material was plasmid pRIT12792 that harbors a 495 bp NcoI-XbaI fragment containing the pre S1-Pre S2 coding sequence except for the last asparagine codon. The NcoI site overlaps the ATG codon of amino acid residue -163 of the HBV pre S1 sequence. The sequence of the NcoI-XbaI fragment is shown in Figure 4A.

An essentially similar plasmid to pRIT12792, identified as pRIT12793 (5940 bp), was deposited in the American Type Culture Collection, Rockville, Maryland, U.S.A., in accordance with the regulations of the Budapest Treaty under accession number ATCC 67193 on September 5, 1986. From plasmid pRIT12793, a 495 bp NcoI-XbaI fragment can be recovered which contains a complete pre S1-pre S2 coding sequence. This fragment differs from that shown in Figure 4A by one nucleotide having the sequence ACGAACTAATAATC-TAGA at the 3' end of the fragment. The nucleotide difference is underscored. In the following examples, pRIT12792 can be replaced with pRIT12793. Both the NcoI-XbaI fragments contained pRIT12792 and pRIT12793 were derived by in vitro manipulation of DNA from the PreS1-PreS2 region of the DNA of Hepatitis B virus strain (serotype adw) cloned on plasmid pRIT10616. Plasmid pRIT10616 is available from the American Type Culture Collection under accession number ATCC 39131.

52 μg of pRIT12792 were digested with the NcoI and XbaI restriction enzymes and treated with Mung Bean nuclease in order to eliminate the 5' extensions. 600 ng (2 pmol) of the treated and purified 495 bp NcoIXbaI fragment were ligated to 170 ng (0.02 pmol) of pRIT12314 vector that had been previously opened by BamHI and SmaI enzymes and also was treated with mung bean nuclease. pRIT12314 contains the entire 2 micron fragment from S. cerevisiae and an expression cassette in which the TDH3 promoter is separated from the ARG3 terminator by a BamHI-SmaI-EcoRI linker:

```
       BamHI          EcoRI


       _____       _____

              SmaI

              _____

. . . . . . . ATGGATCCCCGGGAATTC . . . .

   pTDH₃                        tARG₃
```

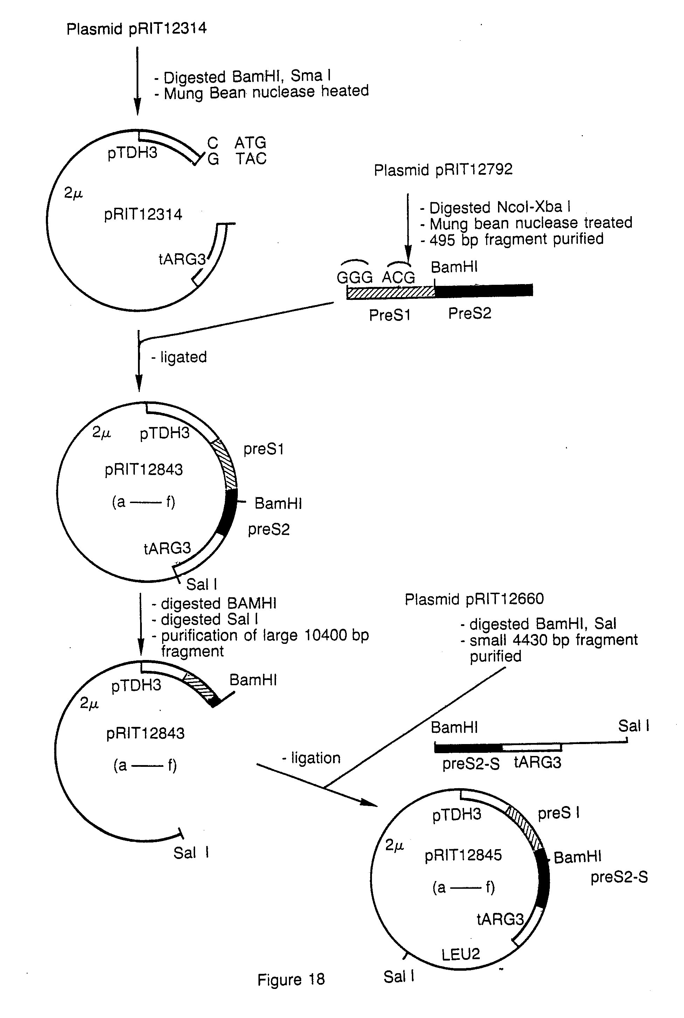

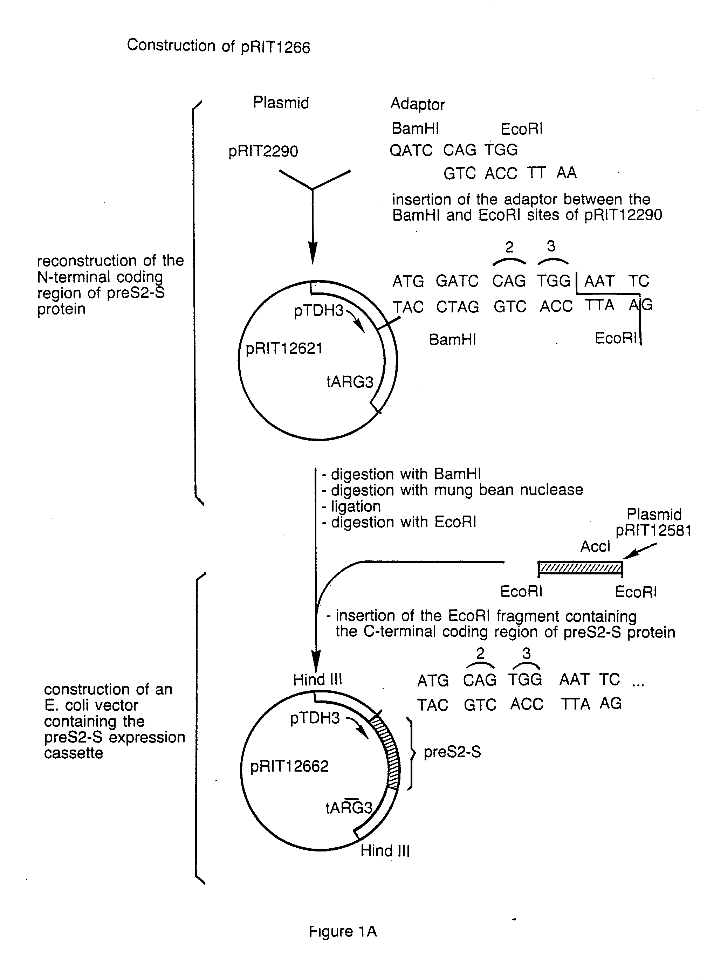

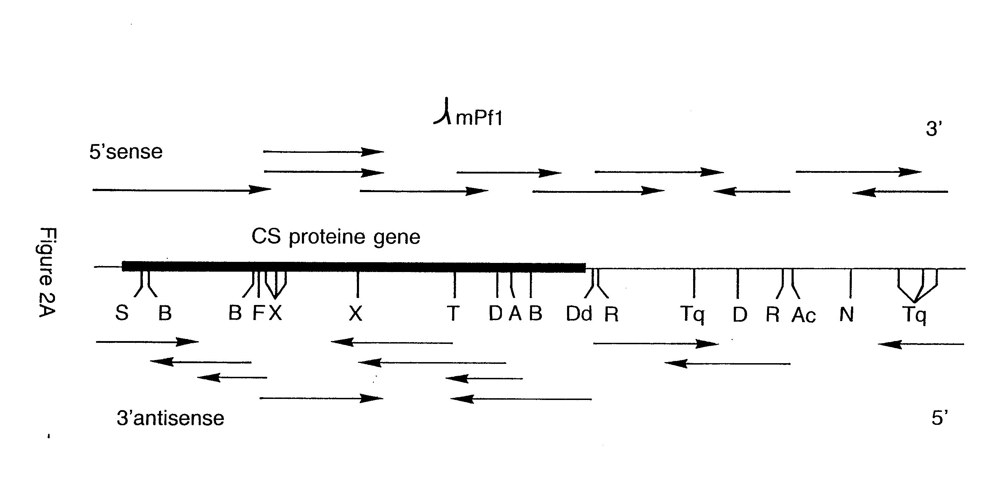

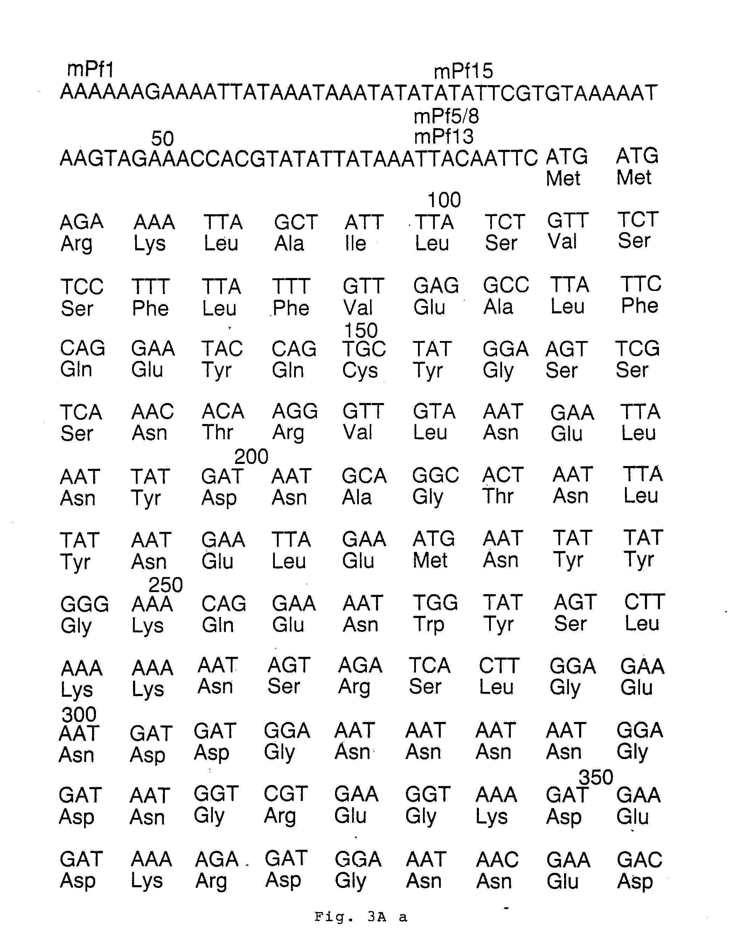

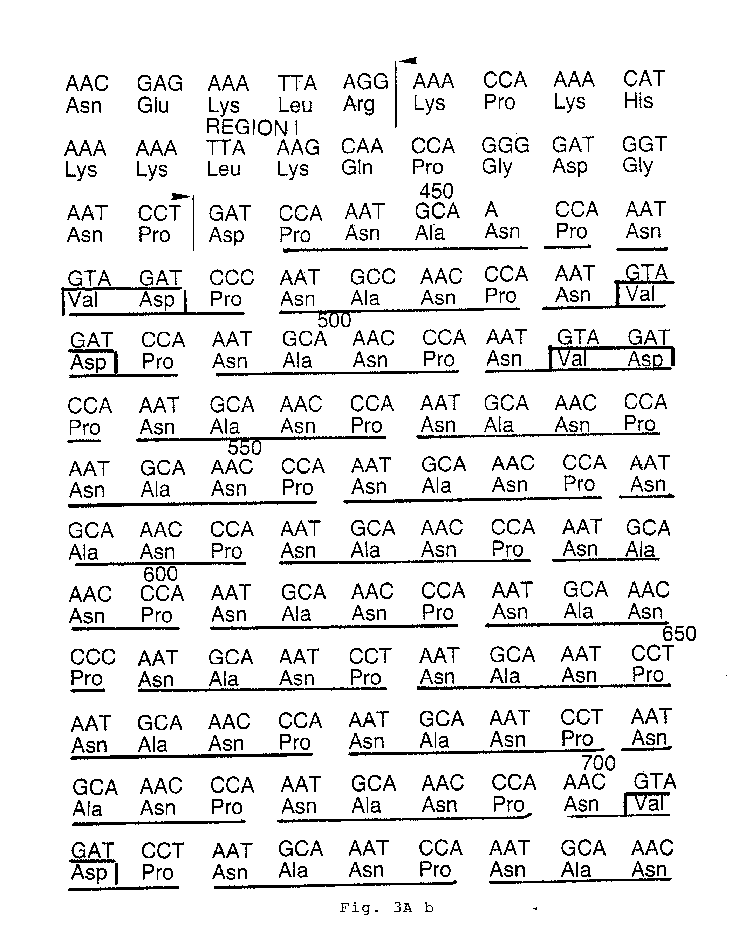

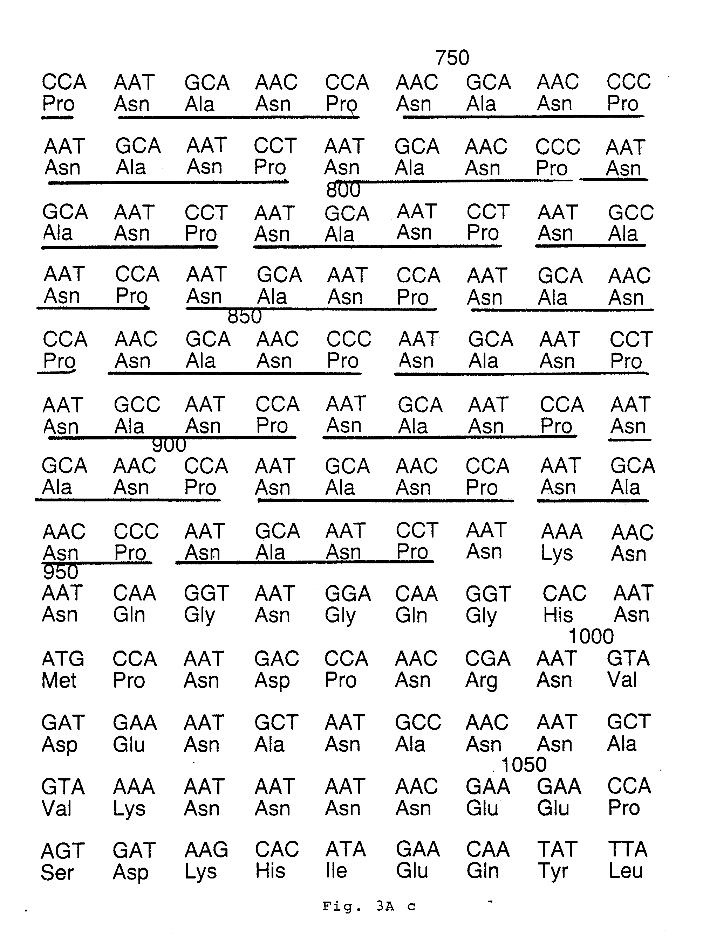

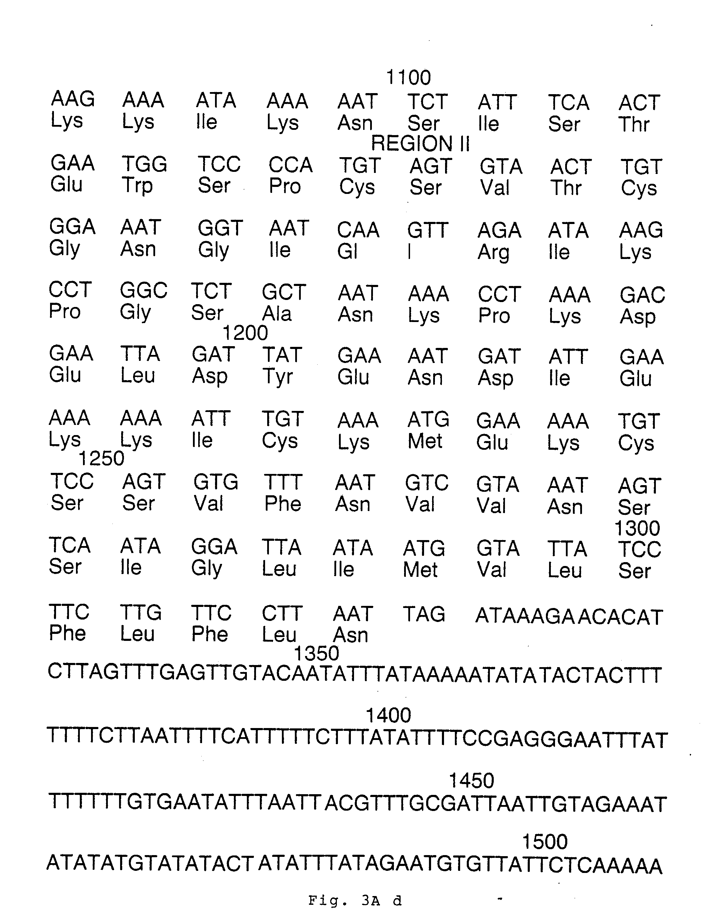

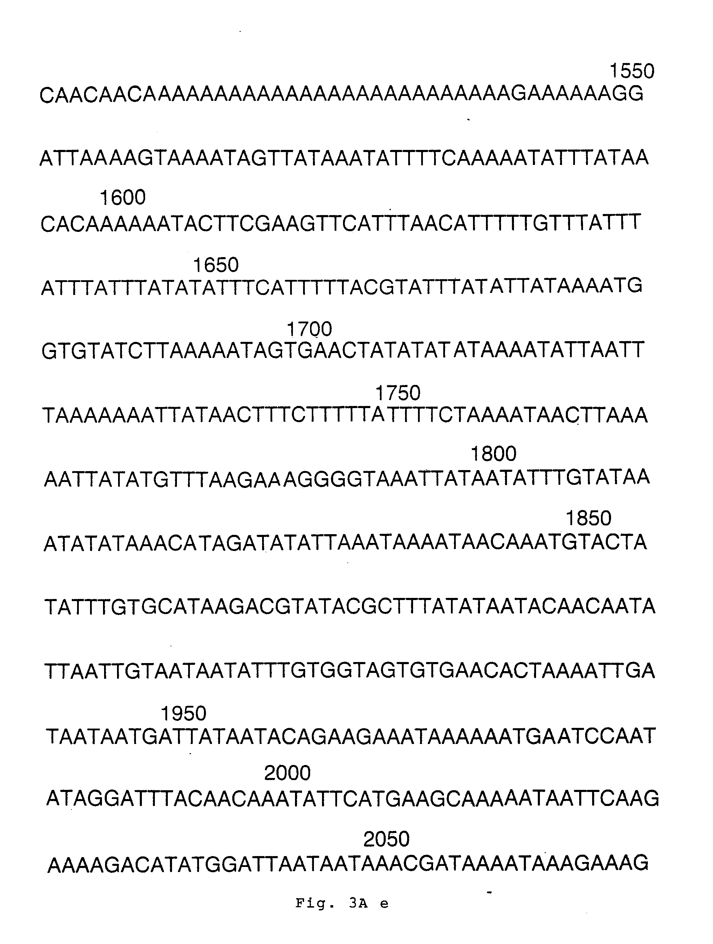

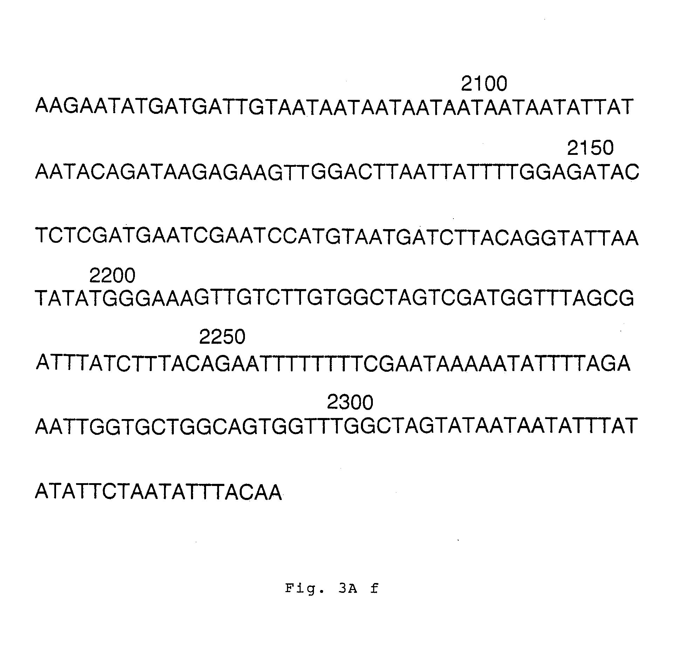

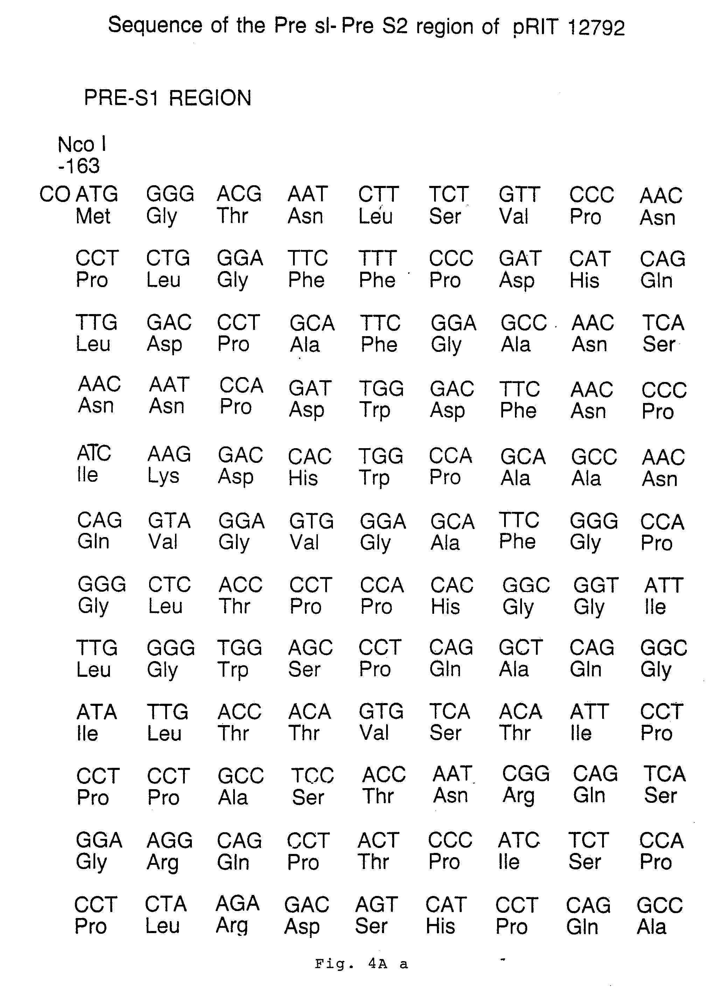

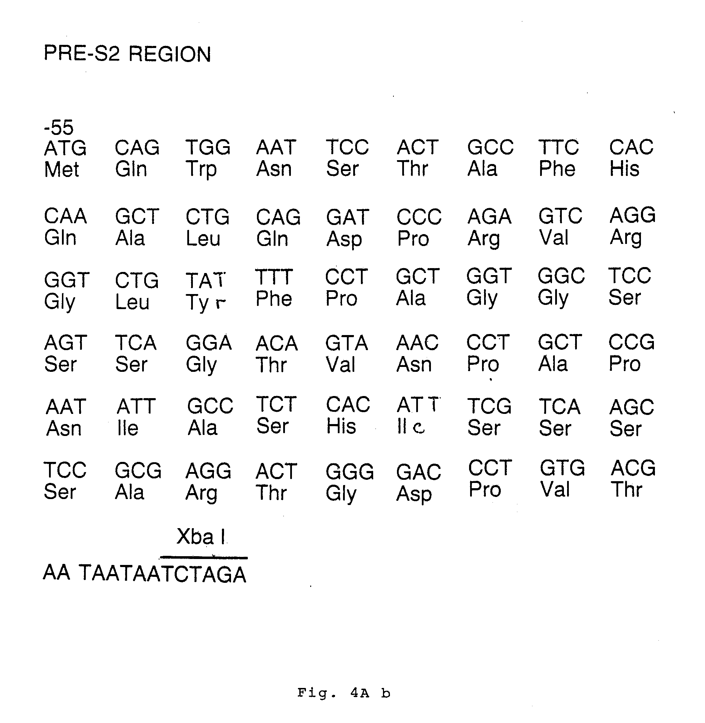

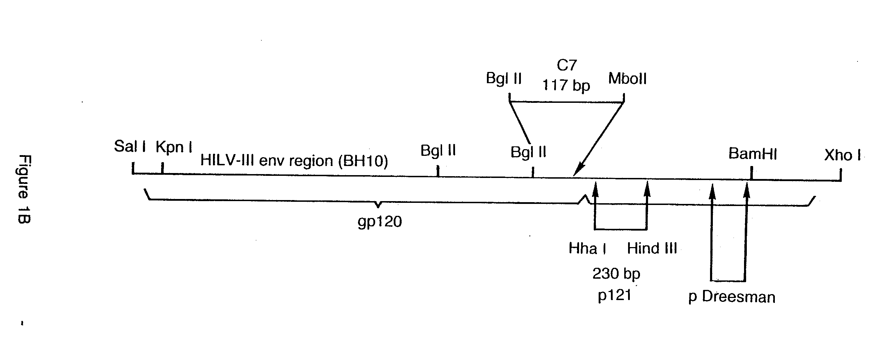

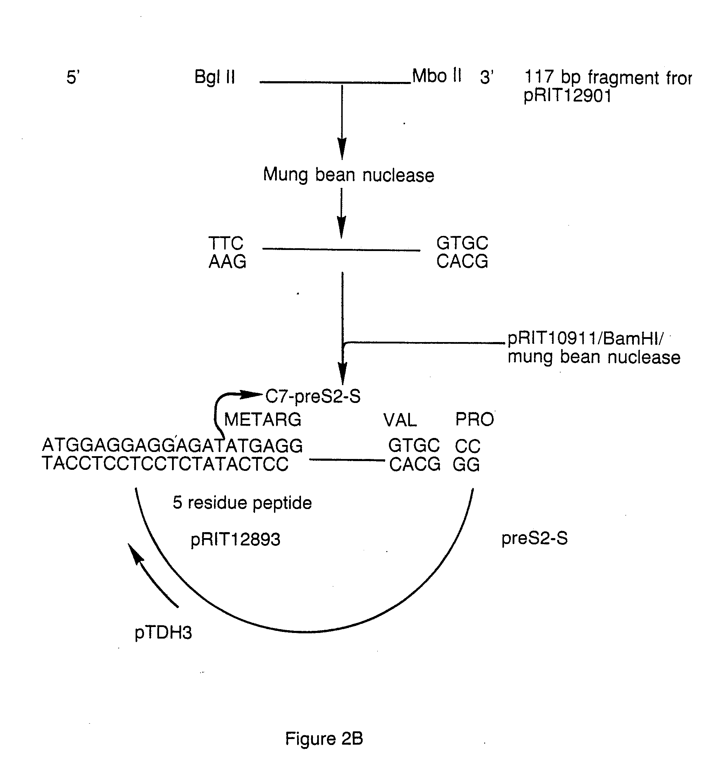

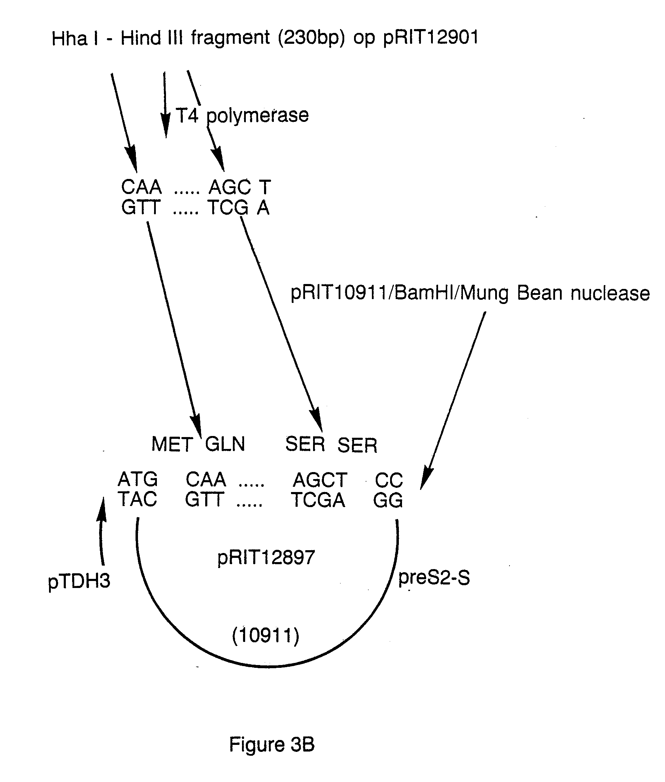

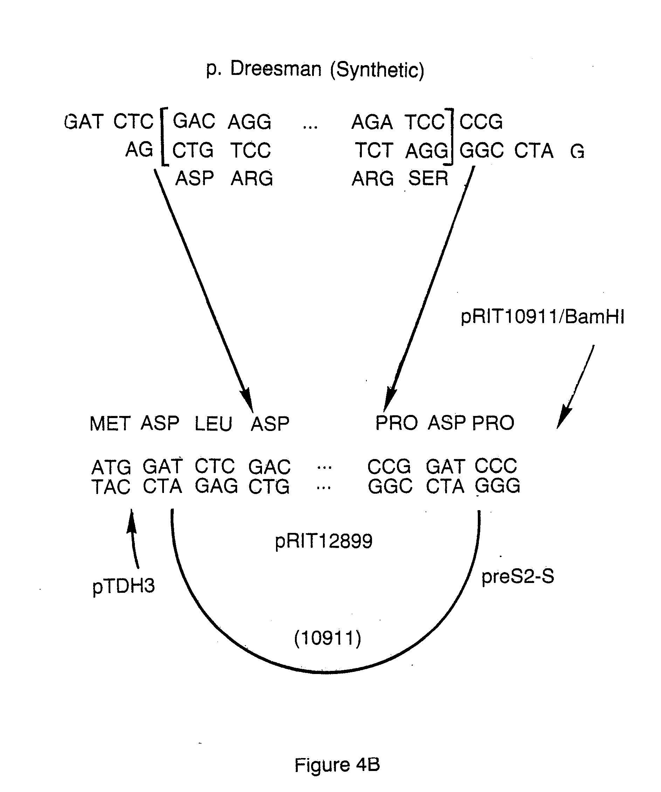

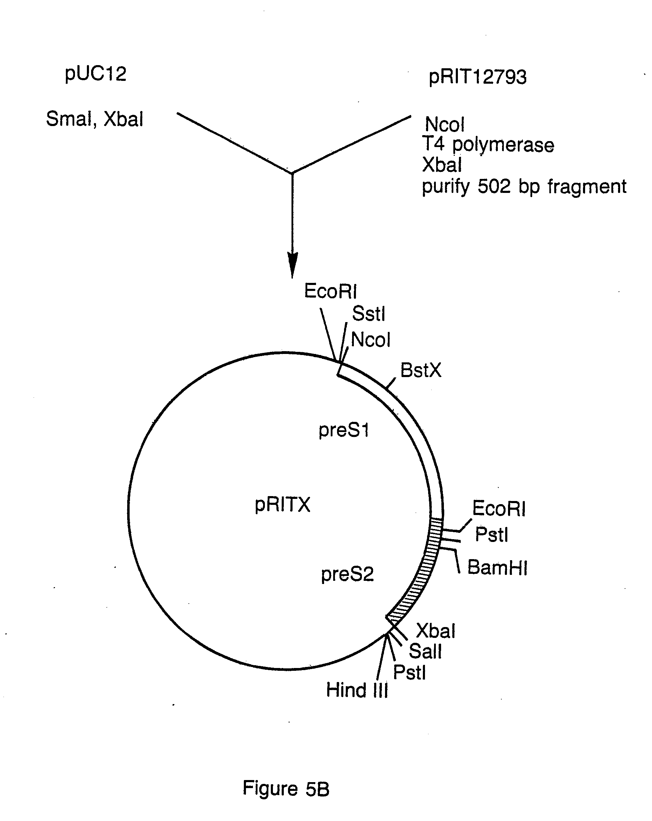

A more detailed description of the construction of pRIT12314 is found in Example 10(B) above. The ligation mix described above was used to transform E. coli MM294 to ampicillin resistance according to the method of Cohen et al., cited above. Six transformants containing a plasmid in which the pre S1-pre S2 fragment was inserted in the correct orientation were retained. These were the plasmids pRIT12843 (a.....f) (Figure 18).

The second step was the insertion into each plasmid pRIT12843 (a.....f) described above, of the S coding region to reconstitute the entire pre S1-S2-S gene.

This was done as follows: plasmids pRIT12843 (a.....f) were each digested with BamHI and SalI enzymes. The BamHI site is placed at the beginning of the pre S2 region, the SalI site is placed behind the ARG3 terminator in pBR 327. 80 ng (0.012 μmol) of the largest BamHI-SalI fragment (10,400 bp) purified from each plasmid pRIT12843 (a.....f) were ligated to 300 ng (0.11 pmol) of the small BamHI-SalI fragment from pRIT12660 (Example 16). The small (4800 bp) BamHI-SalI fragment of pRIT12660 contains part of the pre S2-S coding sequence followed by the ARG terminator sequences and the LEU2 yeast sequence. After ligation and transformation of E. coli MM294 with each of plasmids pRIT12843 (a.....f) according to the method of Cohen et al., cited above, a series of ampicillin resistant clones containing the plasmids named pRIT12845 (a.....f) was obtained. Figure 18 is a flow sheet illustrating preparation of pRIT12845 (a.....f). These plasmids pRIT12845 (a.....f), were used to individually transform the S. cerevisiae strain 10S44C cir° according to the method of Ito et al., cited above. The screening of cultures grown from individual clones from the yeast transformations was done by testing crude cells extracts in the commercially available AUSRIA test (Abbott). Crude cell extracts were made by disrupting yeast cells resuspended in PBS, containing 0.5% Tween 20, 2 mM PMSF and 5% isopropanol. One transformant of each of the six separate transformations [with pRIT12845 (a.....f)] was tested. Five out of six tested transformants gave a positive AUSRIA.

To test whether a protein of the correct size gave the S-related antigenicity detected by the AUSRIA, samples of the crude extracts described above, were subjected to the immunoblot procedure described in example 20. Four of the five AUSRIA positive transformants showed a S-related protein of about 39K. One transformant (extract a) showed a S related protein of 23K. Retained as pRIT12845 was the yeast tranformant expressing the 39K protein present in extract (e). See, Figure 18.

Example 32. The preS1-preS2-S protein is assembled into particles carrying a receptor for polymerized human serum albumin and a preS1 epitope on their surface.

Crude cell extracts from yeast strain 10S44C cir° harboring either pRIT12845, pRIT12660 or pRIT12377 and yeast strain DC5 cir° harboring pRIT12363 were prepared as described in Example 19. Total protein concentration, HBsAg related antigenicity and detection of a receptor for polymerized human serum albumin were performed as described in Example 19. The result showed a strong binding activity for polymerised human serum albumin present in extracts from strain 10S44C cir° containing pRIT12843 and pRIT12660 (see also Example 10) which is absent in extracts from strain 10S44C cir° harboring pRIT12377 or strain DC5 cir° harboring pRIT12363.

CsCl equilibrium centrifugation of crude cell extracts from 10S44C cir° cells containing pRIT12845 (described in Example 12) and measurement of HBsAg related antigenicity by AUSRIA assay in the CsCl fractions, showed the antigen to band around rho = 1.2 g/cm³. This was confirmed by immunoblot analysis of the CsCl fractions. Pre S1 antibody binding activity in the CsCl fractions was tested by the ELISA assay described in Example 12. After allowing the antigen to bind to the solid-phase anti-HBsAg, the bound antigen was incubated with monoclonal antibody MA18/7 (Example 31) which was then detected by biotinylated anti-mouse Ig, streptavidin-biotinylated horseradish peroxidase com- plex and chromogen as described in Example 12.

The pre S1 specific antibody binding activity was found to coequilibrate with the HBsAg related antigenicity in the CsCl gradient. These results show that the pre S1-pre S2-S protein produced in 10S44C cir° cells harboring pRIT 12845 is assembled into particles like the 22 nm HBsAg particles derived from serum. The particles expose pre S1 specific sequences as well as a receptor for polymerised human serum albumin on their surface. From the level of activity of those particles in the AUSRIA test and the intensity of the reaction of the pre S1-pre S2-S protein in an immunoblot analysis, it can be concluded that the particle's S determinants are partially obstructed or deformed by the presence of pre S sequences in the particle.

Example 33. Two proteins of molecular weight of about 38 and 45 kilodaltons (kD), both carrying preS1, preS2 and S epitopes are expressed in yeast transformed with RIT12845

HBsAg related protein monomers expressed in pRIT12845 containing cells were analysed as described in Example 20.

Migration and immune reactivity of the cellular proteins were compared to the proteins from HBsAg particles (serotype ad) purified from human serum (obtained from Dr. W.H. Gerlich, Department of Medical Microbiology, University of Göttingen, Federal Republic of Germany).

Three immunoreagents were used to analyse the proteins:
- the monoclonal antibody (obtained from H. Thomas -Example 20) recognizing a S epitope
- a polyvalent rabbit antiserum raised against the synthetic pre S2 peptide (described in Example 23)
- monoclonal antibody MA18/7 specific for a pre S1 epitope as described by Heermann et al. (J. Virol., 52, 396-402, 1984).

The immunoblot showed, among the cellular proteins derived from pRIT12845 containing 10S44C cells, the presence of two proteins reacting specifically with all three immunoreagents described above. The molecular weight of these two proteins was estimated to be about 38,000 and 45,000 daltons. The molecular weight estimation was based on the migration of the proteins from serum derived HBsAg as defined by Heerman et al. (J. Virol., 52, 396-402, 1984). The 38,000 dalton pre S1-pre S2-S protein directed by pRIT12845 migrates slightly faster than the p39 pre S1-pre S2-S protein derived from HBsAg particles. The p39 protein from human serum particles of serotype ad presumably has a pre S1 region of 119 amino acids (Heermann et al., J. Virol., 52, 396-402, 1984). pRIT12845 codes for a pre S1 region of 108 amino acids.

The above results show that yeast cells harboring pRIT12845 express two proteins of about 38 and 45 kD carrying pre S1, pre S2 as well as S epitopes.

Example 34. The 45 Kd preS1-preS2-S protein species carries a N-glycosidically bound high-mannose type oligosaccharide chain

Cells harboring pRIT12845 were disrupted with an equal weight of glass beads (diameter 0.45-0.50 mm) in an equal weight of 10 mM sodium phosphate buffer (pH 7.4) containing 2% SDS and 8 mM EDTA by agitation with a Vortex mixer.

After centrifugation, the supernatant was heated for 4 minutes at 100°.

10 µl of the heated supernatant fluid was diluted with 40 µl 25 mM sodium citrate buffer (pH 5.0), and 2 µl EndoH (74 µg/ml) (see, Example 27) was added. Samples with and without added EndoH were incu- bated for 2.5 hrs at 37°.

The EndoH treated and untreated samples were analysed by SDS-polyacrylamide gel electrophoresis and immunoblotting as described in Example 20.

The blot was analysed with monoclonal antibody 6 (S specific) and MA18/7 (pre S1 specific) (see, Example 33).

The immunoblot showed both with monoclonal 6 and monoclonal MA18/7, the disappearance of the 45 kD band and the appearance of a 41 kD band upon Endo H treatment. The migration of the 38 kD band was not affected by the Endo H treatment.

These results show that the 45 kD pre S1-pre S2-S protein species carries a N-glycosidically bound high-mannose type oligosaccharide chain.

Example 35. The 38kD preS1-preS2-S protein contains covalently bound myristic acid present in an amide linkage

Cells from yeast strain 10S44C cir° harboring either pRIT12845, pRIT12660 or pRIT12377 were grown in YNB to an $OD_{620nm}$ of 0.4.

Cells (20 ml culture) were labeled for 60 min with 1 mCi of 3H-labeled myristic acid (New England Nuclear) and then collected by centrifugation. Cells were washed with cold $H_2O$, resuspended in 100 µl 5 mM tris-HCl (pH 7.4) containing 3 mM (DTT, 1% SDS and 1 mM PMSF, and broken with 100 mg glass beeds by six 30-second spurts of vigorous mixing in a Vortex, cooling on ice between each mixing. Debris was removed by centrifugation for 1 minute in a Eppendorf 5414 centrifuge (Towler and Glaser, Proc. Natl. Acad. Sci., 83, 2812-2816, 1986). 20 µl of the extract was treated for 3.5 hrs at room temperature with 7 µl of freshly prepared 4 M hydroxylamine, 20 mM glycine, pH 10.

Hydroxylamine treated and untreated samples were subjected to SDS polyacrylamide gel electrophoresis and fluorography as described in Example 26. The hydroxylamine untreated samples were also subjected to immunoblot analysis with the monoclonal antibody recognizing a S epitope, as described in Example 20.

The immunoblot showed results identical to those described in Examples 20 and 33.

The fluorograph showed a labeled band of 38kD present only in the cellular extract from cells harboring pRIT12845, which was hydroxylamine-stable, indicating that the 3H-myristate label is present in an amide linkage. No labeled bands specific for the extracts from cells harboring pRIT 12660 were detected.

These results show that the 38kD pre S1-S2-S protein contains covalently bound myristic acid present in an amide linkage. In general, myristate becomes covalently bound to proteins via amide linkage to an amino-terminal glycine. This suggests that the initiator Met has been removed from the pre S1-pre S2-S protein and that the Gly residue at position 2 has become available for acylation with myristic acid.

Example 36. Use of preS1 vectors for insertion of functional DNA coding sequences

The starting material is DNA of plasmid pRIT 12793 described in Example 31 above. pRIT12793 contains the pre S1-pre S2 coding sequence on a 495 bp Ncol-Xbal fragment. DNA of plasmid pRIT12793 is digested with Ncol restriction endonuclease, treated with T4 DNA polymerase to fill in the sticky ends and digested with Xbal endonuclease. The Ncol/T4 DNA polymerase/Xbal fragment is purified by polyacrylamide gel electrophoresis and electroelution and inserted on vector pUC12 previously digested with Smal and Xbal endonucleases. The cloning vector pUC12 is commercially available from Amersham (Little Chalfont, Bucks, United Kingdom) and from Pharmacia (Piscataway, NJ). The plasmid pRITX is obtained as shown in Figure 5B. In the pre S1-pre S2 region of plasmid pRITX, a single Ncol site overlaps the ATG start codon of the pre S1 region, and a Xbal site is present adjacent to and downstream of the TAA stop codon at the C-terminus of the pre S2 coding sequences. A single BstX restriction site is present on the plasmid close to the N-terminal end of the pre S1 coding region.

It will be appreciated by one skilled in the art that the vector pRITX or similar derivative vectors containing the pre S1-S2 sequences may be used for introduction of further functional DNA coding sequences encoding peptides of interest to create in phase fusions within the pre S1 region by insertion at the BstX site.

Furthermore, functional DNA coding sequences may be introduced by digestion of pRITX and derivatives with combinations of BstX and BamHI or EcoRI or PstI endonucleases and insertion of the functional DNA coding sequence between these sites, thus removing the greater part of the pre S1 coding region and the N-terminal part of the pre S2 sequence. Such fusions will be of the type pre S1-introduced functional DNA coding sequence pre S2. Once such fusions are made, they can be combined onto other plasmids containing the rest of the pre S1-pre S2 sequences and sequences necessary for maintenance and replication in E. coli and S. cerevisiae as exemplified in Example 16A.

Example 37. Fusion of peptide from proteins of HTLV-III virus to the preS2 region of preS2-S sequences to create novel hybrid particles

The preS2 region of a HBV preS2-S coding sequence can also be used for the insertion or fusion of peptide sequences of other viral antigens including those of the causative agent of human AIDS syndrome, the retrovirus known as HIV, HTLV-III or LAV.

The cloned genome of a HIV virus has been described by Ratner L. et al. (Nature, 313, 277-284, 1985) and Shaw G. et al. (Science, 226, 1165-1171, 1984). From this genomic clone various subfragments can be obtained as functional DNA coding sequences which correspond to peptide regions of interest for fusion to the preS2-S sequence to form novel hybrid particles containing epitopes of the HIV virus proteins. Such hybrid particles can serve as the basis of a human vaccine for protection against the infective agent HIV.

Among the peptide regions of interest are the following:

a. C7 peptide.

This region corresponds to a stretch of 45 amino acid residues immediately adjacent to the processing site of the envelope precursor and was defined by Starcich B.R. et al. (Cell, 45, 637-648, 1986).

b. Peptide 121.

This region corresponds to a relatively conserved amino acid sequence present in several retroviral transmembrane envelope proteins (see Cianciolo G.J. et al. (Science, 230, 453-455, 1985). This sequence is thought to participate in the pathogenesis of retroviral-induced immunosuppression (Snyder man R. and Cianciolo G.J., Immunol. Today, 5, 240, 1984).

c. "Dressman peptide".

A synthetic peptide corresponding to amino acid sequence 735 to 752 of the precursor envelope glycoprotein of HIV was used by Kennedy R.C. et al. (Science, 231, 1556-1559, 1986) to immunize rabbits and rabbit antiserum was used to investigate the recognition of HIV envelope proteins by induced antibodies. The results suggest that this region could induce an immune response directed against the native glycoprotein.

A. Fusion of the DNA sequence corresponding to the C7 peptide from a HTLV-III isolate to the preS2-S region on pRIT10911.

The starting material was plasmid pBH10-R2 containing the cloned genome of HIV isolate BH10. This plasmid was obtained from R.C. Gallo, National Institutes of Health, Bethesda, MD. A 3108 bp Sall-Xhol fragment of DNA of viral origin which was excised from pBH10-R2 and cloned between the Sall and Xhol sites of pUC18 to create plasmid pRIT12901. A restriction map of this Sall-Xhol fragment is shown in Figure IB. Plasmid pUCl8 is described by Norrander J. et al. (Gene, 26, 101-106, 1983) and is commercially available from Pharmacia Inc., Piscataway N.J.

To create the fusion, DNA of pRIT12901 was digested with Bglll and Mboll endonucleases, and the 117 bp fragment isolated by acrylamide gel electrophoresis and electroelution (see, Figure IB). DNA of this fragment was treated with Mung Bean nuclease to remove single stranded extensions and ligated with DNA of pRIT10911 (see, Example 5, above) which had been digested with BamHI endonuclease and treated with Mung Bean nuclease. From this ligation mixture a plasmid, pRIT12893, was isolated in which the 117 bp Bglll-Mboll fragment from pRIT12901 has been inserted on pRIT10911. The nucleotide sequence across the fusion junction between the TDH3 promoter and the 117 bp HTLV-III Bglll-Mboll, mung bean nuclease treated fragment insert on pRIT12893 was determined and it was found that 10 nucleotides in excess had been removed from the Bglll end of the fragment. The sequence found was

5'    ATGGAGGAGGAGATATGAGGGA    3'

where the first ATG codon underscored is that of the TDH3 promoter region from pRIT10911 and the second is an ATG codon internal to the C7 peptide fragment. The second ATG condon overlaps a TGA termination codon. This termination codon is in the same reading frame as the first ATG codon. DNA sequence determination of the 3' end of the fusion region on pRIT12893 showed the correct sequence for in phase fusion of the Mboll-mung bean nuclease treated end of the HTLV-III fragment to the preS2 sequence on pRIT10911 as shown in Figure 2B.

DNA of pRIT12893 was then digested with Hindlll endonuclease and the 3250 bp fragment isolated and purified by agarose gel electrophoresis and electroelution. The 3250 bp Hindlll fragment was then inserted at the Hindlll site of plasmid YEp13 to create pRIT12894. DNA of the plasmid was then used to transform cells of yeast strains DC5 and 10S44C to leucine independance by the method of Ito et al. as cited in Example 10 above.

Transcription and translation in yeast cells of the C7-preS2-S fusion DNA on pRIT12894 will result in synthesis of a small 5 residue peptide commencing at the TDH3 ATG codon and terminating at the TGA codon underscored above and of the C7-preS2-S fusion peptide commencing at the second ATG codon internal to the C7 sequence. This fusion contains 38 amino acid residues from the C7 peptide in place of the 45 residues from the intact Bglll-Mboll-mung bean nuclease treated C7 fragment. It will be appreciated that other plasmids recovered from the same ligation mixture as pRIT12893 can be examined and sequenced to determine if they carry a fusion corresponding to the complete fragment.

B. Fusion of the DNA sequence corresponding to the Peptide 121 to the preS2-S region on pRIT10911.

To create the fusion, DNA of pRIT12901 (described in Part A, above) was digested with Hhal and Hindlll endonucleases to liberate the 230 bp fragment shown in Figure 1B. This fragment was purified by acrylamide gel electrophoresis and electroelution and treated with T4 DNA polymerase to fill in single strand extensions. The so treated fragment was ligated with DNA of pRIT10911 which had been digested with BamHI and Mung Bean nuclease. From this ligation mixture, a plasmid was identified as pRIT12897 on which the 230 bp fragment of HIV DNA has been inserted in the correct reading frame of fusion to the preS2 sequence of pRI10911 as

**0 278 940**

shown in Figure 3B. DNA of plasmid pRIT12897 was then digested with HindIII endonuclease, and the 3365 bp fragment containing the TDH3 promoter, HIV-preS2-S fusion and the ARG3 terminator was purified by agarose gel electrophoresis and electroelution. This 3365 bp fragment was ligated onto YEp13 digested with HindIII endonuclease to form plasmid pRIT12898. DNA of this plasmid (pRIT 12898) was then used to transform cells of yeast strains DC5 and 10S44C to leucine independence by the method of Ito et al. as cited in Example 10, above.

C. Fusion of the DNA sequence corresponding to the "Dreesman"peptide to the preS2 region on pRIT10911.

A 60 bp fragment of synthetic DNA with the sequence shown below was synthesized by conventional means as two single strands which were then annealed together:

5' GATCTCGACAGGCCCGAAGGAATAGAAGAAGAAGGTGGAGAGAGA 3'

3'      AGCTGTCCGGGCTTCCTTATCTTCTTCTTCCACCTCTCTCT 5'


5' GACAGAGACAGATCCCCG 3'

3' CTGTCTCTGTCTAGGGGCCTAC 5'


This fragment has 5' BglII and 3' BamHI single strand extensions. About 200 ng of a double stranded fragment and 300 ng of DNA of plasmid pRIT10911 digested with BamHI were mixed and ligated. From this ligation mixture, a plasmid, pRIT12899, was identified and recovered on which the 60 bp fragment has been inserted at the BamHI site of pRIT10911 so as to create an inframe fusion as shown in Figure 4B. DNA of pRIT12899 was digested with HindIII endonuclease, and the 3200 bp HindIII fragment was purified by agarose gel electrophoresis and electroelution. This HindIII fragment was inserted at the HindIII site of YEp13 to create plasmid pRIT12900. DNA of plasmid pRIT12900 was then used to transform cells of yeast strains DC5 and 10S44C to leucine independence by the method of Ito et al. cited in Example 10, above.

Example 38. Expression of fusion proteins from pRIR12894 and pRIT12898

Yeast strains DC5 or 10S44C harboring either YEp13, pRIT12363, pRIT10912, pRIT12894 or pRIT12898 were separately grown in liquid medium lacking leucine (YNB + 80 µg/ml histidine or YNB), and cellular proteins were analyzed by the immunoblot procedure as described in Example 11 using as the first antibody a monoclonal antibody directed against a denaturation and reduction resistant epitope of human derived HBsAg (monoclonal 6 obtained from H. Thomas, Royal Free Hospital, London, England). pRIT10912 is described in Example 5 above. pRIT12894 and pRIT12898 are described in Example 37, above. pRIT12363 carries the S gene of HBV fused to the TDH3 promoter as the 2900 bp HindIII fragment from pRIT12322 (Example 8) inserted on a yeast vector plasmid identical to pRIT12377 (Example 10, part B above) and produces HBsAg under the control of the TDH3 promoter.

The immunoblot showed a protein band related to S protein of estimated molecular weight of about 32 kD among the total cellular proteins from yeast strains DC5 or 10S44C transformed with pRIT12894. A protein band related to S protein of estimated molecular weight of about 45 kD was present among the total cellular proteins from yeast strains DC5 or 10S44C harboring pRIT12898, while a protein band related to S protein of estimated molecular weight of about 29 kD was present among the total cellular proteins from yeast strain DC5 harboring pRIT10912.

Crude cell extracts from yeast strain DC5 harboring either pRIT12363, pRIT10912, or pRIT12894 were prepared as described in Example 12. Protein concentration and AUSRIA activity were measured as described in Example 12. Immunoblot analysis of these extracts was performed as described above.

CsCl equilibrium centrifugation of the crude extracts, (described in Example 12) and measurement of HBsAg related antigenicity by AUSRIA assay in the CsCl fractions, showed the antigen to band around rho = 1.2 g/cm$^3$. This was confirmed by immunoblot analysis of the CsCl fractions.

These results show that the 32 kD fusion protein produced in yeast strain DC5 cells carrying pRIT12894 is assembled into particles like the 22 nm HBsAg particles derived from human serum. From the level of activity of those particles in the AUSRIA test, and the intensity of the reaction of the 32 kD protein in the immunoblot analysis, it can be concluded that the particles' S determinants are partially obstructed or deformed by the presence of C7 sequences.

From these results it is concluded that yeast cells of strains DC5 and 10S44C transformed with either pRIT12894 or pRIT12898 specifically synthesize a fusion protein of molecular weight of about 32KD or 45 kD respectively, both carrying a S epitope.

It will be appreciated by one of skill in the art that hybrid particles carrying the HIV virus peptide epitopes can be extracted and purified by conventional means from cultures of yeast cells transformed with pRIT12894, pRIT12898 and pRIT12900. Such purified particles may then be formulated in the presence of adjuvants such

50

as Al(OH)₃ or AlPO₄ as vaccines for human use. The preferred dose will be in the range of 1-1000 µg protein and is determined by conventional trials. It will also be understood that the invention as exemplified herein includes other peptide regions from HIV viral proteins which can be fused to the preS2-S region of HBV coding sequences to form hybrid particles presenting the HIV epitope of interest.

## Claims

1. A recombinant DNA molecule comprising a functional DNA coding sequence fused, in phase, to a portion of the Pre S2 region of a HBV Pre S2-S protein coding sequence wherein the functional DNA coding sequence comprises the PreS2 coding sequence, PreS1 coding sequence or entire PreS1-PreS2 coding sequence, the circumsporozoite protein coding sequence of Plasmodium, or a HIV coding sequence such as a HIV envelope gene sequence such as the HIV C7 protein coding region, the HIV Peptide 121 coding region or the HIV Dreesman peptide coding region.

2. A recombinant vector which comprises a recombinant DNA molecule operatively linked to a regulatory region, wherein the DNA molecule contains a functional DNA coding sequence fused, in phase, to a portion of the Pre S2 region of a HBV Pre S2-S protein coding sequence wherein the regulatory region comprises the TDH3 promoter.

3. A recombinant vector which comprises a recombinant DNA molecule operatively linked to a regulatory region, wherein the DNA molecule contains a functional DNA coding sequence fused, in phase, to a portion of the pre S2 region of a HBV Pre S2-S protein coding sequence wherein the functional DNA coding sequence comprises the PreS2 coding sequence, PreS1 coding sequence or entire PreS1-PreS2 coding sequence, circumsporozoite (CS) protein coding sequence of Plasmodium, or a HIV coding sequence such as a HIV envelope gene sequence such as the HIV C7 protein, the HIV Peptide 121 protein or the HIV Dreesman peptide.

4. The vector of Claim 3 which comprises a 192 bp Sau3A fragment coding for 16 tetrapeptide repeats of the CS protein of P.falciparum wherein such fragment is one which could be derived from Sau3A digestion of a 1215 bp StuI - RsaI fragment of plasmid WR201 containing the P. falciparum CS protein coding sequence minus the first 52 bp of such sequence.

5. The vector of Claim 4 which comprises additional Sau3A fragments of CS protein DNA.

6. A recombinant vector which comprises a recombinant DNA molecule operatively linked to a regulatory region, wherein the DNA molecule contains a functional DNA coding sequence fused, in phase, to a portion of the Pre S2 region of a HBV Pre S2-S protein coding sequence wherein the functional DNA coding sequence comprises all or part of the Pre S2 protein coding sequence.

7. The vector of Claim 6 wherein the functional DNA coding sequence comprises the following amino acid coding sequence:

## PRE-S2 REGION

-55

```
ATG CAG TGG AAT TCC ACT GCC TTC CAC CAA
Met Gln Trp Asn Ser Thr Ala Phe His Gln


GCT CTG CAG GAT CCC AGA GTC AGG GGT CTG
Ala Leu Gln Asp Pro Arg Val Arg Gly Leu


TAT TTT CCT GCT GGT GGC TCC AGT TCA GGA
Tyr Phe Pro Ala Gly Gly Ser Ser Ser Gly ⌐
```

```
ACA GTA AAC CCT GCT CCG AAT ATT GCC TCT
Thr Val Asn Pro Ala Pro Asn Ile Ala Ser


CAC ATA TCG TCA AGC TCC GCG AGG ACT GGG
His Ile Ser Ser Ser Ser Ala Arg Thr Gly


GAC CCT GTG ACG AAC
Asp Pro Val Thr Asn.
```

8. The vector of Claim 6 wherein the functional DNA coding sequence comprises all or part of the Pre S1-Pre S2 protein coding sequence.

9. The vector of Claim 8 wherein the functional DNA coding sequence comprises the following amino acid coding sequence:

PRE-S1 REGION

-163

```
ATG GGG ACG AAT CTT TCT GTT CCC AAC CCT CTG
Met Gly Thr Asn Leu Ser Val Pro Asn Pro Leu


GGA TTC TTT CCC GAT CAT CAG TTG GAC CCT
Gly Phe Phe Pro Asp His Gln Leu Asp Pro


GCA TTC GGA GCC AAC TCA AAC AAT CCA GAT
Ala Phe Gly Ala Asn Ser Asn Asn Pro Asp


TGG GAC TTC AAC CCC ATC AAG GAC CAC TGG
Trp Asp Phe Asn Pro Ile Lys Asp His Trp


CCA GCA GCC AAC CAG GTA GGA GTG GGA GCA
Pro Ala Ala Asn Gln Val Gly Val Gly Ala
```

```
TTC GGG CCA GGG CTC ACC CCT CCA CAC GGC
Phe Gly Pro Gly Leu Thr Pro Pro His Gly


GGT ATT TTG GGG TGG AGC CCT CAG GCT CAG
Gly Ile Leu Gly Trp Ser Pro Gln Ala Gln


GGC ATA TTG ACC ACA GTG TCA ACA ATT CCT
Gly Ile Leu Thr Thr Val Ser Thr Ile Pro


CCT CCT GCC TCC ACC AAT CGG CAG TCA GGA
Pro Pro Ala Ser Thr Asn Arg Gln Ser Gly


AGG CAG CCT ACT CCC ATC TCT CCA CCT CTA
Arg Gln Pro Thr Pro Ile Ser Pro Pro Leu


AGA GAC AGT CAT CCT CAG GCC
Arg Asp Ser His Pro Gln Ala
```

## PRE-S2 REGION

```
-55
ATG CAG TGG AAT TCC ACT GCC TTC CAC CAA
Met Gln Trp Asn Ser Thr Ala Phe His Gln


GCT CTG CAG GAT CCC AGA GTC AGG GGT CTG
Ala Leu Gln Asp Pro Arg Val Arg Gly Leu


TAT TTT CCT GCT GGT GGC TCC AGT TCA GGA
Tyr Phe Pro Ala Gly Gly Ser Ser Ser Gly


ACA GTA AAC CCT GCT CCG AAT ATT GCC TCT
Thr Val Asn Pro Ala Pro Asn Ile Ala Ser


CAC ATA TCG TCA AGC TCC GCG AGG ACT GGG
His Ile Ser Ser Ser Ser Ala Arg Thr Gly
```

```
GAC CCT GTG ACG AAC
Asp Pro Val Thr Asn.
```

10. A eukaryotic host cell transformed with a recombinant vector, wherein said vector is according to Claim 5.

11. The host of Claim 10 which is a yeast cell.

12. A method of preparing a hybrid particle containing HBsAg protein which contains and/or presents a peptide encoded by a functional DNA coding sequence which comprises culturing a eukaryotic host cell transformed with a vector in appropriate culture media and isolating the particle from a cell lysate or extract of such host's culture, wherein said vector is according to Claim 5.

13. A particle prepared by the method of Claim 12.

14. A hybrid immunogenic particle comprising HBsAg protein wherein said particle contains and/or presents a peptide encoded by a functional DNA coding sequence.

15. A vaccine comprising an immunoprotective amount of a particle according to Claim 14.

16. A micelle comprising the particle according to Claim 14 and polysorbate.

17. A vaccine comprising an immunoprotective amount of a micelle according to Claim 16.

18. A HBV Pre S1 protein coding region which codes for the following amino sequence:

−163

```
ATG GGG ACG AAT CTT TCT GTT CCC AAC CCT CTG
Met Gly Thr Asn Leu Ser Val Pro Asn Pro Leu

GGA TTC TTT CCC GAT CAT CAG TTG GAC CCT
Gly Phe Phe Pro Asp His Gln Leu Asp Pro

GCA TTC GGA GCC AAC TCA AAC AAT CCA GAT
Ala Phe Gly Ala Asn Ser Asn Asn Pro Asp

TGG GAC TTC AAC CCC ATC AAG GAC CAC TGG
Trp Asp Phe Asn Pro Ile Lys Asp His Trp

CCA GCA GCC AAC CAG GTA GGA GTG GGA GCA
Pro Ala Ala Asn Gln Val Gly Val Gly Ala

TTC GGG CCA GGG CTC ACC CCT CCA CAC GGC
Phe Gly Pro Gly Leu Thr Pro Pro His Gly

GGT ATT TTG GGG TGG AGC CCT CAG GCT CAG
Gly Ile Leu Gly Trp Ser Pro Gln Ala Gln

GGC ATA TTG ACC ACA GTG TCA ACA ATT CCT
Gly Ile Leu Thr Thr Val Ser Thr Ile Pro

CCT CCT GCC TCC ACC AAT CGG CAG TCA GGA
Pro Pro Ala Ser Thr Asn Arg Gln Ser Gly

AGG CAG CCT ACT CCC ATC TCT CCA CCT CTA
Arg Gln Pro Thr Pro Ile Ser Pro Pro Leu

AGA GAC AGT CAT CCT CAG GCC
Arg Asp Ser His Pro Gln Ala
```

19. A recombinant DNA vector comprising the Pre S1 protein coding region of Claim 18 operatively linked to a regulatory region.

20. A transformed host cell comprising the Pre S1 protein coding region of Claim 18.

21. Protein encoded by the Pre S1 protein coding region of Claim 18.

22. A vaccine comprising an immunoprotective amount of the protein encoded by the Pre S1 protein coding region of Claim 18.

23. A HBV Pre S2 protein coding region or a HBV Pre S2-S protein coding region wherein the Pre S2 portion thereof codes for the following amino acid sequence:

                                    Thr

```
-55                    -50                      or
Met-Gln-Trp-Asn-Ser-Thr-Ala-Phe-His-Gln-Ala-Leu-Gln-Asp-


   -40                                         -30
Pro-Arg-Val-Arg-Gly-Leu-Tyr-Phe-Pro-Ala-Gly-Gly-Ser-Ser-
Ser-
                          -20
Gly-Thr-Val-Asn-Pro-Ala-Pro-Asn-Ile-Ala-Ser-His-Ile-Ser-


      -10
Ser-Ser-Ser-Ala-Arg-Thr-Gly-Asp-Pro-Val-Thr-Asn
```

24. A recombinant DNA vector comprising the Pre S2 or the Pre S2-S protein coding region of Claim 23 operatively linked to a regulatory region.

25. A transformed host cell comprising the Pre S2 of the Pre S2-S protein coding region of Claim 23.

26. Protein encoded by the Pre S2 or the Pre S2-S protein coding region of Claim 23.

27. A vaccine comprising an immunoprotective amount of protein encoded by the Pre S2 or the Pre S2-S protein coding region of Claim 23.

28. A particle comprising protein encoded by the Pre S2-S protein coding region of Claim 23.

29. A vaccine comprising an immunoprotective amount of a particle encoded by the Pre S2-S protein coding region of Claim 23.

30. A micelle containing the particle of Claim 28 and polysorbate.

31. A vaccine comprising an immunoprotective amount of the micelle of Claim 30.

32. A recombinant DNA vector comprising a Pre S1-Pre S2-S protein coding sequence operatively linked to a regulatory region wherein the Pre S1 - Pre S2 portion of such sequences codes for the following amino acid coding sequence:

PRE-S1 REGION

-163
```
ATG GGG ACG AAT CTT TCT GTT CCC AAC CCT CTG
Met Gly Thr Asn Leu Ser Val Pro Asn Pro Leu
```

```
GGA TTC TTT CCC GAT CAT CAG TTG GAC CCT
Gly Phe Phe Pro Asp His Gln Leu Asp Pro


GCA TTC GGA GCC AAC TCA AAC AAT CCA GAT
Ala Phe Gly Ala Asn Ser Asn Asn Pro Asp


TGG GAC TTC AAC CCC ATC AAG GAC CAC TGG
Trp Asp Phe Asn Pro Ile Lys Asp His Trp


CCA GCA GCC AAC CAG GTA GGA GTG GGA GCA
Pro Ala Ala Asn Gln Val Gly Val Gly Ala


TTC GGG CCA GGG CTC ACC CCT CCA CAC GGC
Phe Gly Pro Gly Leu Thr Pro Pro His Gly


GGT ATT TTG GGG TGG AGC CCT CAG GCT CAG
Gly Ile Leu Gly Trp Ser Pro Gln Ala Gln


GGC ATA TTG ACC ACA GTG TCA ACA ATT CCT
Gly Ile Leu Thr Thr Val Ser Thr Ile Pro


CCT CCT GCC TCC ACC AAT CGG CAG TCA GGA
Pro Pro Ala Ser Thr Asn Arg Gln Ser Gly


AGG CAG CCT ACT CCC ATC TCT CCA CCT CTA
Arg Gln Pro Thr Pro Ile Ser Pro Pro Leu


AGA GAC AGT CAT CCT CAG GCC
Arg Asp Ser His Pro Gln Ala
```

PRE-S2 REGION

-55

```
ATG CAG TGG AAT TCC ACT GCC TTC CAC CAA
Met Gln Trp Asn Ser Thr Ala Phe His Gln
```

```
GCT CTG CAG GAT CCC AGA GTC AGG GGT CTG
Ala Leu Gln Asp Pro Arg Val Arg Gly Leu


TAT TTT CCT GCT GGT GGC TCC AGT TCA GGA
Tyr Phe Pro Ala Gly Gly Ser Ser Ser Gly


ACA GTA AAC CCT GCT CCG AAT ATT GCC TCT
Thr Val Asn Pro Ala Pro Asn Ile Ala Ser


CAC ATA TCG TCA AGC TCC GCG AGG ACT GGG
His Ile Ser Ser Ser Ser Ala Arg Thr Gly


GAC CCT GTG ACG AAC
Asp Pro Val Thr Asn
```

33. A yeast host cell transformed with the vector of Claim 32.

34. Protein prepared by the PreS1-PreS2-S sequence of the vector of Claim 32.

35. A vaccine comprising an immunoprotective amount of the protein of Claim 34.

36. A particle comprising protein encoded by a PreS1-PreS2-S protein coding region.

37. A vaccine comprising an immunoprotective amount of the particle of Claim 36.

38. A micelle comprising the particle of Claim 36 and polysorbate.

39. A vaccine comprising an immunoprotective amount of the micelle of Claim 38.

40. A Pre S1-Pre S2 protein coding sequence which comprises the following amino acid sequence:

## Pre S1 Region

-163

| ATG | GGG | ACG | AAT | CTT | TCT | GTT | CCC | AAC | CCT | CTG |
|-----|-----|-----|-----|-----|-----|-----|-----|-----|-----|-----|
| Met | Gly | Thr | Asn | Leu | Ser | Val | Pro | Asn | Pro | Leu |

| GGA | TTC | TTT | CCC | GAT | CAT | CAG | TTG | GAC | CCT |
|-----|-----|-----|-----|-----|-----|-----|-----|-----|-----|
| Gly | Phe | Phe | Pro | Asp | His | Gln | Leu | Asp | Pro |

| GCA | TTC | GGA | GCC | AAC | TCA | AAC | AAT | CCA | GAT |
|-----|-----|-----|-----|-----|-----|-----|-----|-----|-----|
| Ala | Phe | Gly | Ala | Asn | Ser | Asn | Asn | Pro | Asp |

| TGG | GAC | TTC | AAC | CCC | ATC | AAG | GAC | CAC | TGG |
|-----|-----|-----|-----|-----|-----|-----|-----|-----|-----|
| Trp | Asp | Phe | Asn | Pro | Ile | Lys | Asp | His | Trp |

| CCA | GCA | GCC | AAC | CAG | GTA | GGA | GTG | GGA | GCA |
|-----|-----|-----|-----|-----|-----|-----|-----|-----|-----|
| Pro | Ala | Ala | Asn | Gln | Val | Gly | Val | Gly | Ala |

| TTC | GGG | CCA | GGG | CTC | ACC | CCT | CCA | CAC | GGC |
|-----|-----|-----|-----|-----|-----|-----|-----|-----|-----|
| Phe | Gly | Pro | Gly | Ieu | Thr | Pro | Pro | His | Gly |

| GGT | ATT | TTG | GGG | TGG | AGC | CCT | CAG | GCT | CAG |
|-----|-----|-----|-----|-----|-----|-----|-----|-----|-----|
| Gly | Ile | Leu | Gly | Trp | Ser | Pro | Gin | Ala | Gln |

| GGC | ATA | TTG | ACC | ACA | GTG | TCA | ACA | ATT | CCT |
|-----|-----|-----|-----|-----|-----|-----|-----|-----|-----|
| Gly | Ile | Leu | Thr | Thr | Val | Ser | Thr | Ile | Pro |

| CCT | CCT | GCC | TCC | ACC | AAT | CGG | CAG | TCA | GGA |
|-----|-----|-----|-----|-----|-----|-----|-----|-----|-----|
| Pro | Pro | Ala | Ser | Thr | Asn | Arg | Gln | Ser | Gly |

| AGG | CAG | CCT | ACT | CCC | ATC | TCT | CCA | CCT | CTA |
|-----|-----|-----|-----|-----|-----|-----|-----|-----|-----|
| Arg | Gln | Pro | Thr | Pro | Ile | Ser | Pro | Pro | Leu |

```
AGA   GAC   AGT   CAT   CCT   CAG   GCC
Arg   Asp   Ser   His   Pro   Gln   Ala
```

Pre S2 Region

-55

```
ATG   CAG   TGG   AAT   TCC   ACT   GCC   TTC   CAC   CAA
Met   Gln   Trp   Asn   Ser   Thr   Ala   Phe   His   Gln
```

```
GCT   CTG   CAG   GAT   CCC   AGA   GTC   AGG   GGT   CTG
Ala   Leu   Gln   Asp   Pro   Arg   Val   Arg   Gly   Leu
```

```
TAT   TTT   CCT   GCT   GGT   GGC   TCC   AGT   TCA   GGA
Tyr   Phe   Pro   Ala   Gly   Gly   Ser   Ser   Ser   Gly
```

```
ACA   GTA   AAC   CCT   GCT   CCG   AAT   ATT   GCC   TCT
Thr   Val   Asn   Pro   Ala   Pro   Asn   Ile   Ala   Ser
```

```
CAC   ATA   TCG   TCA   AGC   TCC   GCG   AGG   ACT   GGG
His   Ile   Ser   Ser   Ser   Ser   Ala   Arg   Thr   Gly
```

```
GAC   CCT   GTG   ACG   AAC
Asp   Pro   Val   Thr   Asn
```

41. A recombinant DNA vector comprising the Pre S1-Pre S2 protein coding sequence of Claim 40 operatively linked to a regulatory region.

42. A yeast host cell transformed with the vector of Claim 41.

43. Protein encoded by the Pre S1-Pre S2 protein coding region of Claim 40.

44. A vaccine comprising an immunoprotective amount of protein encoded by the Pre S1-Pre S2 protein coding region of Claim 40.

45. A recombinant DNA vector comprising a Pre S1 functional DNA coding sequence - Pre S2-S protein coding sequence operatively linked to an expression control sequence.

46. A yeast host cell transformed with the vector of Claim 45.

47. Protein expressed by the host of Claim 46.

48. A vaccine comprising an immunoprotective amount of the protein of Claim 47.

Claims for the following contracting states : Es, Gr

1. A method of preparing a transformed eukaryotic host cell which comprises transforming a eukaryotic host cell with a vector which comprises a recombinant DNA molecule operatively linked to a regulatory region containing a functional DNA coding sequence fused, in phase, to a portion of the PreS2 region of a HBV PreS2-S protein sequence wherein the functional DNA coding sequence also encodes the circumsporozoite (CS) protein of Plasmodium, or a HIV envelope gene sequence such as the HIVC7 protein, the HIV peptide 121 protein or the HIV Dreesman peptide.

2. The method of Claim 1 wherein the host is a yeast cell.

3. The method of Claim 2 wherein the host belongs to the genus Saccharomyces.

4. The method of Claim 3 wherein the host belongs to the species Saccharomyces cerevisiae or Saccharomyces carlsbergensis.

5. The method of Claim 4 wherein the host is strain DC5, DC5 cir°, 10S44C or 10S44C or 10S44C cir°.

6. A method of preparing a hybrid particle containing HBsAg protein which contains and/or presents a peptide encoded by a functional DNA coding sequence which comprises culturing a eukaryotic host cell transformed with a vector in appropriate culture media and isolating the particle from a cell lysate or extract of such host's culture, wherein said vector is a vector which comprises a recombinant DNA molecule operatively linked to a regulatory region containing a functional DNA coding sequence fused, in phase, to a portion of the PreS2 region of a HBV Pre S2-S protein sequence wherein the functional DNA coding sequence also encodes the circumsporozoite (CS) protein of Plasmodium, or a HIV envelope gene sequence such as the HIVC7 protein, the HIV peptide 121 protein or the HIV Dreesman peptide.

7. A process of preparing a transformed host cell which comprises transforming a host cell with the HBV Pre S1 protein coding region which codes for the following amino sequence:

```
-163
ATG GGG ACG AAT CTT TCT GTT CCC AAC CCT CTG
Met Gly Thr Asn Leu Ser Val Pro Asn Pro Leu


GGA TTC TTT CCC GAT CAT CAG TTG GAC CCT
Gly Phe Phe Pro Asp His Gln Leu Asp Pro


GCA TTC GGA GCC AAC TCA AAC AAT CCA GAT
Ala Phe Gly Ala Asn Ser Asn Asn Pro Asp


TGG GAC TTC AAC CCC ATC AAG GAC CAC TGG
Trp Asp Phe Asn Pro Ile Lys Asp His Trp


CCA GCA GCC AAC CAG GTA GGA GTG GGA GCA
Pro Ala Ala Asn Gln Val Gly Val Gly Ala


TTC GGG CCA GGG CTC ACC CCT CCA CAC GGC
Phe Gly Pro Gly Leu Thr Pro Pro His Gly


GGT ATT TTG GGG TGG AGC CCT CAG GCT CAG
Gly Ile Leu Gly Trp Ser Pro Gln Ala Gln
```

```
GGC ATA TTG ACC ACA GTG TCA ACA ATT CCT
Gly Ile Leu Thr Thr Val Ser Thr Ile Pro


CCT CCT GCC TCC ACC AAT CGG CAG TCA GGA
Pro Pro Ala Ser Thr Asn Arg Gln Ser Gly


AGG CAG CCT ACT CCC ATC TCT CCA CCT CTA
Arg Gln Pro Thr Pro Ile Ser Pro Pro Leu


AGA GAC AGT CAT CCT CAG GCC
Arg Asp Ser His Pro Gln Ala
```

8. The process of Claim 7 wherein the region is comprised by a recombinant DNA vector.

9. The process of Claim 7 wherein the host is a yeast cell.

10. The process of Claim 9 wherein the host belongs to the genus Saccharomyces.

11. A process for preparing the protein encoded by the Pre S1 protein coding region of Claim 7 which comprises culturing a host cell transformed with the region in appropriate culture media and isolating the protein from a cell lysate or extract of such host's culture.

12. A process of preparing a transformed host cell which comprises transforming a host cell with a HBV PreS2 protein coding region or a HBV PreS2-S protein coding region wherein the PreS2 portion thereof codes for the following amino acid reference:

```
                                Thr
-55                   -50                      or
Met-Gln-Trp-Asn-Ser-Thr-Ala-Phe-His-Gln-Ala-Leu-Gln-Asp-


         -40                              -30
Pro-Arg-Val-Arg-Gly-Leu-Tyr-Phe-Pro-Ala-Gly-Gly-Ser-Ser-
Ser-
                          -20
Gly-Thr-Val-Asn-Pro-Ala-Pro-Asn-Ile-Ala-Ser-His-Ile-Ser-


         -10
Ser-Ser-Ser-Ala-Arg-Thr-Gly-Asp-Pro-Val-Thr-Asn
```

13. The process of Claim 12 wherein the region is comprised by a recombinant DNA vector.

14. A process for preparing protein encoded by the Pre S2 or the Pre S2-S protein coding region of Claim 12 which comprises culturing a host cell transformed with the region in appropriate culture media and isolating the protein from a cell lysate or extract of such host's culture.

15. A process for preparing a particle encoded by the Pre S2-S protein coding region of Claim 12 which comprises culturing a host cell transformed with the region in appropriate culture media and isolating the protein from a cell lysate or extract of such host's culture.

16. A method of preparing a transformed yeast host cell which comprises transforming a yeast host cell with a recombinant DNA vector comprising a PreS1-PreS2-S protein coding sequence operatively linked to a regulatory region.

17. A method of preparing protein encoded by the Pre S1-Pre S2-S protein coding sequence which comprises culturing a yeast host cell transformed with recombinant DNA vector comprising a PreS1-PreS2-S protein coding sequence operatively linked to a regulatory region in appropriate culture media and isolating the protein from a cell lysate or extract of such host's culture.

18. A method of preparing a particle containing polypeptide encoded by the Pre S1-Pre S2-S protein coding sequence which comprises culturing a yeast host cell transformed with a recombinant DNA vector comprising a PreS1-PreS2-S protein coding sequence operatively linked to a regulatory region in appropriate culture media and isolating the particle from a cell lysate or extract of such host's culture.

19. A method of preparing a transformed yeast host cell which comprises transforming a yeast host cell with a recombinant DNA vector comprising the following PreS1-S2-protein coding sequence operatively linked to a regulatory region.

## Pre S1 Region

-163

| ATG | GGG | ACG | AAT | CTT | TCT | GTT | CCC | AAC | CCT | CTG |
|-----|-----|-----|-----|-----|-----|-----|-----|-----|-----|-----|
| Met | Gly | Thr | Asn | Leu | Ser | Val | Pro | Asn | Pro | Leu |

| GGA | TTC | TTT | CCC | GAT | CAT | CAG | TTG | GAC | CCT |
|-----|-----|-----|-----|-----|-----|-----|-----|-----|-----|
| Gly | Phe | Phe | Pro | Asp | His | Gln | Leu | Asp | Pro |

| GCA | TTC | GGA | GCC | AAC | TCA | AAC | AAT | CCA | GAT |
|-----|-----|-----|-----|-----|-----|-----|-----|-----|-----|
| Ala | Phe | Gly | Ala | Asn | Ser | Asn | Asn | Pro | Asp |

```
TGG   GAC   TTC   AAC   CCC   ATC   AAG   GAC   CAC   TGG
Trp   Asp   Phe   Asn   Pro   Ile   Lys   Asp   His   Trp


CCA   GCA   GCC   AAC   CAG   GTA   GGA   GTG   GGA   GCA
Pro   Ala   Ala   Asn   Gln   Val   Gly   Val   Gly   Ala


TTC   GGG   CCA   GGG   CTC   ACC   CCT   CCA   CAC   GGC
Phe   Gly   Pro   Gly   Ieu   Thr   Pro   Pro   His   Gly


GGT   ATT   TTG   GGG   TGG   AGC   CCT   CAG   GCT   CAG
Gly   Ile   Leu   Gly   Trp   Ser   Pro   Gln   Ala   Gln


GGC   ATA   TTG   ACC   ACA   GTG   TCA   ACA   ATT   CCT
Gly   Ile   Leu   Thr   Thr   Val   Ser   Thr   Ile   Pro


CCT   CCT   GCC   TCC   ACC   AAT   CGG   CAG   TCA   GGA
Pro   Pro   Ala   Ser   Thr   Asn   Arg   Gln   Ser   Gly


AGG   CAG   CCT   ACT   CCC   ATC   TCT   CCA   CCT   CTA
Arg   Gln   Pro   Thr   Pro   Ile   Ser   Pro   Pro   Leu


AGA   GAC   AGT   CAT   CCT   CAG   GCC
Arg   Asp   Ser   His   Pro   Gln   Ala
```

Pre S2 Region
-55

```
ATG   CAG   TGG   AAT   TCC   ACT   GCC   TTC   CAC   CAA
Met   Gln   Trp   Asn   Ser   Thr   Ala   Phe   His   Gln


GCT   CTG   CAG   GAT   CCC   AGA   GTC   AGG   GGT   CTG
Ala   Leu   Gln   Asp   Pro   Arg   Val   Arg   Gly   Leu


TAT   TTT   CCT   GCT   GGT   GGC   TCC   AGT   TCA   GGA
Tyr   Phe   Pro   Ala   Gly   Gly   Ser   Ser   Ser   Gly
```

| ACA | GTA | AAC | CCT | GCT | CCG | AAT | ATT | GCC | TCT |
|-----|-----|-----|-----|-----|-----|-----|-----|-----|-----|
| Thr | Val | Asn | Pro | Ala | Pro | Asn | Ile | Ala | Ser |

| CAC | ATA | TCG | TCA | AGC | TCC | GCG | AGG | ACT | GGG |
|-----|-----|-----|-----|-----|-----|-----|-----|-----|-----|
| His | Ile | Ser | Ser | Ser | Ser | Ala | Arg | Thr | Gly |

| GAC | CCT | GTG | ACG | AAC |
|-----|-----|-----|-----|-----|
| Asp | Pro | Val | Thr | Asn |

20. A process for preparing protein encoded by the Pre S1-Pre S2 protein coding region of Claim 19

Figure 1

Figure 2

Figure 3

pMC200

7660bp

EcoRI
Hind III
Hinc II
Pst I
Xho I
BstE II
Hinc II
Bgl II
EcoRI
Xba I
Hind III
Hinc II
Bam HI

■ pBR322
□ arg 3

Figure 4

TCTAAAGGCA   GTTTATTCCT   TGTATGTCCT

TTAAG<u>TAC</u>AG   TTAATAACGA   GCAATTTTTT
     Rsa I

TTTTTTTTTT   TAGCCATCTA   CCCATCAACT

T<u>GTAC</u>ACTCG   TTACC|ATG|TC   AA<u>CCACAGCA</u>
  Rsa I                 Hinc III

TCCACGCCTT   CATCTTTACG   TCATTTGATT

TCTATAAA<u>AG   ATC</u>TTTCTGA   TGAA<u>GAATTC</u>
        Bgl II                 EcoRI

AGAATCTTAG   TACAAAGAGC   TCAACATTTC

Figure 5

Figure 6

0278940

Figure 7

**Restriction enzyme Symbols**

A...Acc I
B...BamH I
C...Cla I
E...Ecor I
H...Hind III
Hp...Hpa I
S...Sal I
X...Xba I
Xh...Xho I

Vector Sequences
Cloned Fragments
S gene Sequences

pRIT 10158
7660 bp

C H
Hae III
X
E
Xh
H
B

Reclone 3300 bp ARG 3 gene fragment from pMC 200 on pBR 322 deleted for EcoR I site

1150 bp Cla I - Hae III fragment with ARG 3 transcription terminator

TDH 3 promoter fragment cloned on pUC 9 BamH I site at ATG (ATGGATCC)

pRIT 10167
3750 bp

H E B

EcoRI, BAMHI

pRIT 10610
7200 bp

B X Hp B
E
E

Clone 1372 bp BamHI fragment on pBR 327

pRIT 10677
5730 bp

E C H B
Hp
X
B

Cla I, Hpa I

pRIT 10909
6100 bp

E C H
Hp, Hae III
X
B

2100 bp EcoRI - BamHI fragment

pRIT 10911
5850 bp

E C H
H
B X

Fig. 8a

0278940

0278940

pRIT 10158
EcoRI
Xba I

pRIT 10679
5200 bp

Isolate 126 bp EcoRI
Xba fragment

pRIT 10903
7350 bp

EcoRI
Bal 31
Xho I
T 4 DNA polymerase ligation
verify sequence across
Xho I site (CTCGAGAAC)

pRIT 10914
6500 bp

93 bp Xho I Xba I fragment

pRIT 12209
6700 bp

BamHI
Xho I
Mung bean nuclease
T 4 DNA polymerase
ligation

pRIT 12230
5650 bp

Xho I
Xba I

pRIT 12211
8450 bp

pRIT 10158

2700 bp BamHI Xba I
fragment

BamHI
Xba I

Fig. 8b

isolate 1225 bp
Fnu D II fragment
pRIT ← attach EcoRI linkers ←pRIT 10616
10679 clone in pACYC 184

EC
H

E

pRIT 12288

5920 bp

Xn

H

Clone 2650 bp
Hind III fragment
on pBR 327

C
H

E

pRIT 10162

7000 bp

Xn

H

Clone 1150 bp Cla I Hae III
fragment on EcoRI
T 4 DNA polymerase
Cla I treated plasmid

pRIT 10158

Fig. 8c

Acc I EcoRI synthetic
linker
ATACATTTAACG
TGTAAATTGCTTAA

1180 bp
EcoRI fragment

pRIT 12230
CAAAATGGAGAAC
correct sequence for
S gene fusion to ATG

E C
H

pRIT 12322

5600 bp

H

X

A E

0278940

0278940

Figure 9

Figure 10

1180 bp EcoRI fragment
from pRIT12288

Cla I
EcoRI
Hind III
Sac II
AccI-EcoRI
Synthetic
linker
EcoRI
AccI
pRIT 12322
5600 bp
S gene
pUC9
Xbal
Hind III
Hind III - AccI fragment
from pRIT12230

Figure 11

Stul - Rsal fragment from WR201

Figure 12a

Figure 12b

Figure 13

pRIT12309

Cla, Sal I

2830 bp ClaI - Sal I
fragment from
pRIT12290

EcoRI Cla I

BamHI
EcoRI
Sac II

pRIT12314

11790 bp

EcoRI

Sal I

EcoRI

Sal I

2218 bp Xho I - Sal I
LEU2 fragment from
YEP13

EcoRI

BamHI
EcoRI
SAC II

pRIT12544

14000 bp

Sal I

EcoRI

EcoRI

X (Sal I/Xho I)

EcoRI

Figure 14

Construction of pRIT12658

reclone 3328bp Hind III fragment
of pRIT 12574 on pBR322△Bam.

Figure 15

0278940

Figure 16

pRIT12309

2218 bp LEU2
Xho I - Sal I fragment
from YEp13

Sal I

EcoRI   BamHI

X (SalI/Xho I)

Leu2

EcoRI

2 micron

pRIT12377

11804 bp

Sal I

EcoRI

pBR327

EcoRI

BamHI,          EcoRI

T4 polymerase

3050 bp Hind III
fragment from
pRIT12662

T4 polymerase

EcoRI    X

EcoRI

BamHI

EcoRI

pRIT12660

14840 bp

X

X (Sal I/Xho I)

EcoRI

EcoRI

EcoRI

Sal I

EcoRI

Figure 17

Plasmid pRIT12314

- Digested BamHI, Sma I
- Mung Bean nuclease heated

pTDH3

C ATG
G TAC

2µ

pRIT12314

tARG3

Plasmid pRIT12792

- Digested NcoI-Xba I
- Mung bean nuclease treated
- 495 bp fragment purified

BamHI

GGG ACG

PreS1    PreS2

- ligated

2µ    pTDH3

pRIT12843

(a —— f)

preS1

BamHI

preS2

tARG3

Sal I

- digested BAMHI
- digested Sal I
- purification of large 10400 bp
  fragment

Plasmid pRIT12660

- digested BamHI, Sal
- small 4430 bp fragment
  purified

pTDH3    BamHI

2µ

pRIT12843

(a —— f)

Sal I

- ligation

BamHI    Sal I

preS2-S    tARG3

pTDH3    preS I

2µ    pRIT12845

(a —— f)

BamHI

preS2-S

tARG3

LEU2

Sal I

Figure 18

Construction of pRIT1266

Plasmid                      Adaptor

pRIT2290                     BamHI            EcoRI

                             QATC  CAG  TGG

                                   GTC  ACC  TT  AA

                             insertion of the adaptor between the
                             BamHI and EcoRI sites of pRIT12290

reconstruction of the
N-terminal coding                          2    3
region of preS2-S
protein                      ATG  GATC  CAG  TGG | AAT  TC
                             TAC  CTAG  GTC  ACC  TTA  A|G

                                   BamHI          .        EcoRI|

**pRIT12621**

pTDH3

tARG3

- digestion with BamHI
- digestion with mung bean nuclease
- ligation                                        Plasmid
- digestion with EcoRI                            pRIT12581

                                            AccI

                                         ////////////////

                                    EcoRI            EcoRI

- insertion of the EcoRI fragment containing
  the C-terminal coding region of preS2-S protein

                                          2     3

construction of an           Hind III     ATG  CAG  TGG  AAT  TC  ...
E. coli vector                            TAC  GTC  ACC  TTA  AG
containing the
preS2-S expression
cassette              pTDH3

                                                preS2-S
**pRIT12662**

                      tARG3

                             Hind III

Figure 1A

Figure 2A

mPf1                                    mPf15
AAAAAAGAAAATTATAAATAAATATATATATTCGTGTAAAAAT
                                        mPf5/8
     50                                 mPf13
AAGTAGAAACCACGTATATTATAAATTACAATTC ATG   ATG
                                        Met   Met

                                  100
AGA    AAA    TTA    GCT    ATT    TTA    TCT    GTT    TCT
Arg    Lys    Leu    Ala    Ile    Leu    Ser    Val    Ser

TCC    TTT    TTA    TTT    GTT    GAG    GCC    TTA    TTC
Ser    Phe    Leu    Phe    Val    Glu    Ala    Leu    Phe
                                  150
CAG    GAA    TAC    CAG    TGC    TAT    GGA    AGT    TCG
Gln    Glu    Tyr    Gln    Cys    Tyr    Gly    Ser    Ser

TCA    AAC    ACA    AGG    GTT    GTA    AAT    GAA    TTA
Ser    Asn    Thr    Arg    Val    Leu    Asn    Glu    Leu
                     200
AAT    TAT    GAT    AAT    GCA    GGC    ACT    AAT    TTA
Asn    Tyr    Asp    Asn    Ala    Gly    Thr    Asn    Leu

TAT    AAT    GAA    TTA    GAA    ATG    AAT    TAT    TAT
Tyr    Asn    Glu    Leu    Glu    Met    Asn    Tyr    Tyr
       250
GGG    AAA    CAG    GAA    AAT    TGG    TAT    AGT    CTT
Gly    Lys    Gln    Glu    Asn    Trp    Tyr    Ser    Leu

AAA    AAA    AAT    AGT    AGA    TCA    CTT    GGA    GAA
Lys    Lys    Asn    Ser    Arg    Ser    Leu    Gly    Glu
300
AAT    GAT    GAT    GGA    AAT    AAT    AAT    AAT    GGA
Asn    Asp    Asp    Gly    Asn    Asn    Asn    Asn    Gly
                                                350
GAT    AAT    GGT    CGT    GAA    GGT    AAA    GAT    GAA
Asp    Asn    Gly    Arg    Glu    Gly    Lys    Asp    Glu

GAT    AAA    AGA    GAT    GGA    AAT    AAC    GAA    GAC
Asp    Lys    Arg    Asp    Gly    Asn    Asn    Glu    Asp

Fig. 3A a

| AAC | GAG | AAA | TTA | AGG | AAA | CCA | AAA | CAT |
|-----|-----|-----|-----|-----|-----|-----|-----|-----|
| Asn | Glu | Lys | Leu | Arg | Lys | Pro | Lys | His |

REGION I

| AAA | AAA | TTA | AAG | CAA | CCA | GGG | GAT | GGT |
|-----|-----|-----|-----|-----|-----|-----|-----|-----|
| Lys | Lys | Leu | Lys | Gln | Pro | Gly | Asp | Gly |

450

| AAT | CCT | GAT | CCA | AAT | GCA | A | CCA | AAT |
|-----|-----|-----|-----|-----|-----|---|-----|-----|
| Asn | Pro | Asp | Pro | Asn | Ala | Asn | Pro | Asn |

| GTA | GAT | CCC | AAT | GCC | AAC | CCA | AAT | GTA |
|-----|-----|-----|-----|-----|-----|-----|-----|-----|
| Val | Asp | Pro | Asn | Ala | Asn | Pro | Asn | Val |

500

| GAT | CCA | AAT | GCA | AAC | CCA | AAT | GTA | GAT |
|-----|-----|-----|-----|-----|-----|-----|-----|-----|
| Asp | Pro | Asn | Ala | Asn | Pro | Asn | Val | Asp |

| CCA | AAT | GCA | AAC | CCA | AAT | GCA | AAC | CCA |
|-----|-----|-----|-----|-----|-----|-----|-----|-----|
| Pro | Asn | Ala | Asn | Pro | Asn | Ala | Asn | Pro |

550

| AAT | GCA | AAC | CCA | AAT | GCA | AAC | CCA | AAT |
|-----|-----|-----|-----|-----|-----|-----|-----|-----|
| Asn | Ala | Asn | Pro | Asn | Ala | Asn | Pro | Asn |

| GCA | AAC | CCA | AAT | GCA | AAC | CCA | AAT | GCA |
|-----|-----|-----|-----|-----|-----|-----|-----|-----|
| Ala | Asn | Pro | Asn | Ala | Asn | Pro | Asn | Ala |

600

| AAC | CCA | AAT | GCA | AAC | CCA | AAT | GCA | AAC |
|-----|-----|-----|-----|-----|-----|-----|-----|-----|
| Asn | Pro | Asn | Ala | Asn | Pro | Asn | Ala | Asn |

650

| CCC | AAT | GCA | AAT | CCT | AAT | GCA | AAT | CCT |
|-----|-----|-----|-----|-----|-----|-----|-----|-----|
| Pro | Asn | Ala | Asn | Pro | Asn | Ala | Asn | Pro |

| AAT | GCA | AAC | CCA | AAT | GCA | AAT | CCT | AAT |
|-----|-----|-----|-----|-----|-----|-----|-----|-----|
| Asn | Ala | Asn | Pro | Asn | Ala | Asn | Pro | Asn |

700

| GCA | AAC | CCA | AAT | GCA | AAC | CCA | AAC | GTA |
|-----|-----|-----|-----|-----|-----|-----|-----|-----|
| Ala | Asn | Pro | Asn | Ala | Asn | Pro | Asn | Val |

| GAT | CCT | AAT | GCA | AAT | CCA | AAT | GCA | AAC |
|-----|-----|-----|-----|-----|-----|-----|-----|-----|
| Asp | Pro | Asn | Ala | Asn | Pro | Asn | Ala | Asn |

Fig. 3A b

750

| CCA | AAT | GCA | AAC | CCA | AAC | GCA | AAC | CCC |
|-----|-----|-----|-----|-----|-----|-----|-----|-----|
| Pro | Asn | Ala | Asn | Pro | Asn | Ala | Asn | Pro |

| AAT | GCA | AAT | CCT | AAT | GCA | AAC | CCC | AAT |
|-----|-----|-----|-----|-----|-----|-----|-----|-----|
| Asn | Ala | Asn | Pro | Asn | Ala | Asn | Pro | Asn |

800

| GCA | AAT | CCT | AAT | GCA | AAT | CCT | AAT | GCC |
|-----|-----|-----|-----|-----|-----|-----|-----|-----|
| Ala | Asn | Pro | Asn | Ala | Asn | Pro | Asn | Ala |

| AAT | CCA | AAT | GCA | AAT | CCA | AAT | GCA | AAC |
|-----|-----|-----|-----|-----|-----|-----|-----|-----|
| Asn | Pro | Asn | Ala | Asn | Pro | Asn | Ala | Asn |

850

| CCA | AAC | GCA | AAC | CCC | AAT | GCA | AAT | CCT |
|-----|-----|-----|-----|-----|-----|-----|-----|-----|
| Pro | Asn | Ala | Asn | Pro | Asn | Ala | Asn | Pro |

| AAT | GCC | AAT | CCA | AAT | GCA | AAT | CCA | AAT |
|-----|-----|-----|-----|-----|-----|-----|-----|-----|
| Asn | Ala | Asn | Pro | Asn | Ala | Asn | Pro | Asn |

900

| GCA | AAC | CCA | AAT | GCA | AAC | CCA | AAT | GCA |
|-----|-----|-----|-----|-----|-----|-----|-----|-----|
| Ala | Asn | Pro | Asn | Ala | Asn | Pro | Asn | Ala |

| AAC | CCC | AAT | GCA | AAT | CCT | AAT | AAA | AAC |
|-----|-----|-----|-----|-----|-----|-----|-----|-----|
| Asn | Pro | Asn | Ala | Asn | Pro | Asn | Lys | Asn |

950

| AAT | CAA | GGT | AAT | GGA | CAA | GGT | CAC | AAT |
|-----|-----|-----|-----|-----|-----|-----|-----|-----|
| Asn | Gln | Gly | Asn | Gly | Gln | Gly | His | Asn |

1000

| ATG | CCA | AAT | GAC | CCA | AAC | CGA | AAT | GTA |
|-----|-----|-----|-----|-----|-----|-----|-----|-----|
| Met | Pro | Asn | Asp | Pro | Asn | Arg | Asn | Val |

| GAT | GAA | AAT | GCT | AAT | GCC | AAC | AAT | GCT |
|-----|-----|-----|-----|-----|-----|-----|-----|-----|
| Asp | Glu | Asn | Ala | Asn | Ala | Asn | Asn | Ala |

1050

| GTA | AAA | AAT | AAT | AAT | AAC | GAA | GAA | CCA |
|-----|-----|-----|-----|-----|-----|-----|-----|-----|
| Val | Lys | Asn | Asn | Asn | Asn | Glu | Glu | Pro |

| AGT | GAT | AAG | CAC | ATA | GAA | CAA | TAT | TTA |
|-----|-----|-----|-----|-----|-----|-----|-----|-----|
| Ser | Asp | Lys | His | Ile | Glu | Gln | Tyr | Leu |

Fig. 3A c

1100

| AAG | AAA | ATA | AAA | AAT | TCT | ATT | TCA | ACT |
|-----|-----|-----|-----|-----|-----|-----|-----|-----|
| Lys | Lys | Ile | Lys | Asn | Ser | Ile | Ser | Thr |

REGION II

| GAA | TGG | TCC | CCA | TGT | AGT | GTA | ACT | TGT |
|-----|-----|-----|-----|-----|-----|-----|-----|-----|
| Glu | Trp | Ser | Pro | Cys | Ser | Val | Thr | Cys |

| GGA | AAT | GGT | AAT | CAA | GTT | AGA | ATA | AAG |
|-----|-----|-----|-----|-----|-----|-----|-----|-----|
| Gly | Asn | Gly | Ile | Gl | I | Arg | Ile | Lys |

| CCT | GGC | TCT | GCT | AAT | AAA | CCT | AAA | GAC |
|-----|-----|-----|-----|-----|-----|-----|-----|-----|
| Pro | Gly | Ser | Ala | Asn | Lys | Pro | Lys | Asp |

1200

| GAA | TTA | GAT | TAT | GAA | AAT | GAT | ATT | GAA |
|-----|-----|-----|-----|-----|-----|-----|-----|-----|
| Glu | Leu | Asp | Tyr | Glu | Asn | Asp | Ile | Glu |

| AAA | AAA | ATT | TGT | AAA | ATG | GAA | AAA | TGT |
|-----|-----|-----|-----|-----|-----|-----|-----|-----|
| Lys | Lys | Ile | Cys | Lys | Met | Glu | Lys | Cys |

1250

| TCC | AGT | GTG | TTT | AAT | GTC | GTA | AAT | AGT |
|-----|-----|-----|-----|-----|-----|-----|-----|-----|
| Ser | Ser | Val | Phe | Asn | Val | Val | Asn | Ser |

1300

| TCA | ATA | GGA | TTA | ATA | ATG | GTA | TTA | TCC |
|-----|-----|-----|-----|-----|-----|-----|-----|-----|
| Ser | Ile | Gly | Leu | Ile | Met | Val | Leu | Ser |

| TTC | TTG | TTC | CTT | AAT | TAG | ATAAAGA ACACAT |
|-----|-----|-----|-----|-----|-----|------|
| Phe | Leu | Phe | Leu | Asn |  |  |

1350

CTTAGTTTGAGTTGTACAATATTTATAAAAATATATACTACTTT

1400

TTTTCTTAATTTTCATTTTTCTTTATATTTTCCGAGGGAATTTAT

1450

TTTTTTGTGAATATTTAATT ACGTTTGCGATTAATTGTAGAAAT

1500

ATATATGTATATACT ATATTTATAGAATGTGTTATTCTCAAAAA

Fig. 3A d

CAACAACAAAAAAAAAAAAAAAAAAAAAAAAAAAAGAAAAAAGG

ATTAAAAGTAAAATAGTTATAAATATTTTCAAAAATATTTATAA

CACAAAAAATACTTCGAAGTTCATTTAACATTTTTGTTTATTT

ATTTATTTATATATTTCATTTTTACGTATTTATATTATAAAATG

GTGTATCTTAAAAATAGTGAACTATATATATAAAATATTAATT

TAAAAAAATTATAACTTTCTTTTTATTTTCTAAAATAACTTAAA

AATTATATGTTTAAGAAAGGGGTAAATTATAATATTTGTATAA

ATATATAAACATAGATATATTAAATAAAATAACAAATGTACTA

TATTTGTGCATAAGACGTATACGCTTTATATAATACAACAATA

TTAATTGTAATAATATTTGTGGTAGTGTGAACACTAAAATTGA

TAATAATGATTATAATACAGAAGAAATAAAAAATGAATCCAAT

ATAGGATTTACAACAAATATTCATGAAGCAAAAATAATTCAAG

AAAAGACATATGGATTAATAATAAACGATAAAATAAAGAAAG

Fig. 3A e

2100
AAGAATATGATGATTGTAATAATAATAATAATAATAATATTAT

2150
AATACAGATAAGAGAAGTTGGACTTAATTATTTTGGAGATAC

TCTCGATGAATCGAATCCATGTAATGATCTTACAGGTATTAA

2200
TATATGGGAAAGTTGTCTTGTGGCTAGTCGATGGTTTAGCG

2250
ATTTATCTTTACAGAATTTTTTTTCGAATAAAAATATTTTAGA

2300
AATTGGTGCTGGCAGTGGTTTGGCTAGTATAATAATATTTAT

ATATTCTAATATTTACAA


Fig. 3A f

## Sequence of the Pre sl- Pre S2 region of pRIT 12792

PRE-S1 REGION

Nco I
-163

| CO | ATG | GGG | ACG | AAT | CTT | TCT | GTT | CCC | AAC |
|----|-----|-----|-----|-----|-----|-----|-----|-----|-----|
|    | Met | Gly | Thr | Asn | Leu | Ser | Val | Pro | Asn |

|  | CCT | CTG | GGA | TTC | TTT | CCC | GAT | CAT | CAG |
|--|-----|-----|-----|-----|-----|-----|-----|-----|-----|
|  | Pro | Leu | Gly | Phe | Phe | Pro | Asp | His | Gln |

|  | TTG | GAC | CCT | GCA | TTC | GGA | GCC | AAC | TCA |
|--|-----|-----|-----|-----|-----|-----|-----|-----|-----|
|  | Leu | Asp | Pro | Ala | Phe | Gly | Ala | Asn | Ser |

|  | AAC | AAT | CCA | GAT | TGG | GAC | TTC | AAC | CCC |
|--|-----|-----|-----|-----|-----|-----|-----|-----|-----|
|  | Asn | Asn | Pro | Asp | Trp | Asp | Phe | Asn | Pro |

|  | ATC | AAG | GAC | CAC | TGG | CCA | GCA | GCC | AAC |
|--|-----|-----|-----|-----|-----|-----|-----|-----|-----|
|  | Ile | Lys | Asp | His | Trp | Pro | Ala | Ala | Asn |

|  | CAG | GTA | GGA | GTG | GGA | GCA | TTC | GGG | CCA |
|--|-----|-----|-----|-----|-----|-----|-----|-----|-----|
|  | Gln | Val | Gly | Val | Gly | Ala | Phe | Gly | Pro |

|  | GGG | CTC | ACC | CCT | CCA | CAC | GGC | GGT | ATT |
|--|-----|-----|-----|-----|-----|-----|-----|-----|-----|
|  | Gly | Leu | Thr | Pro | Pro | His | Gly | Gly | Ile |

|  | TTG | GGG | TGG | AGC | CCT | CAG | GCT | CAG | GGC |
|--|-----|-----|-----|-----|-----|-----|-----|-----|-----|
|  | Leu | Gly | Trp | Ser | Pro | Gln | Ala | Gln | Gly |

|  | ATA | TTG | ACC | ACA | GTG | TCA | ACA | ATT | CCT |
|--|-----|-----|-----|-----|-----|-----|-----|-----|-----|
|  | Ile | Leu | Thr | Thr | Val | Ser | Thr | Ile | Pro |

|  | CCT | CCT | GCC | TCC | ACC | AAT | CGG | CAG | TCA |
|--|-----|-----|-----|-----|-----|-----|-----|-----|-----|
|  | Pro | Pro | Ala | Ser | Thr | Asn | Arg | Gln | Ser |

|  | GGA | AGG | CAG | CCT | ACT | CCC | ATC | TCT | CCA |
|--|-----|-----|-----|-----|-----|-----|-----|-----|-----|
|  | Gly | Arg | Gln | Pro | Thr | Pro | Ile | Ser | Pro |

|  | CCT | CTA | AGA | GAC | AGT | CAT | CCT | CAG | GCC |
|--|-----|-----|-----|-----|-----|-----|-----|-----|-----|
|  | Pro | Leu | Arg | Asp | Ser | His | Pro | Gln | Ala |

Fig. 4A a

## PRE-S2 REGION

-55

| | | | | | | | | |
|------|------|------|------|------|------|------|------|------|
| ATG | CAG | TGG | AAT | TCC | ACT | GCC | TTC | CAC |
| Met | Gln | Trp | Asn | Ser | Thr | Ala | Phe | His |
| | | | | | | | | |
| CAA | GCT | CTG | CAG | GAT | CCC | AGA | GTC | AGG |
| Gln | Ala | Leu | Gln | Asp | Pro | Arg | Val | Arg |
| | | | | | | | | |
| GGT | CTG | TAT | TTT | CCT | GCT | GGT | GGC | TCC |
| Gly | Leu | Tyr | Phe | Pro | Ala | Gly | Gly | Ser |
| | | | | | | | | |
| AGT | TCA | GGA | ACA | GTA | AAC | CCT | GCT | CCG |
| Ser | Ser | Gly | Thr | Val | Asn | Pro | Ala | Pro |
| | | | | | | | | |
| AAT | ATT | GCC | TCT | CAC | ATT | TCG | TCA | AGC |
| Asn | Ile | Ala | Ser | His | Ile | Ser | Ser | Ser |
| | | | | | | | | |
| TCC | GCG | AGG | ACT | GGG | GAC | CCT | GTG | ACG |
| Ser | Ala | Arg | Thr | Gly | Asp | Pro | Val | Thr |

Xba I

AA TAATAATCTAGA

Fig. 4A b

Figure 1B

Figure 2B

Hha I - Hind III fragment (230bp) op pRIT12901

T4 polymerase

CAA ..... AGC T
GTT ..... TCG A

pRIT10911/BamHI/Mung Bean nuclease

MET GLN      SER SER
ATG  CAA .....  AGCT  CC
TAC  GTT .....  TCGA  GG

pRIT12897

pTDH3                              preS2-S

(10911)

Figure 3B

p. Dreesman (Synthetic)

```
GAT CTC [ GAC AGG    ...    AGA TCC ] CCG
     AG [ CTG TCC           TCT AGG ] GGC CTA  G
          ASP ARG           ARG  SER
```

pRIT10911/BamHI

```
MET ASP LEU  ASP          PRO ASP PRO

ATG GAT CTC GAC   ...   CCG GAT CCC
TAC CTA GAG CTG   ...   GGC CTA GGG
```

pRIT12899

pTDH3                                      preS2-S

(10911)

Figure 4B

0278940

Figure 5B